(19) Europäisches Patentamt
European Patent Office
Office européen des brevets

(11) EP 1 560 025 A2

(12) **EUROPEAN PATENT APPLICATION**

(43) Date of publication:
**03.08.2005 Bulletin 2005/31**

(51) Int Cl.[7]: **G01N 33/68**, C07K 14/47

(21) Application number: **04022939.5**

(22) Date of filing: **27.09.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **03.10.2003 US 508699 P**

(71) Applicant: **F. Hoffmann-La Roche AG**
**4070 Basel (CH)**

(72) Inventors:
- **Kochan, Jarema Peter**
  **Towaco, New Jersey 07082 (US)**
- **Martin, Mitchell Lee**
  **Verona New Jersey 07044 (US)**
- **Rosinski, James Andrew**
  **Nutley New Jersey 07110 (US)**

(54) **Specific markers for diabetes**

(57) The present invention provides polypeptides which are correlated with pre-diabetes, diabetes or susceptibility to diabetes which can be used as markers for diagnosis of pre-diabetes, diabetes or a susceptibility or predisposition to develop diabetes. The invention also provides methods for the diagnosis of pre-diabetes, diabetes and/or the susceptibility to diabetes by obtaining a biological sample and detecting and/or measuring the increase of one or more polypeptides as disclosed herein. Screening methods relating to agonists and antagonists of the specific polypeptides disclosed herein are provided. Antibodies may also be raised against these polypeptide markers for the detection and/or treatment of diabetes. Proteins, protein fragments or peptides can be used for the treatment of diabetes or pre-diabetes.

EP 1 560 025 A2

## Description

The present invention generally relates to markers for diagnosis of pre-diabetes, diabetes or patients susceptible to developing diabetes. Additionally, the present invention generally relates to an in vitro method for the diagnosis of pre-diabetes, diabetes and/or the susceptibility to diabetes comprising the steps of a) obtaining a biological sample; and b) detecting and/or measuring the increase or decrease of specific markers as disclosed herein. Furthermore, screening methods relating to activators, agonists or antagonists of the specific markers disclosed herein are provided. Moreover, the present invention provides using gene expression profiles or their products in blood, to classify individuals who take part in clinical studies for the identification of therapeutics efficacious in the treatment of diabetes.

The development of diabetes, and, more particularly, Type II diabetes, and diabetes related co-morbidities, takes place over a period of years or decades. During this time period, a process that is dependent on both genetic and environmental contributions takes shape and eventually leads to the development of diabetes and / or diabetes related co-morbidities, such as CVD, nephropathy, neuropathy, retinopathy and the like. The ability to identify individuals with an increased risk of developing these conditions may provide the opportunity to intervene pharmacologically, or to change the individual's lifestyle, as to prevent the onset of these medical conditions.

According to one aspect of the present invention, there is provided a method for the diagnosis of pre-diabetes, diabetes or the susceptibility to diabetes which comprises obtaining a biological sample; and detecting or measuring the level of a polypeptide marker, the polypeptide marker comprising at least one polypeptide selected from the group consisting of the polypeptides having SEQ ID Nos. 2, 4, 6, 8, 10, 12, 14, 16, 18, 20, 22, 24, 26, 28, 30, 32 and 34.

There is also provided a method for the diagnosis of pre-diabetes, diabetes or the susceptibility to diabetes which comprises detecting or measuring the level of a polypeptide marker in a biological sample, the polypeptide marker comprising at least one polypeptide selected from the group consisting of the polypeptides having SEQ ID Nos. 2, 4, 6, 8, 10, 12, 14, 16, 18, 20, 22, 24, 26, 28, 30, 32 and 34.

According to another aspect of the present invention, there is provided a method for the diagnosis of pre-diabetes, diabetes or the susceptibility to diabetes which comprises obtaining a biological sample; and detecting or measuring the level of a marker, the nucleic acid marker comprising at least one nucleic acid molecule selected from the group consisting of the nucleic acid molecules of SEQ ID Nos. 1, 3, 5, 7, 9, 11, 13, 15, 17, 19, 21, 23, 25, 27, 29, 31 and 33.

There is provided a method for the diagnosis of pre-diabetes, diabetes or the susceptibility to diabetes which comprises detecting or measuring the level of a marker in a biological sample, the nucleic acid marker comprising at least one nucleic acid molecule selected from the group consisting of the nucleic acid molecules of SEQ ID Nos. 1, 3, 5, 7, 9, 11, 13, 15, 17, 19, 21, 23, 25, 27, 29, 31 and 33.

According to a further aspect of the present invention, there is provided a screening method for identifying a compound which interacts with a polypeptide whose expression is regulated in diabetes, the polypeptide being selected from the group consisting of the polypeptides having SEQ ID Nos. 2, 4, 6, 8, 10, 12, 14, 16, 18, 20, 22, 24, 26, 28, 30, 32 and 34, which comprises contacting said polypeptide with a compound or a plurality of compounds under conditions which allow interaction of the compound with the polypeptide; and detecting the interaction between the compound or plurality of compounds with said polypeptide.

According to yet another aspect of the present invention, there is provided a screening method for identifying a compound which is an agonist or an antagonist of a polypeptide whose expression is regulated in diabetes, the polypeptide being selected from the group consisting of the polypeptides having SEQ ID Nos. 2, 4, 6, 8, 10, 12, 14, 16, 18, 20, 22, 24, 26, 28, 30, 32 and 34, which comprises contacting the polypeptide with a compound under conditions which allow interaction of the compound with the polypeptide; determining a first level of activity of the polypeptide; determining a second level of activity of the polypeptide expressed in a host which has not been contacted with the compound; and quantitatively relating the first level of activity with the second level of activity, wherein when the first level of activity is less than the second level of activity, the compound is identified as an agonist or antagonist of the polypeptide.

According to still a further aspect of the present invention, there is provided a screening method for identifying a compound which is an inhibitor of the expression of a polypeptide whose expression is upregulated in diabetes, the polypeptide being selected from the group consisting of the polypeptides having SEQ ID Nos. 2, 4, 6, 8, 10, 12, 14, 16, 18, 20, 22, 24, 26, 28, 30, 32 and 34, which comprises contacting a host which expresses said polypeptide with a compound; determining a first expression level or activity of the polypeptide; determining a second expression level or activity of the polypeptide in a host which has not been contacted with the compound; and quantitatively relating the first expression level or activity with the second expression level or activity, wherein when the first expression level or activity is less than the second expression level or activity, the compound is identified as an inhibitor of the expression of the polypeptide.

According to another aspect of the present invention, there are provided antibodies against the proteins, or antigen-binding fragments thereof, for the use in an in vitro method for the diagnosis of pre-diabetes, diabetes or susceptibility to diabetes, the proteins being selected from the group consisting of the proteins having SEQ ID Nos. 2, 4, 6, 8, 10, 12, 14, 16, 18, 20, 22, 24, 26, 28, 30, 32 and 34.

According to still another aspect of the present invention, there is provided a method of correlating protein levels in a mammal with a diagnosis of pre-diabetes, diabetes or susceptibility to develop diabetes, which comprises selecting one or more proteins selected from the group consisting of the proteins having SEQ ID Nos. 2, 4, 6, 8, 10, 12, 14, 16, 18, 20, 22, 24, 26, 28, 30, 32 and 34; determining the level of the one or more proteins in the mammal; and generating an index number, Y, which indicates a presence of diabetes or a susceptibility thereto.

Preferably, the method hereinbefore described comprises comparing index number, Y, to index numbers of known diabetics and non-diabetics. In another preferred embodiment, said method comprises monitoring said index number, Y, over time to determine the stage of diabetes or susceptibility thereto.

According to a further aspect of the present invention, there is provided a kit for screening of compounds that activate or inhibit a polypeptides or stimulate or inhibit the expression of any of the polypeptides, the polypeptides being selected from the group consisting of the polypeptides having SEQ ID Nos. 2, 4, 6, 8, 10, 12, 14, 16, 18, 20, 22, 24, 26, 28, 30, 32 and 34.

According to yet a further aspect of the present invention, there is provided a method for monitoring serum levels of one or more proteins to detect a pre-diabetic disease state, a diabetic disease state or a susceptibility to develop a diabetic disease state, the method comprises raising antibodies of the one or more proteins; detecting the serum level of the proteins; and comparing the serum level to those of known diabetics and known non-diabetics.

According to another aspect of the present invention, there is provided a method for treating diabetes and pre-diabetes which comprises administering, to a patient in need thereof, a therapeutically effective amount of at least one antibody against at least one protein, or antigen-binding fragment thereof, selected from the group consisting of the proteins having SEQ ID Nos. 2, 4, 6, 8, 10, 12, 14, 16, 18, 20, 22, 24, 26, 28, 30, 32 and 34.

According to a further aspect of the present invention, there is provided a method for treating diabetes and pre-diabetes comprising administering, to a patient in need thereof, a therapeutically effective amount of at least one protein, protein fragment or peptide selected from the group consisting of the proteins having SEQ ID Nos. 2, 4, 6, 8, 10, 12, 14, 16, 18, 20, 22, 24, 26, 28, 30, 32 and 34.

The above, and other objects, features and advantages of the present invention will become apparent from the following description read in conjunction with the accompanying drawings.

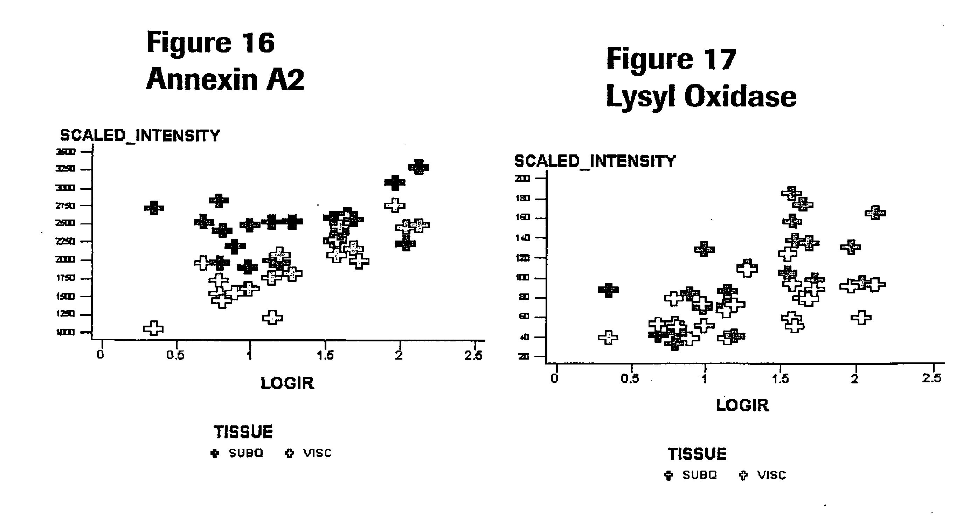

Figures 1 through 17 are graphs of the scaled intensity vs. log of the insulin resistance for various adipose levels of RNA measured by Affymetrix analysis in the Type II diabetic patients' visceral and subcutaneous adipose tissues.

The problem of identifying genes and polypeptides suitable as markers of diabetes for early diagnosis of the disease, and the long felt need for such markers, was overcome by the present invention. It was surprisingly found that a specific set of genes that are secreted in subcutaneous and visceral adipose tissues when comparing normal, IGT (impaired glucose tolerant) or diabetic individuals. The differentially expressed genes, and the polypeptides they encode, along with their accession numbers, are listed in Table 1.

Table 1 :

| Adipose Secreted Proteins in T2D | | | | | | |
|---|---|---|---|---|---|---|
| Name | Abbreviation | Alias | GenBank | Locus Link | MRNA | Protein |
| Vascular endothelial growth factor B | VEGF-B | VRG, VEGFL | BC008818 | 7423 | NM 003377 SEQ ID 1 | NP 003368 SEQ ID 2 |
| Apolipoprotein D | APOD | | J02611 | 347 | NM 001647 SEQ ID 3 | NP 001638 SEQ ID 4 |
| Amine oxidase, copper containing 3 | AOC3 | VAP1, VAP-1 : vascular adhesion protein I; HPAO | U39447 | 8639 | NM 003734 SEQ ID 5 | NP 003725 SEQ ID 6 |

Table 1 : (continued)

| Adipose Secreted Proteins in T2D | | | | | | |
|---|---|---|---|---|---|---|
| Name | Abbreviation | Alias | GenBank | Locus Link | MRNA | Protein |
| Dipeptidyl peptidase IV | DPPIV, DPP4 | CD26, ADCP2, TP103, ADABP: adenosine deaminase complexing protein 2, T-cell activation antigen CD26 | S79876 | 1803 | NM 001935 SEQ ID 7 | NP 001926 SEQ ID 8 |
| Fibroblast Growth Factor 2 (basic) | FGF2, BFGF | Prostatropin, HBGH-2:heparin binding growth factor 2 precursor | J04513 | 2247 | NM 002006 SEQ ID 9 | NP 001997 SEQ ID 10 |
| Thrombospond in 2 | THBS2, TSP2 | | L12350 | 7058 | NM 003247 SEQ ID 11 | NP 003238 SEQ ID 12 |
| Fibulin 1* | FBLN1 | | X53743 | 2192 | NM 001996 SEQ ID 13 | NP 001987 SEQ ID 14 |
| Annexin XI | ANXA11 | CAP50: calcyclin-associated annexin 50 autoantigen, 56-kD | AJ278463 | 311 | NM 001157 SEQ ID 15 | NP 001148 SEQ ID 16 |
| Protein S (alpha) | PROS1, PSA | | M15036 | 5627 | NM 000313 SEQ ID 17 | NP 000304 SEQ ID 18 |
| H factor 1 (complement) | HF1 | CFH, HUS | Y00716 | 3075 | NM 000186 SEQ ID 19 | NP 000177 SEQ ID 20 |
| Superoxide dismutase 3 | SOD-3 | Extracellular superoxide dismutase | U10116 | 6649 | NM 003102 SEQ ID 21 | NP 003093 SEQ ID 22 |
| Neuronatin | NNAT | Peg5 | U31767 | 4826 | NM 005386 SEQ ID 23 | NP 005377 SEQ ID 24 |
| Follistatin-like | FSTL3 | Secreted | U76702 | 10272 | NM 005860 | NP 005851 |
| 3 | | glycoprotein FLRG, FSRP: follistatin-related protein | | | SEQ ID 25 | SEQ ID 26 |
| Protease, serine 23 | SPUVE | ZSIG13, MGC5107, PRSS23-pending, umbilical endothelium | AF015287 | 11098 | NM 007173 SEQ ID 27 | NP 009104 SEQ ID 28 |
| Annexin A2 | ANXA2 | ANX2, LIP2, LPC2, CAL1H, LPC2D, ANX2L4 | D00017 | 302 | NM 004039 SEQ ID 29 | NP 004030 SEQ ID 30 |
| Lysyl oxidase | LOX | protein-lysine 6-oxidase | AF039290 | 4015 | NM 002317 SEQ ID 31 | NP 002308 SEQ ID 32 |

Table 1 : (continued)

| Adipose Secreted Proteins in T2D | | | | | | |
|---|---|---|---|---|---|---|
| Name | Abbreviation | Alias | GenBank | Locus Link | MRNA | Protein |
| ECGF 1 | ECGF1 | endothelial cell growth factor 1 (platelet-derived) | M58602, M63193 | 1890 | NM 001953 SEQ ID 33 | NP 001944 SEQ ID 34 |

Based on the polypeptides listed in table 1, the present invention provides a marker for diagnosis of diabetes or an early stage of diabetes (pre-diabetes) comprising at least one polypeptide selected from the group consisting of the polypeptides listed in table 1 (Seq ID Nos. 2, 4, 6, 8, 10, 12, 14, 16, 18, 20, 22, 24, 26, 28, 30, 32 and 34). Thus, the term "marker" as used herein refers to one or more polypeptides that are regulated in visceral or subcutaneous adipose tissue and that can be used to diagnose diabetes, pre-diabetes or a susceptibility to diabetes, either alone or as combinations of multiple polypeptides that are known to be regulated in adipose tissues, or in other tissues in diabetes.

The term "polypeptide" as used herein, refers to a polymer of amino acids, and not to a specific length. Thus, peptides, oligopeptides and proteins are included within the definition of polypeptide.

Preferably, the marker of this invention is a marker comprising at least one polypeptide selected from the group consisting of the polypeptides listed in table 1.

With the identification of polypeptides regulated in diabetes, the present invention provides an in vitro method for the diagnosis of diabetes, pre-diabetes and/or the susceptibility to diabetes comprising the steps of obtaining a biological sample; and detecting and/or measuring the increase or decrease of a marker described hereinbefore.

The term "differentially expressed" in accordance with this invention relates to marker genes which are either up- or downregulated in tissues and/or cells derived from diabetic or pre-diabetic individuals/patients or individuals susceptible to diabetes in comparison to healthy individuals or individuals which do not suffer from diabetes or are not prone to suffer from diabetes.

As illustrated in appended Table 2 and in the appended examples, specific marker genes which are upregulated comprise, but are not limited to dipeptidyl peptidase IV, fibroblast growth factor 2 (basic), thrombospondin 2, fibulin 1, protein S (alpha), H factor 1 (complement), protease serine 23, annexin A2, and lysyloxidase.

Table 2 :

| Genes upregulated in diabetes | | | | | | |
|---|---|---|---|---|---|---|
| Name | Abbreviation | Alias | GenBank | Locus Link | MRNA | Protein |
| Dipeptidyl peptidase IV | DPPIV, DPP4 | CD26, ADCP2, TP103, ADABP: adenosine deaminase complexing protein 2, T-cell activation antigen CD26 | S79876 | 1803 | NM 001935 SEQ ID 7 | NP 001926 SEQ ID 8 |
| Fibroblast Growth Factor 2 (basic) | FGF2, BFGF | Prostatropin, HBGH-2:heparin binding growth factor 2 precursor | J04513 | 2247 | NM 002006 SEQ ID 9 | NP 001997 SEQ ID 10 |
| Thrombos pondin 2 | THBS2, TSP2 | | L12350 | 7058 | NM 003247 SEQ ID 11 | NP 003238 SEQ ID 12 |
| Fibulin 1* | FBLN1 | | X53743 | 2192 | NM 001996 SEQ ID 13 | NP 001987 SEQ ID 14 |
| Protein S (alpha) | PROS1, PSA | | M15036 | 5627 | NM 000313 SEQ ID 17 | NP 000304 SEQ ID 18 |

Table 2 : (continued)

| Genes upregulated in diabetes | | | | | | |
|---|---|---|---|---|---|---|
| Name | Abbreviation | Alias | GenBank | Locus Link | MRNA | Protein |
| H factor 1 (compleme nt) | HF1 | CFH, HUS | Y00716 | 3075 | NM 000186 SEQ ID 19 | NP 000177 SEQ ID 20 |
| Protease, serine 23 | SPUVE | ZSIG13, MGC5107, PRSS23-pending, umbilical endothelium | AF01528 | 11098 | NM 007173 SEQ ID 27 | NP 009104 SEQ ID 28 |
| Annexin A2 | ANXA2 | ANX2, LIP2, LPC2, CAL1H, LPC2D, ANX2L4 | D00017 | 302 | NM 004039 SEQ ID 29 | NP 004030 SEQ ID 30 |
| Lysyl oxidase | LOX | protein-lysine 6-oxidase | AF039290 | 4015 | NM 002317 SEQ ID 31 | NP 002308 SEQ ID 32 |

Marker genes which are downregulated in diabetes comprise, but are not limited to vascular endothelial growth factor, apolipoprotein D, amine oxidase, copper containing 3, superoxide dimutase 3 and neuronatin, follistatin-like 3.

Table 3 :

| Genes downregulated in diabetes | | | | | | |
|---|---|---|---|---|---|---|
| Name | Abbreviation | Alias | GenBank | Locus Link | MRNA | Protein |
| Vascular endothelial growth factor B | VEGF-B | VRG, VEGFL | BC008818 | 7423 | NM 003377 SEQ ID 1 | NP 003368 SEQ ID 2 |
| Apolipopro tein D | APOD | | J02611 | 347 | NM 001647 SEQ ID 3 | NP 001638 SEQ ID 4 |
| Amine oxidase, copper containing 3 | AOC3 | VAP1, VAP-1 : vascular adhesion protein I; HPAO | U39447 | 8639 | NM 003734 SEQ ID 5 | NP 003725 SEQ ID 6 |
| Superoxide dismutase 3 | SOD-3 | Extracellular superoxide dismutase | U10116 | 6649 | NM 003102 SEQ ID 21 | NP 003093 SEQ ID 22 |
| Neuronatin | NNAT | Peg5 | U31767 | 4826 | NM 005386 SEQ ID 23 | NP 005377 SEQ ID 24 |
| Follistatin-like 3 | FSTL3 | Secreted glycoprotein FLRG, FSRP: follistatin-related protein | U76702 | 10272 | NM 005860 SEQ ID 25 | NP 005851 SEQ ID 26 |

In accordance with the present invention, the term "biological sample" as employed herein means a sample which comprises material wherein the differential expression of marker genes may be measured and may be obtained from an individual. Particular preferred samples comprise body fluids, like blood, serum, plasma, urine, synovial fluid, spinal fluid, cerebrospinal fluid, semen or lymph, as well as body tissues, such as visceral and subcutaneous adipose tissue.

The detection and/or measurement of the differentially expressed marker genes may comprise the detection of an increase, decrease and/or the absence of a specific nucleic acid molecule, for example RNA or cDNA, the measurement/detection of a expressed polypeptide/protein as well as the measurement/detection of a (biological) activity (or lack thereof) of the expressed protein/polypeptide. The (biological) activity may comprise enzymatic activities, activities relating to signaling pathway-events e.g. antigen-recognition as well as effector-events.

Methods for the detection/measurement of RNA and/or cDNA levels are well known in the art and comprise methods as described in the appended examples. Such methods include, but are not limited to PCR-technology, northern blots, affymetrix chips, and the like.

The term "detection" as used herein refers to the qualitative determination of the absence or presence of polypeptides. The term "measured" as used herein refers to the quantitative determination of the differences in expression of polypeptides in biological samples from patients with diabetes and biological samples from healthy individuals. Additionally, the term "measured" may also refer to the quantitative determination of the differences in expression of polypeptides in biological samples from visceral adipose tissue and subcutaneous adipose tissue.

Methods for detection and/or measurement of polypeptides in biological samples are well known in the art and include, but are not limited to, Western-blotting, ELISAs or RIAs, or various proteomics techniques. Monoclonal or polyclonal antibodies recognizing the polypeptides listed in Table 1, or peptide fragments thereof, can either be generated for the purpose of detecting the polypeptides or peptide fragments, eg. by immunizing rabbits with purified proteins, or known antibodies recognizing the polypeptides or peptide fragments can be used. For example, an antibody capable of binding to the denatured proteins, such as a polyclonal antibody, can be used to detect the peptides of this invention in a Western Blot. An example for a method to measure a marker is an ELISA. This type of protein quantitation is based on an antibody capable of capturing a specifc antigen, and a second antibody capable of detecting the captured antigen. A further method for the detection of a diagnostic marker for diabetes is by analysing biopsy specimens for the presence or absence of the markers of this invention. Methods for the detection of these markers are well known in the art and include, but are not limited to, immunohistochemistry or immunofluorescent detection of the presence or absence of the polypeptides of the marker of this invention. Methods for preparation and use of antibodies, and the assays mentioned hereinbefore are described in Harlow, E. and Lane, D. Antibodies: A Laboratory Manual, (1988), Cold Spring Harbor Laboratory Press.

While the analysis of one of the polypeptides listed in Table 1 may accurately diagnose diabetes or pre-diabetes, the accuracy of the diagnosis of diabetes may be increased by analyzing combinations of multiple polypeptides listed in Table 1. Thus, the in vitro method herein before described, comprises a marker which comprises at least two, preferably at least three, more preferably at least four, even more preferably at least five, and most preferably at least six of the polypeptides listed in Table 1.

For diagnosis of diabetes, suitable biological samples need to be analyzed for the presence or absence of a marker. The biological samples can be serum, plasma, or various tissues including cells of adipose tissue. Cells from adipose tissue can be obtained by any known method, such as ERCP, secretin stimulation, fine-needle aspiration, cytologic brushings and large-bore needle biopsy.

It is also possible to diagnose diabetes by detecting and/or measuring nucleic acid molecules coding for the marker hereinbefore described. Preferably, the nucleic acid molecule is RNA or DNA.

In one embodiment of the present invention, the in vitro method herein before described comprises comparing the expression levels of at least one of the nucleic acids encoding the polypeptides in an individual suspected to suffer from pre-diabetes, diabetes and/or to be susceptible to diabetes, to the expression levels of the same nucleic acids in a healthy individual.

In another embodiment of the present invention, the in vitro method herein before described comprises comparing the expression levels of at least one of the polypeptides in an individual suspected to suffer from pre-diabetes, diabetes and/or to be susceptible to diabetes, to the expression levels of the same nucleic acids in a healthy individual.

In yet another embodiment of the present invention the in vitro method herein before described comprises comparing the expression level of the polypeptide marker in an individual suspected to suffer from pre-diabetes, diabetes and/or to be susceptible to diabetes to the expression levels of the same marker in a healthy individual. In a more preferred embodiment of the in vitro method, an increase or decrease of the expression levels of the marker is indicative of diabetes or the susceptibility to diabetes.

In yet another embodiment of the present invention the in vitro method herein before described comprises comparing the expression level of the nucleic acid marker in an individual suspected to suffer from pre-diabetes, diabetes and/or to be susceptible to diabetes to the expression levels of the same marker in a healthy individual. In a more preferred embodiment of the in vitro method, an increase or decrease of the expression levels of the marker is indicative of diabetes or the susceptibility to diabetes.

Yet, in another embodiment of the present invention, the inventive in vitro method comprises a method, wherein the detection and/or measuring step is carried out by detecting and/or measuring (a) protein(s)/(a) polypeptide(s) or a fragment thereof encoded by the gene(s) as listed in Table 1. Again, these detection/measuring steps comprise methods known in the art, like inter alia, proteomics, immuno-chemical methods like Western-blots, ELISAs and the like.

Preferably, in the in vitro method of the present invention the expression levels of at least two marker genes as listed in Table 1 are compared. It is also preferred in the in vitro method of the present invention that the expression levels of at least one marker gene as listed in Table 2 is compared with at least one marker gene as listed in Table 3. For example, it is envisaged that the inventive method comprises the measurement/detection of at least one up-regulated and at least one down-regulated marker gene or gene product.

The present invention also provides a screening method for identifying and/or obtaining a compound which interacts with a polypeptide listed in table 1, whose expression is regulated in diabetes, comprising the steps of contacting the polypeptide with a compound or a plurality of compounds under conditions which allow interaction of the compound with the polypeptide; and detecting the interaction between the compound or plurality of compounds with the polypeptide.

For polypeptides that are associated with the cell membrane on the cell surface, or which are expressed as transmembrane or integral membrane polypeptides, the interaction of a compound with the polypeptides can be detected with different methods which include, but are not limited to, methods using cells that either normally express the polypeptide or in which the polypeptide is overexpressed, eg. by detecting displacement of a known ligand which is labeled by the compound to be screened. Alternatively, membrane preparations may be used to test for interaction of a compound with such a polypeptide.

Interaction assays to be employed in the method disclosed herein may comprise FRET-assays (fluorescence resonance energy transfer; as described, inter alia, in Ng, Science 283 (1999), 2085-2089 or Ubarretxena-Belandia, Biochem. 38 (1999), 7398-7405), TR-FRETs and biochemical assays as disclosed herein. Furthermore, commercial assays like "Amplified Luminescent Proximity Homogenous Assay™" (BioSignal Packard) may be employed. Further methods are well known in the art and, inter alia, described in Fernandez, Curr. Opin. Chem. Biol. 2 ( 1998), 547-603.

The "test for interaction" may also be carried out by specific immunological and/or biochemical assays which are well known in the art and which comprise, e.g., homogenous and heterogenous assays as described herein below. The interaction assays employing read-out systems are well known in the art and comprise, inter alia, two-hybrid screenings (as, described, inter alia, in EP-0 963 376, WO 98/25947, WO 00/02911; and as exemplified in the appended examples), GST-pull-down columns, coprecipitation assays from cell extracts as described, inter alia, in Kasus-Jacobi, Oncogene 19 (2000), 2052-2059, "interaction-trap" systems (as described, inter alia, in US 6,004,746) expression cloning (e.g. lamda gt11), phage display (as described, inter alia, in US 5,541,109), in vitro binding assays and the like. Further interaction assay methods and corresponding read out systems are, inter alia, described in US 5,525,490, WO 99/51741, WO 00/17221, WO 00/14271 or WO 00/05410. Vidal and Legrain (1999) in Nucleic Acids Research 27, 919-929 describe, review and summarize further interaction assays known in the art which may be employed in accordance with the present invention.

Homogeneous (interaction) assays comprise assays wherein the binding partners remain in solution and comprise assays, like agglutination assays. Heterogeneous assays comprise assays like, inter alia, immuno assays, for example, Enzyme Linked Immunosorbent Assays (ELISA), Radioactive Immunoassays (RIA), Immuno Radiometric Assays (IRMA), Flow Injection Analysis (FIA), Flow Activated Cell Sorting (FACS), Chemiluminescent Immuno Assays (CLIA) or Electrogenerated Chemiluminescent (ECL) reporting.

The present invention further provides a screening method for identifying and/or obtaining a compound which is an agonist or an antagonist of a polypeptide listed in Table 1 whose expression is regulated in diabetic patients, comprising the steps of a) contacting the polypeptide with a compound identified and/or obtained by the screening method described above under conditions which allow interaction of the compound with the polypeptide; b) determining the activity of the polypeptide; c) determining the activity of the polypeptide expressed in the host as defined in (a), which has not been contacted with the compound; and d) quantitatively relating the activity as determined in (b) and (c), wherein a decreased activity determined in (b) in comparison to (c) is indicative for an agonist or antagonist. This screening assay can be performed either as an in vitro assay, or as a host-based assay. The host to be employed in the screening methods of the present invention and comprising and/or expressing a polypeptide listed in Table 1 may comprise prokaryotic as well as eukaryotic cells. The cells may comprise bacterial cells, yeast cells, as well as cultured (tissue) cell lines, inter alia, derived from mammals. Furthermore animals may also be employed as hosts, for example a non-human transgenic animal. Accordingly, the host (cell) may be transfected or transformed with the vector comprising a nucleic acid molecule coding for a polypeptide which is differentially regulated in diabetes as disclosed herein. The host cell or host may therefore be genetically modified with a nucleic acid molecule encoding such a polypeptide or with a vector comprising such a nucleic acid molecule. The term "genetically modified" means that the host cell or host comprises in addition to its natural genome a nucleic acid molecule or vector coding for a polypeptide listed in Table 1 or at least a fragment thereof. The additional genetic material may be introduced into the host (cell) or into one of its predecessors/parents. The nucleic acid molecule or vector may be present in the genetically modified host cell or host either as an independent molecule outside the genome, preferably as a molecule which is capable of replication, or it may be stably integrated into the genome of the host cell or host.

As mentioned herein above, the host cell of the present invention may be any prokaryotic or eukaryotic cell.

Suitable prokaryotic cells are those generally used for cloning like E. coli or Bacillus subtilis. Yet, these prokaryotic host cells are also envisaged in the screening methods disclosed herein. Furthermore, eukaryotic cells comprise, for example, fungal or animal cells. Examples for suitable fungal cells are yeast cells, preferably those of the genus Saccharomyces and most preferably those of the species Saccharomyces cerevisiae. Suitable animal cells are, for instance, insect cells, vertebrate cells, preferably mammalian cells, such as e.g. CHO, HeLa, NIH3T3 or MOLT-4. Further suitable cell lines known in the art are obtainable from cell line depositories, like the American Type Culture Collection (ATCC).

The hosts may also be selected from non-human mammals, most preferably mice, rats, sheep, calves, dogs, monkeys or apes. As described herein above, the animals/mammals also comprise non-human transgenic animals, which preferably express at least one polypeptide differentially regulated in diabetes as disclosed herein. Preferably, the polypeptide is a polypeptide which is regulated in tissue derived from patients with diabetes. Yet it is also envisaged that non-human transgenic animals be produced which do not express marker genes as disclosed herein or who express limited amounts of the marker gene products. The animals are preferably related to polypeptides which are down-regulated in diabetes. Transgenic non-human animals comprising and/or expressing the up-regulated polypeptides of the present invention or alternatively, which comprise silenced or less efficient versions of down-regulated polypeptides, are useful models for studying the development of diabetes and provide for useful models for testing drugs and therapeutics for diabetes treatment and/or prevention.

A compound which interacts with a polypeptide listed in table 1 and which inhibits or antagonizes the polypeptide is identified by determining the activity of the polypeptide in the presence of the compound.

The term "activity" as used herein relates to the functional property or properties of a specific polypeptide. For the enzymes, the term "activity" relates to the enzymatic activity of a specific polypeptide. For adhesion molecules, the term "activity" relates to the adhesive properties of a polypeptide and may be determined using assays such as, but not limited to, adhesion assays, cell spreading assays, or in vitro interaction of the adhesion molecule with a known ligand. For cytoskeletal proteins, the term "activity" relates to the regulation of the cytoskeleton by such polypeptides, or to their incorporation into the cytoskeleton. As a non-limiting example, the ability of Gelsolin to regulate actin polymerization, or of Filamin A to promote orthogonal branching of actin filaments, may be determined using in vitro actin polymerization assays. Activity in relation to the regulation of cytoskeletal structures may further be determined by, as non-limiting examples, cell spreading assays, cell migration assays, cell proliferation assays or immunofluorescence assays, or by staining actin filaments with fluorescently labeled phalloidin. For ion channels the term "activity" relates to ion flux (Chloride flux) across the membrane. For transcription factors, the term "activity" relates to their ability to regulate gene transcription. The transcriptional activity of a gene can be determined using commonly used assays, such as a reporter gene assay. For growth factors and hormones or their receptors, the term "activity" relates to their ability to bind to their receptors or ligands, respectively, and to induce receptor activation and subsequent signaling cascades, and/or it relates to the factor's or receptor's ability to mediate the cellular function or functions eventually caused by growth factor or hormone mediated receptor activation. Growth factor or hormone binding to receptors can be determined by commonly known ligand binding assays. Receptor activation can be determined by testing for receptor autophosphorylation, or by assaying for modification or recruitment of downstream signaling mediators to the receptors (by immunoprecipitation and Western Blotting of signaling complexes). Cellular functions regulated by growth factors or hormones and their receptors can be cell proliferation (eg determined by using thymidine incorporation or cell counts), cell migration assays (eg determined by using modified Boyden chambers), cell survival or apoptosis assays (eg determined by using DAPI staining), angiogenesis assays (eg in vitro assays to measure endothelial tube formation that are commercially available). In addition to these assays, other assays may be used as well to determine these and other cellular functions.

Inhibitors, antagonists, activators or agonists as identified and/or obtained by the methods of the present invention are particularly useful in the therapeutic management, prevention and/or treatment of diabetes.

Inhibitors or antagonists of a polypeptide listed in Table 1 may be identified by the screening method described above when there is a decreased activity determined in the presence of the compound in comparison to the absence of the compound in the screening method, which is indicative for an inhibitor or antagonist.

Therefore, potential inhibitors or antagonists to be identified, screened for and/or obtained with the method of the present invention include molecules, preferably small molecules which bind to, interfere with and/or occupy relevant sites on the expressed marker genes which are up-regulated in tissues or cells derived from diabetic or pre-diabetic patients or individuals susceptible to diabetes.

It is furthermore envisaged that such inhibitors interfere with the synthesis/production of (functional) up-regulated marker genes or gene products, like, e.g. anti-sense constructs, ribozymes and the like. The inhibitors and/or antagonist which can be screened for and obtained in accordance with the method of the present invention include, inter alia, peptides, proteins, nucleic acids including DNA, RNA, RNAi, PNA, ribozymes, antibodies, small organic compounds, small molecules, ligands, and the like.

Accordingly, the inhibitor and/or antagonist of differentially expressed marker genes may comprises (an) antibody(ies). The antibody(ies) may comprise monoclonal antibodies as well as polyclonal antibodies. Furthermore, chimeric antibodies, synthetic antibodies as well as antibody fragments (like Fab, F(ab)2, Fv, scFV), or a chemically modified derivative of antibodies are envisaged. It is envisaged that the antibodies bind to the marker gene or its gene product and/or interfere its activity.

In addition, oligonucleotides and/or aptamers which specifically bind to the marker genes as defined herein or which interfere with the activity of the marker genes are envisaged as inhibitors and/or antagonists. The term "oligonucleotide" as used in accordance with the present invention comprises coding and non-coding sequences, it comprises DNA and RNA and/or comprises also any feasible derivative. The term "oligonucleotide" further comprises peptide nucleic acids (PNAs) containing DNA analogs with amide backbone linkages (Nielson, Science 274 (1991), 1497-1500). Oligonucleotides which may inhibit and/or antagonize the marker gene activity and which can be identified and/or obtained by the method of the present invention can be, inter alia, easily chemically synthesized using synthesizers which are well known in the art and are commercially available like, e.g., the ABI 394 DNA-RNA Synthesizers. Additionally, the use of synthetic small interfering dsRNAs of ~22 nt (siRNAs) may be used for suppressing gene expression.

Further to the screening methods disclosed above, this invention provides a screening method for identifying and/or obtaining a compound which is an inhibitor of the expression of a polypeptide listed in table 1 whose expression is regulated in diabetes, comprising the steps of a) contacting a host which expresses the polypeptide with a compound; b) determining the expression level and/or activity of the polypeptide; c) determining the expression level and/or activity of the polypeptide in the host as defined in (a), which has not been contacted with the compound; and d) quantitatively relating the expression level of the polypeptide as determined in (b) and (c), wherein a decreased expression level determined in (b) in comparison to (c) is indicative for an inhibitor of the expression of the polypeptide.

An inhibitor of the expression of a polypeptide listed in table 1 is identified by the screening method described hereinbefore when a decreased expression of the protein is determined in the presence of the compound in comparison to the absence of the compound in the screening method, which is indicative for an inhibitor of expression of a polypeptide.

The term "express" as used herein relates to expression levels of a polypeptide listed in table 1 which is regulated in diabetes. Preferably, expression levels are at least 2 fold, more preferably at least 3 fold, even more preferably at least 4 fold, most preferably at least 5 fold higher in diabetic adipose tissue cells than in healthy adipose tissue cells.

Furthermore, the present invention provides a compound identified and/or obtained by any of the screening methods hereinbefore described. The compound is further comprised in a pharmaceutical composition. Any conventional carrier material can be utilized. The carrier material can be an organic or inorganic one suitable for eteral, percutaneous or parenteral administration. Suitable carriers include water, gelatin, gum arabic, lactose, starch, magnesium stearate, talc, vegetable oils, polyalkylene-glycols, petroleum jelly and the like. Furthermore, the pharmaceutical preparations may contain other pharmaceutically active agents. Additional additives such as flavoring agents, stabilizers, emulsifying agents, buffers and the like may be added in accordance with accepted practices of pharmaceutical compounding.

The compound may be used for the preparation of a medicament for the treatment or prevention of diabetes. In addition, the compound may also be used for the preparation of a diagnostic composition for diagnosing diabetes or a predisposition for diabetes. Preferably, the compound comprises an antibody, an antibody-derivative, an antibody fragment, a peptide or an antisense construct.

Within the scope of the present invention, antibodies against the proteins listed in table 1, or antigen-binding fragments thereof, may be used in an in vitro method for the diagnosis of diabetes.

In order to efficiently perform diagnostic screenings, the present invention provides a kit for the diagnosis of early diabetes (pre-diabetes), susceptibility to diabetes, or diabetes comprising one or more of the antibodies, or antigen-binding fragments thereof, described above. Another kit provided by this invention is a kit for the diagnosis of pre-diabetes, susceptibility to diabetes, or diabetes comprising one or more of the nucleic acids coding for the marker hereinbefore described. Yet another kit provided by this invention is a kit for screening of compounds that agonize or antagonize any of the polypeptides listed in table 1, or inhibit the expression of any of the polypeptides.

As mentioned herein above, the inhibitor and/or antagonist may also comprise small molecules. Small molecules, however may also be identified as activators or agonists by the herein disclosed methods. The term "small molecule" relates, but is not limited to small peptides, inorganic and/or organic substances or peptide-like molecules, like peptide-analogs comprising D-amino acids.

Furthermore, peptidomimetics and/or computer aided design of appropriate antagonist, inhibitors, agonists or activators may be employed in order to obtain candidate compounds to be tested in the inventive method. Appropriate computer systems for the computer aided design of, e.g., proteins and peptides are described in the prior art, for example, in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used in combination with the method of the invention for, e.g., optimizing known compounds, substances or molecules. Appropriate compounds can also be identified by the synthesis of peptidomimetic combinatorial libraries through successive chemical modification and testing the resulting compounds, e.g., according to the methods described herein. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715. Furthermore, the three-dimensional and/or crystallographic structure of inhibitors activators, agonists or activators of the markers of the present invention or of the nucleic acid molecule encoding the expressed markers can be used for the design of peptidomimetic inhibitors, antagonists, agonists or activators to be tested in the method of the invention (Rose, Biochemistry 35 (1996), 12933-12944; Rutenber, Bioorg. Med. Chem. 4 (1996), 1545-1558).

The compounds to be screened with the method(s) of the present invention do not only comprise single, isolated compounds. It is also envisaged that mixtures of compounds are screened with the method of the present invention. It is also possible to employ extracts, like, inter alia, cellular extracts from prokaryotic or eukaryotic cells or organisms.

In addition, the compound identified or refined by the inventive method can be employed as a lead compound to achieve, modified site of action, spectrum of activity, organ specificity, and/or improved potency, and/or decreased toxicity (improved therapeutic index), and/or decreased side effects, and/or modified onset of therapeutic action, duration of effect, and/or modified pharmakinetic parameters (resorption, distribution, metabolism and excretion), and/or modified physico-chemical parameters (solubility, hygroscopicity, color, taste, odor, stability, state), and/or improved general specificity, organ/tissue specificity, and/or optimized application form and route may be modified by esterification of carboxyl groups, or esterification of hydroxyl groups with carbon acids, or esterification of hydroxyl groups to, e.g. phosphates, pyrophosphates or sulfates or hemi succinates, or formation of pharmaceutically acceptable salts, or formation of pharmaceutically acceptable complexes, or synthesis of pharmacologically active polymers, or introduction of hydrophylic moieties, or introduction/exchange of substituents on aromates or side chains, change of substituent pattern, or modification by introduction of isosteric or bioisosteric moieties, or synthesis of homologous compounds, or introduction of branched side chains, or conversion of alkyl substituents to cyclic analogues, or derivatisation of hydroxyl group to ketales, acetales, or N-acetylation to amides, phenylcarbamates, or synthesis of Mannich bases, imines, or transformation of ketones or aldehydes to Schiffs bases, oximes, acetales, ketales, enolesters, oxazolidines, thiozolidines or combinations thereof.

Additionally, the invention provides for the use of a compound or a plurality of compounds which is obtainable by the method disclosed herein for the preparation of a diagnostic composition for diagnosing pre-diabetes, diabetes or a predisposition for diabetes. It is, for example envisaged that specific antibodies, fragments thereof or derivatives thereof which specifically detect or recognize differentially expressed marker gene products as disclosed herein be employed in such diagnostic compositions. Yet, specific primers/primer pairs which may detect and/or amplify the marker gene of the present invention may be employed in the diagnostic compositions.

The present invention provides the use of a therapeutically effective amount of at least one antibody against at least one protein, or antigen-binding fragment thereof, selected from the group consisting of the proteins having SEQ ID Nos. 2, 4, 6, 8, 10, 12, 14, 16, 18, 20, 22, 24, 26, 28, 30, 32 and 34 for the preparation of a medicament for the treatment of diabetes and pre-diabetes. Furthermore, the present invention also provides the use of a therapeutically effective amount of at least one protein, protein fragment or peptide selected from the group consisting of the proteins having SEQ ID Nos. 2, 4, 6, 8, 10, 12, 14, 16, 18, 20, 22, 24, 26, 28, 30, 32 and 34 for the preparation of a medicament for the treatment of diabetes or pre-diabetes.

Accordingly, the compound to be used in the pharmaceutical as well as in the diagnostic composition may comprise an antibody, an antibody-derivative, an antibody fragment, a peptide or a nucleic acid, like primers/primer pairs as well as anti-sense constructs, RNAi or ribozymes.

The diagnostic composition may also comprise suitable means for detection known in the art.

The invention is further described by reference to the following biological examples which are merely illustrative and are not to be construed as a limitation of scope.

Also claimed are the methods and kits hereinbefore described, especially with reference to the following examples.

## Examples:

Total RNA was extracted using Ultraspec@ RNA (Biotecx, Houston, TX) according to the manufacturer's protocol, and purified using the RNeasy Mini kit (Qiagen, Valencia, CA) with DNase treatment. Double-stranded cDNA was synthesized from 10 ug total RNA by SuperScript$^{TM}$ Double-Stranded cDNA Synthesis Kit (Life Technology, Rockville, MD) using the T7-T24 primer. The double-stranded cDNA product was purified by phenol/chloroform/isoamyl extraction using phase lock gels (Eppendorf, Westbury, NY). Double-stranded cDNA was further converted into cRNA using the *in vitro* transcription (IVT) MEGAscript$^{TM}$ T7 kit (Ambion, Austin, TX) and labelled with biotinylated

nucleotides[1]. The *in vitro* transcription product was purified using the RNeasy Mini kit (Qiagen, Valencia, CA), and fragmented as described (Wodicka L, Dong H, Mittmann M, Ho MH, Lockhart DJ. Genome-wide expression monitoring in Saccharomyces cerevisiae. Nat Biotechnol 1997;15:1359-67). Hybridization of the fragmented *in vitro* transcription product to the Human Genome U95 (HG-U95) Genechip@ array set was performed as suggested by the manufacturer (Affymetrix, Santa Clara, CA).

Statistical Methods

All numeric analyses were conducted on signal intensities as reported by the Affymetrix's MAS algorithms (Affymetrix Technical Note: New Statistical Algorithms for Monitoring Gene Expression on GeneChip® Probe Arrays. (2001)). Chips were each standardized to the overall mean of the all of the chips in the experiment. Genes were not separately standardized.

The analysis of the data was constructed as a linear model (Draper N., Smith H. Applied Regression Analysis, Second Edition John Wiley and Sons. New York, New York. (1966); Searle S. R. Linear Models John Wiley and Sons. New York, New York. (1971)) with factors for BMI, tissue of origin (subcutaneous vs. visceral adipose), insulin resistance (measured by HOMA), fasting glucose, fasting insulin and the interactions between tissue of origin and fasting glucose, fasting insulin, and insulin resistance respectively. Calculations were done using SAS version 8.1. The equation for the model is as follows:

Signal Intensity = BMI + tissue + IR + glucose + insulin + tissue*IR + tissue*gluocose + tissue*insulin + error

Nine statistical tests (contrasts) were then performed using this model. 1) Effect in visceral adipose; 2) Effect in subcutaneous adipose; 3) Differential effect between visceral and subcutaneous adipose. Each of those three tests was performed with the three interaction terms resulting in the final 9 tests.

Results of the model calculations and statistical contrasts were then filtered to result in the final genes of interest. Significance was defined as a p-value for the entire model less than 0.001 and a p-value for the specific contrast of less than 0.01. The p-value cutoffs were chosen so as to control for false positives while still finding the majority of true positives (Sokal R. R., Rohlf F. J. Biometry W. H. Freeman and Company. New York, New York. (1969)).

Finally genes were annotated through linking the Genbank accession numbers provided by Affymetrix with the Unigene http://www.ncbi.nlm.nih.gov /entrez/query.fcgi?db=unigene) and LocusLink (http://www.ncbi.nlm.nih.gov /LocusLink/) annotations for those accession numbers.

All references discussed throughout the above specification are herein incorporated in their entirety by reference for the subject matter they contain.

It should be understood, of course, that the foregoing relates to preferred embodiments of the invention and that modifications may be made without departing from the spirit and scope of the invention as set forth in the following claims.

## Claims

1. A method for the diagnosis of pre-diabetes, diabetes or the susceptibility to diabetes comprising:

    obtaining a biological sample; and
    detecting or measuring the level of a polypeptide marker, said polypeptide marker comprising at least one polypeptide selected from the group consisting of the polypeptides having SEQ ID Nos. 2, 4, 6, 8, 10, 12, 14, 16, 18, 20, 22, 24, 26, 28, 30, 32 and 34.

2. A method for the diagnosis of pre-diabetes, diabetes or the susceptibility to diabetes comprising detecting or measuring the level of a polypeptide marker in a biological sample, said polypeptide marker comprising at least one polypeptide selected from the group consisting of the polypeptides having SEQ ID Nos. 2, 4, 6, 8, 10, 12, 14, 16, 18, 20, 22, 24, 26, 28, 30, 32 and 34.

3. The method of any one of claims claim 1 or 2, wherein said polypeptide marker comprises at least two polypeptides.

4. The method of any one of claims 1 to 3, wherein said biological sample is derived from the group consisting of serum, plasma, and cells of adipose tissue.

5. The method of any one of claims 1 to 4, wherein the level of said polypeptide marker in an individual suspected to suffer from pre-diabetes, diabetes or to be susceptible to diabetes is compared to the expression levels of the same polypeptide marker in a healthy individual.

6. The in vitro method of any one of claims 1 to 5, wherein an increase or a decrease of the level of said polypeptide marker over time is indicative of pre-diabetes, diabetes or the susceptibility to diabetes.

7. A method for the diagnosis of pre-diabetes, diabetes or the susceptibility to diabetes comprising:

    obtaining a biological sample; and
    detecting or measuring the level of a marker, said nucleic acid marker comprising at least one nucleic acid molecule selected from the group consisting of the nucleic acid molecules of SEQ ID Nos. 1, 3, 5, 7, 9, 11, 13, 15, 17, 19, 21, 23, 25. 27, 29, 31 and 33.

8. A method for the diagnosis of pre-diabetes, diabetes or the susceptibility to diabetes comprising detecting or measuring the level of a marker in a biological sample, said nucleic acid marker comprising at least one nucleic acid molecule selected from the group consisting of the nucleic acid molecules of SEQ ID Nos. 1, 3, 5, 7, 9, 11, 13, 15, 17, 19, 21, 23, 25. 27, 29, 31 and 33.

9. The method of any one of claims 7 or 8, wherein said nucleic acid marker is RNA.

10. The method any one of claims 7 to 9, wherein the expression level of said nucleic acid marker in an individual suspected to suffer from pre-diabetes, diabetes or to be susceptible to diabetes is compared to the expression levels of the same nucleic acid marker in a healthy individual.

11. The in vitro method of any one of claims 7 to 10, wherein an increase or decrease of the expression levels of said nucleic acid marker over time is indicative of pre-diabetes, diabetes or the susceptibility to diabetes.

**12.** A screening method for identifying a compound which interacts with a polypeptide whose expression is regulated in diabetes, said polypeptide being selected from the group consisting of the polypeptides having SEQ ID Nos. 2, 4, 6, 8, 10, 12, 14, 16, 18, 20, 22, 24, 26, 28, 30, 32 and 34, comprising:

contacting said polypeptide with a compound or a plurality of compounds under conditions which allow interaction of said compound with said polypeptide; and
detecting the interaction between said compound or plurality of compounds with said polypeptide.

**13.** A screening method for identifying a compound which is an agonist or an antagonist of a polypeptide whose expression is regulated in diabetes, said polypeptide being selected from the group consisting of the polypeptides having SEQ ID Nos. 2, 4, 6, 8, 10, 12, 14, 16, 18, 20, 22, 24, 26, 28, 30, 32 and 34, comprising:

contacting said polypeptide with a compound under conditions which allow interaction of said compound with said polypeptide;
determining a first level of activity of said polypeptide;
determining a second level of activity of said polypeptide expressed in a host which has not been contacted with said compound; and
quantitatively relating the first level of activity with the second level of activity, wherein when said first level of activity is less than said second level of activity, said compound is identified as an agonist or antagonist of said polypeptide.

**14.** A screening method for identifying a compound which is an inhibitor of the expression of a polypeptide whose expression is upregulated in diabetes, said polypeptide being selected from the group consisting of the polypeptides having SEQ ID Nos. 2, 4, 6, 8, 10, 12, 14, 16, 18, 20, 22, 24, 26, 28, 30, 32 and 34, comprising:

contacting a host which expresses said polypeptide with a compound;
determining a first expression level or activity of said polypeptide;
determining a second expression level or activity of said polypeptide in a host which has not been contacted with said compound; and
quantitatively relating the first expression level or activity with the second expression level or activity, wherein when said first expression level or activity is less than said second expression level or activity, said compound is identified as an inhibitor of the expression of said polypeptide.

**15.** Antibodies against the proteins, or antigen-binding fragments thereof, for the use in an in vitro method for the diagnosis of pre-diabetes, diabetes or susceptibility to diabetes, said proteins being selected from the group consisting of the proteins having SEQ ID Nos. 2, 4, 6, 8, 10, 12, 14, 16, 18, 20, 22, 24, 26, 28, 30, 32 and 34.

**16.** A method of correlating protein levels in a mammal with a diagnosis of pre-diabetes, diabetes or susceptibility to develop diabetes, comprising:

selecting one or more proteins selected from the group consisting of the proteins having SEQ ID Nos. 2, 4, 6, 8, 10, 12, 14, 16, 18, 20, 22, 24, 26, 28, 30, 32 and 34;
determining the level of said one or more proteins in said mammal; and
generating an index number, Y, which indicates a presence of diabetes or a susceptibility thereto.

**17.** The method according to claim 16, further comprising comparing index number, Y, to index numbers of known diabetics and non-diabetics.

**18.** The method according to claim 16, further comprising monitoring said index number, Y, over time to determine the stage of diabetes or susceptibility thereto.

**19.** A kit for the diagnosis of diabetes and pre-diabetes comprising one or more of the antibodies, or antigen-binding fragments thereof, of claim 15.

**20.** A kit for the diagnosis of diabetes and pre-diabetes comprising one or more of the nucleic acids coding for the polypeptide marker of claims 1.

**21.** A kit for screening of compounds that activate or inhibit a polypeptides or stimulate or inhibit the expression of any of said polypeptides, said polypeptides being selected from the group consisting of the polypeptides having SEQ ID Nos. 2, 4, 6, 8, 10, 12, 14, 16, 18, 20, 22, 24, 26, 28, 30, 32 and 34.

**22.** A method for monitoring serum levels of one or more proteins to detect a pre-diabetic disease state, a diabetic disease state or a susceptibility to develop a diabetic disease state, said method comprising:

raising antibodies of said one or more proteins;
detecting the serum level of said proteins; and
comparing said serum level to those of known diabetics and known non-diabetics.

**23.** A method for treating diabetes and pre-diabetes comprising administering, to a patient in need thereof, a therapeutically effective amount of at least one antibody against at least one protein, or antigen-binding fragment thereof, selected from the group consisting of the proteins having SEQ ID Nos. 2, 4, 6, 8, 10, 12, 14, 16, 18, 20, 22, 24, 26, 28, 30, 32 and 34.

**24.** Use of a therapeutically effective amount of at least one antibody against at least one protein, or antigen-binding fragment thereof, selected from the group consisting of the proteins having SEQ ID Nos. 2, 4, 6, 8, 10, 12, 14, 16, 18, 20, 22, 24, 26, 28, 30, 32 and 34 for the preparation of a medicament for the treatment of diabetes and pre-diabetes.

**25.** Use of a therapeutically effective amount of at least one protein, protein fragment or peptide selected from the group consisting of the proteins having SEQ ID Nos. 2, 4, 6, 8, 10, 12, 14, 16, 18, 20, 22, 24, 26, 28, 30, 32 and 34 for the preparation of a medicament for the treatment of diabetes or pre-diabetes.

**26.** The methods and kits hereinbefore described, especially with reference to the foregoing examples.

Figure 1
Follistatin-like 3

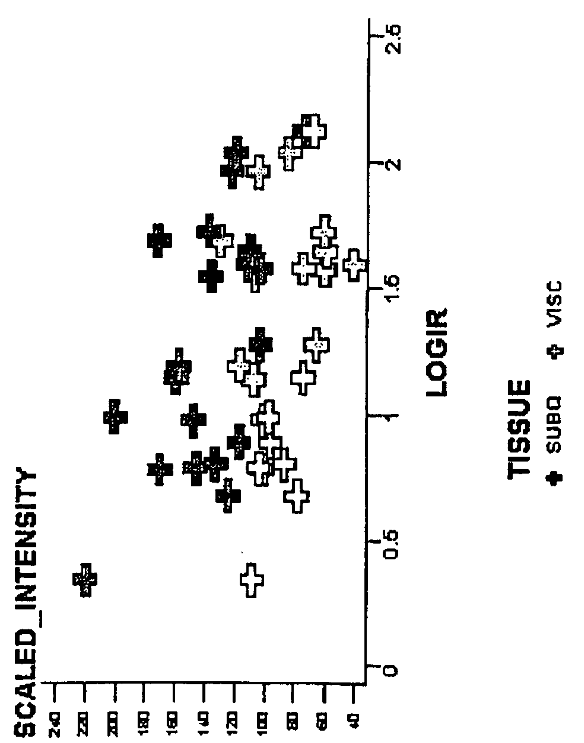


Figure 2
Neuronatin

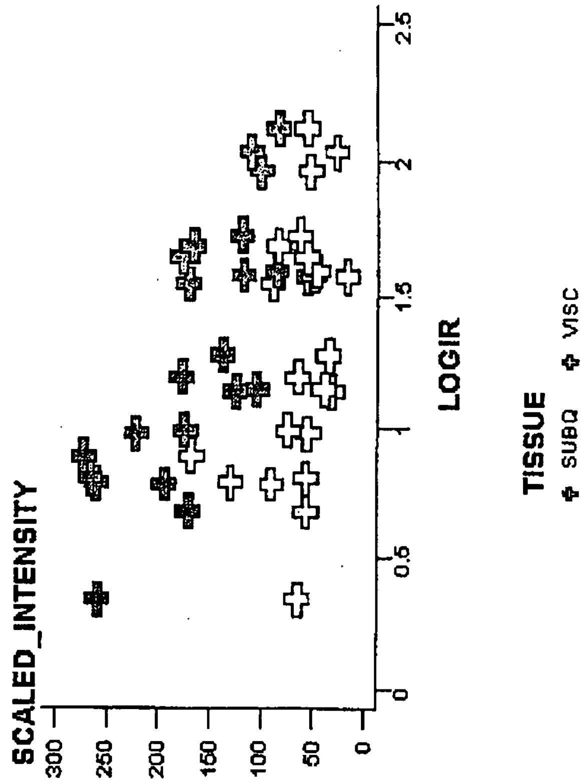

Figure 3
Apolipoprotein D

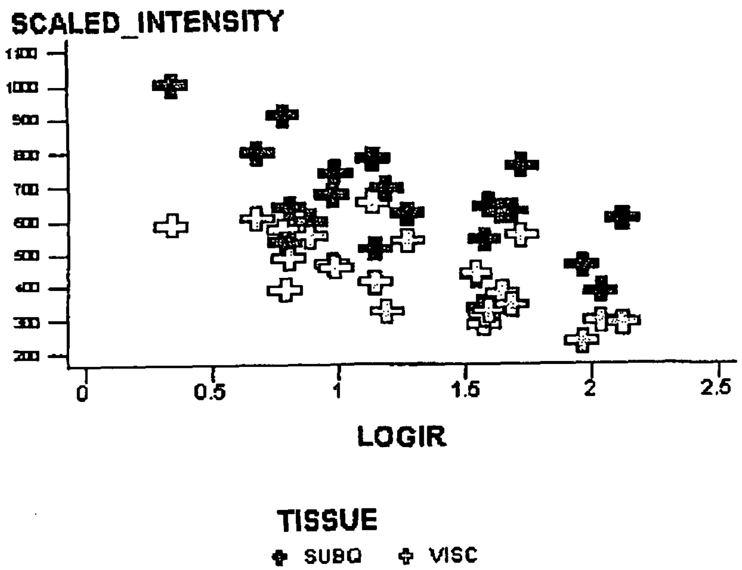


Figure 4
vascular endothelial growth factor B

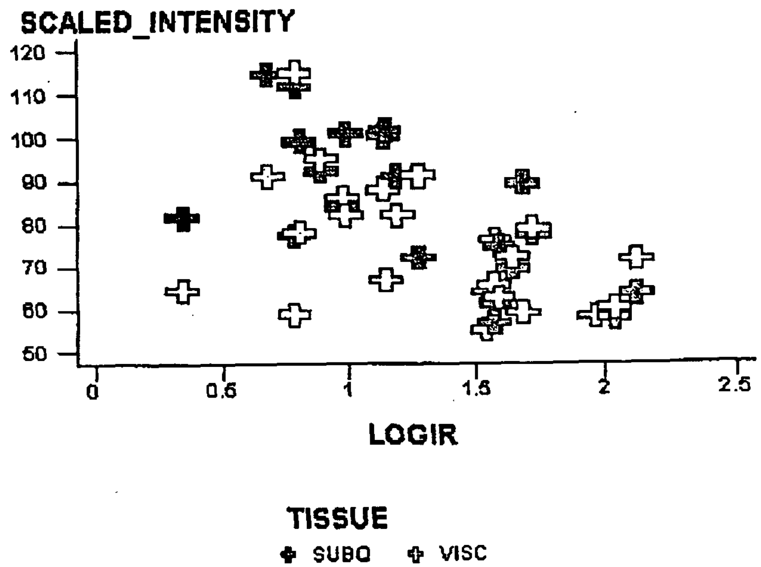

**Figure 5**
**Annexin A11**

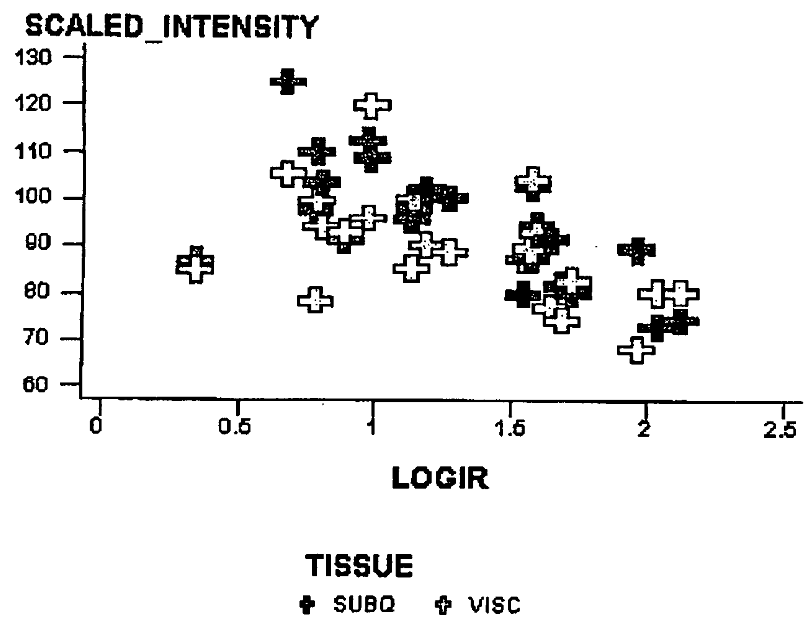


Figure 6
superoxide dismutase 3. extracellular

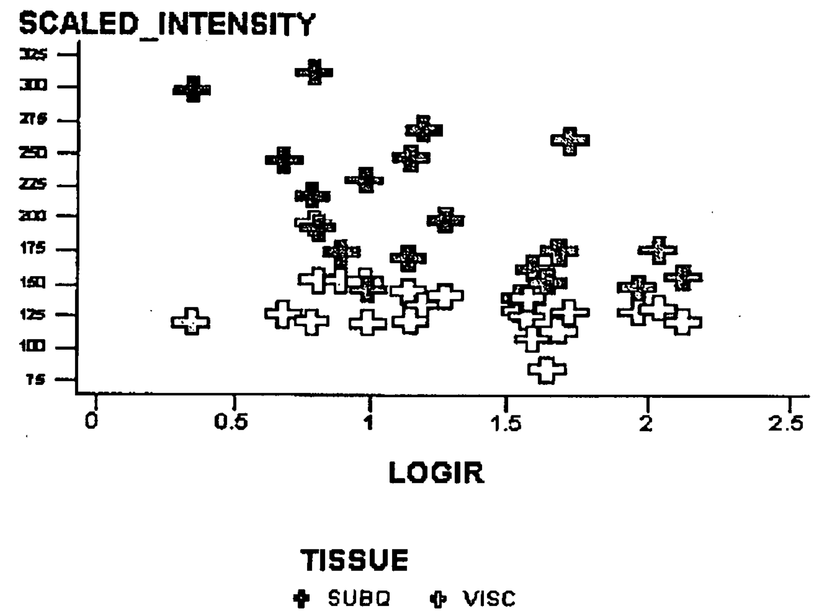

Figure 7
Endothelial cell growth factor

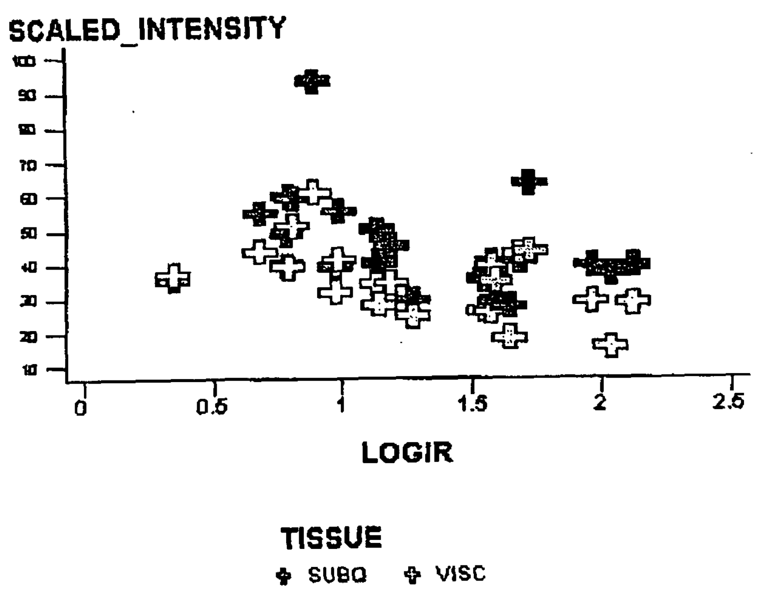


Figure 8
amine oxidase, copper containing 3 (vascular adhesion protein 1)

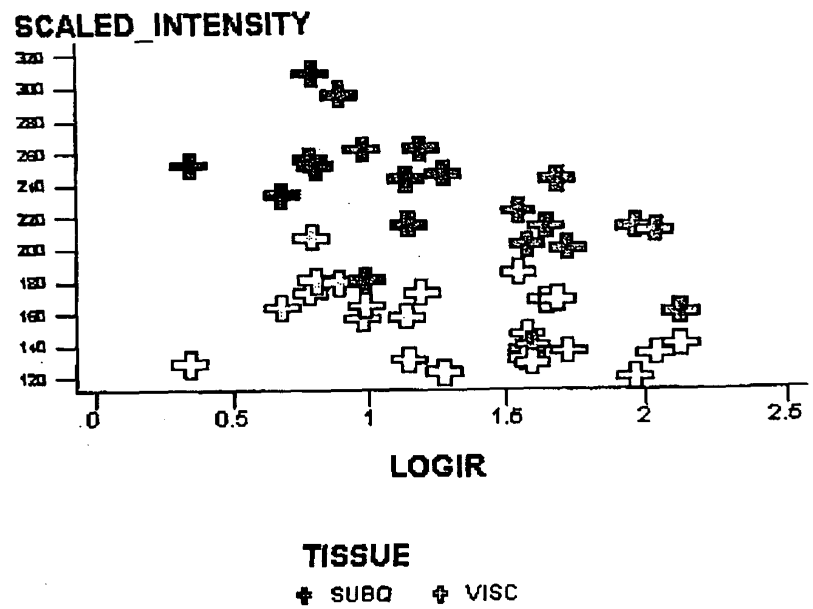

# Figure 9
## Dipeptidylpeptidase IV

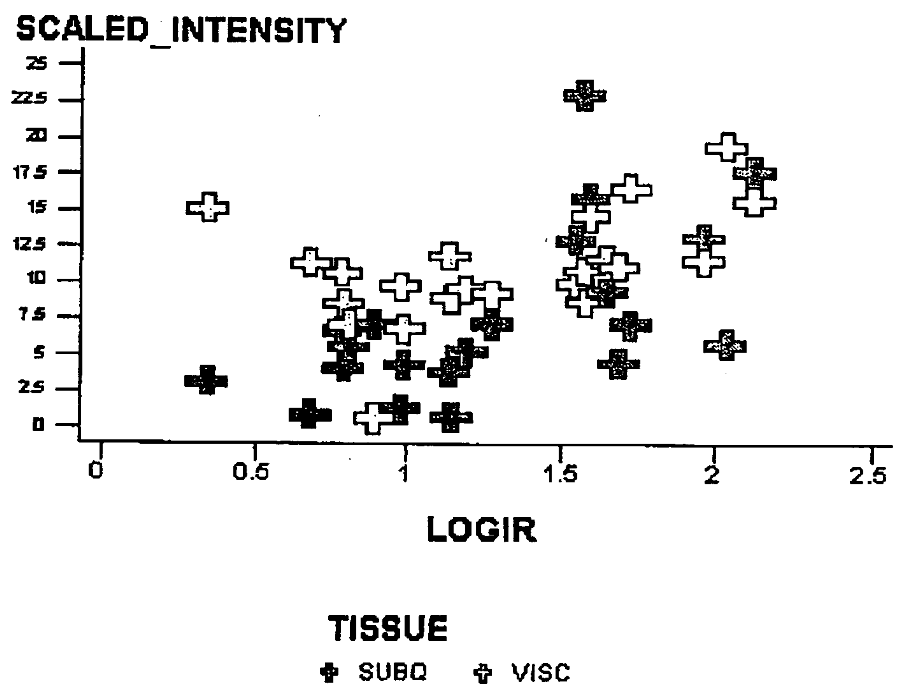

Figure 10
H factor 1 (complement)

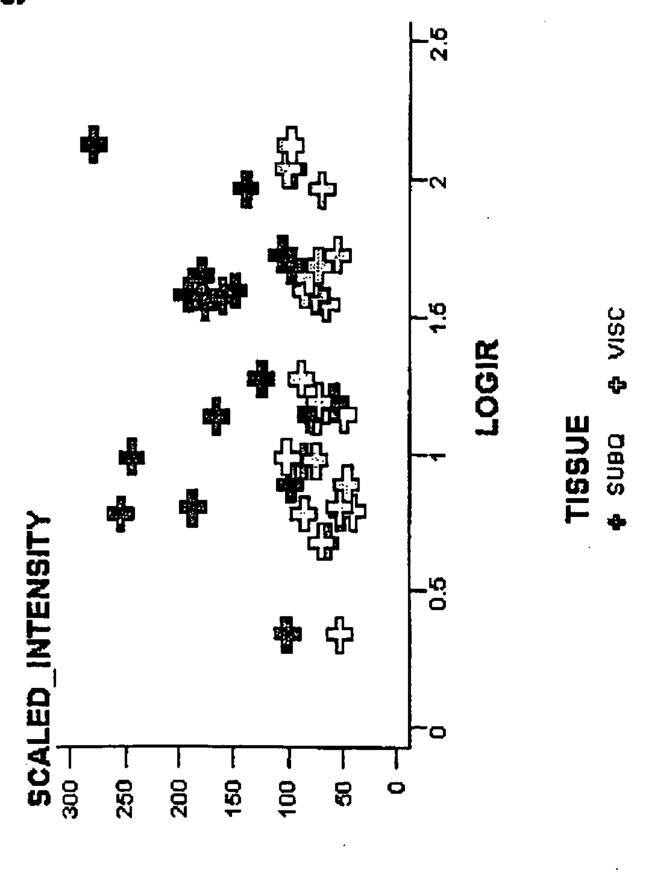


Figure 11
Fibroblast Growth Factor 2

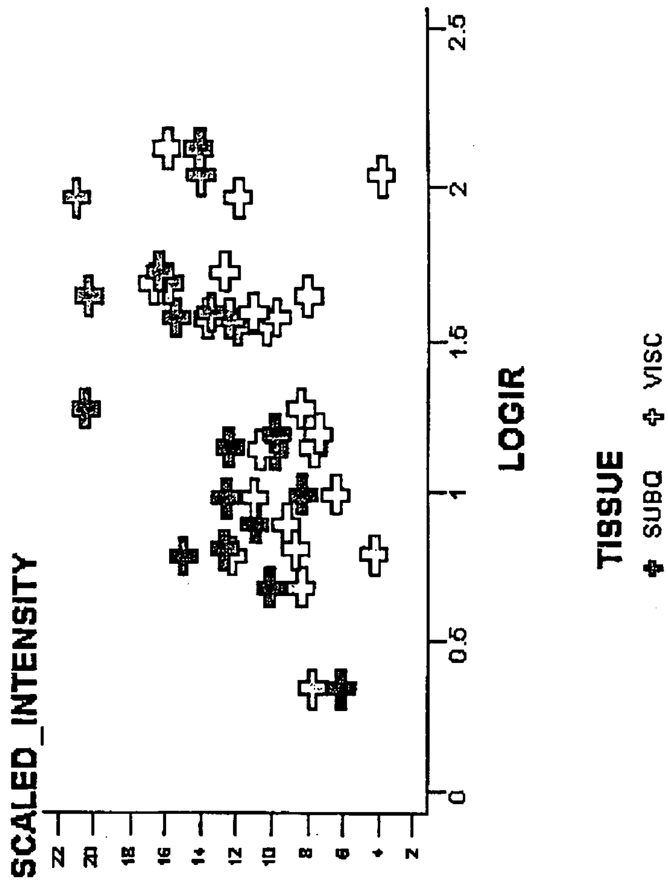

Figure 13
SPUVE

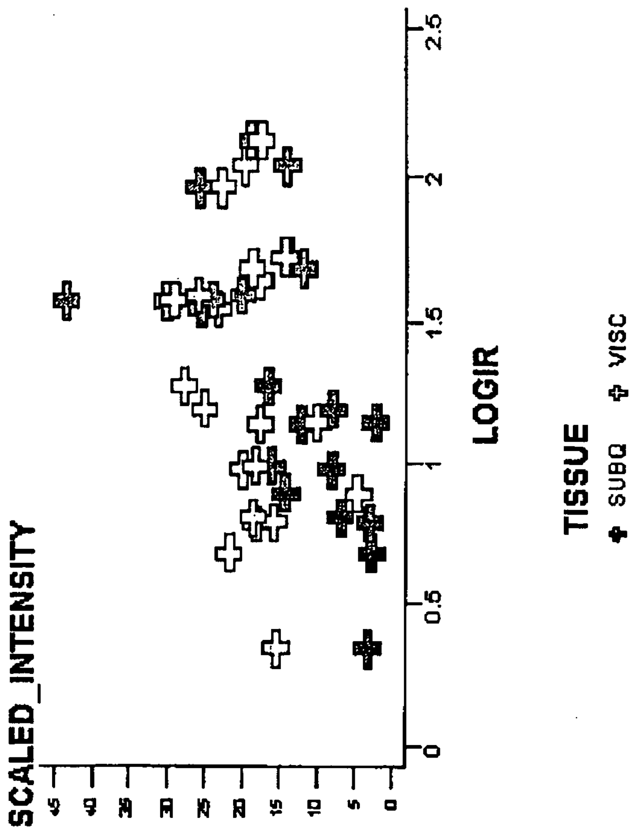


Figure 12
fibulin 1

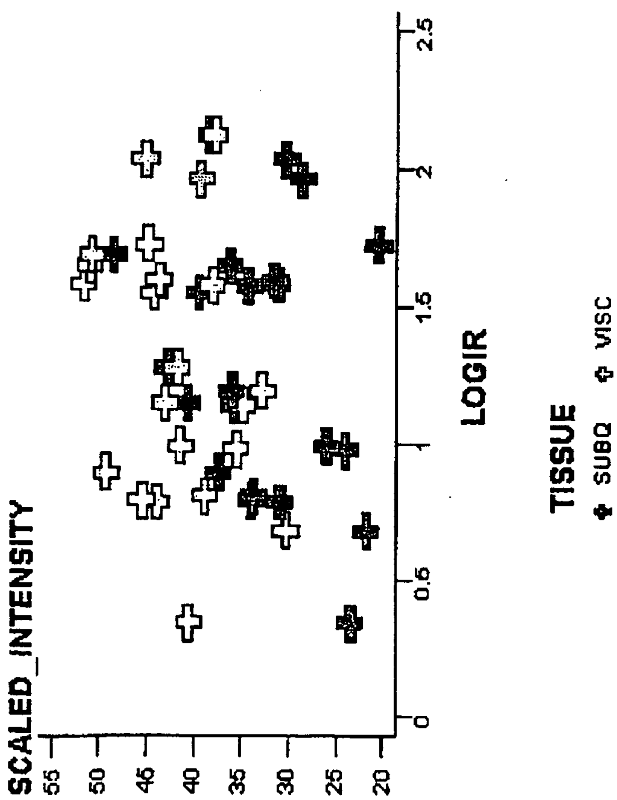

Figure 14
thrombospondin 2

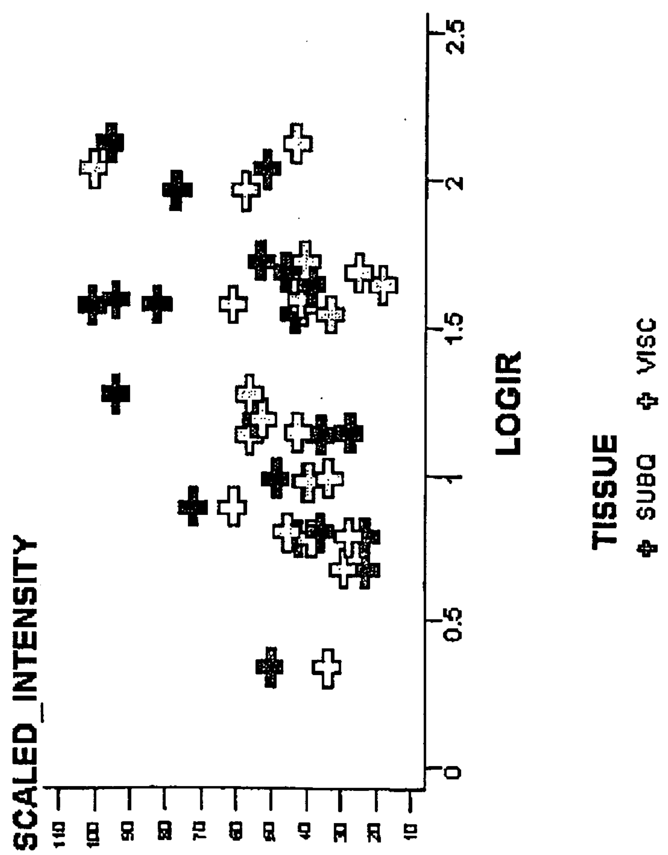


Figure 15
protein S (alpha)

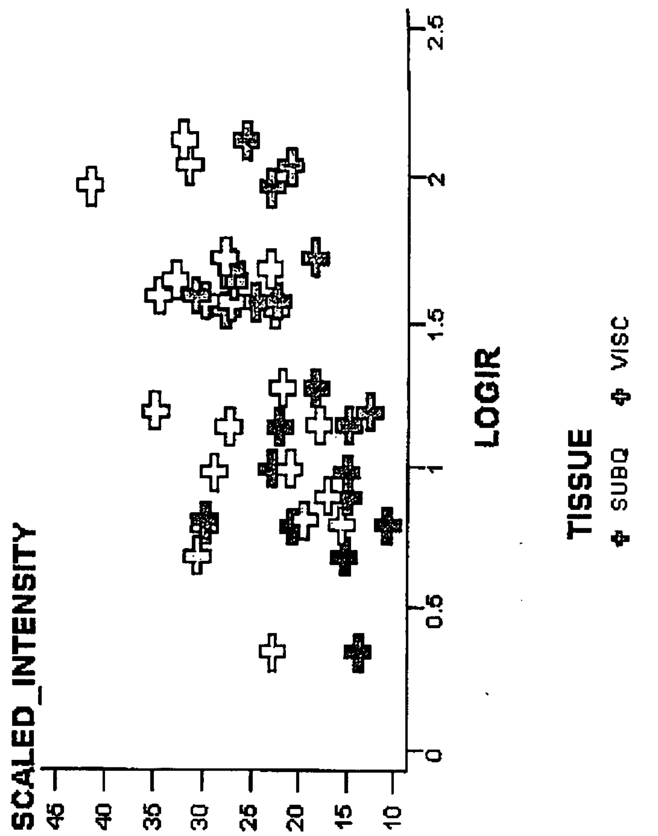

# Figure 16
# Annexin A2

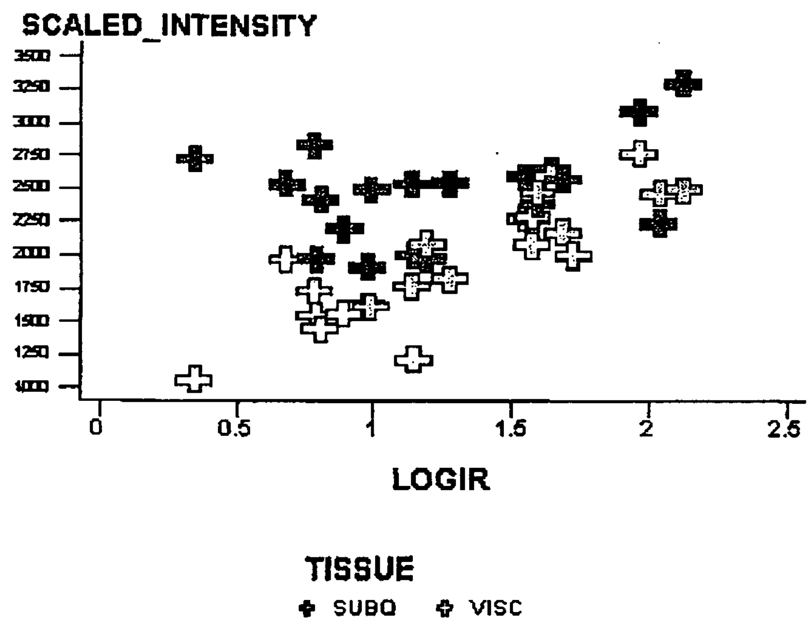


# Figure 17
# Lysyl Oxidase

SEQUENCE LISTING

<110> F. Hoffmann-La Roche AG

<120> Specific markers for diabetes

<130> 21270

<160> 34

<170> PatentIn version 3.2

<210> 1

<211> 1172

<212> DNA

<213> Homo sapiens

<220>

<221> VEGF-B mRNA

<222> (1)..(1172)

<400> 1

gcgatgcggg cgcccccggc gggcggcccc ggcgggcacc atgagccctc tgctccgccg 60

cctgctgctc gccgcactcc tgcagctggc ccccgcccag gcccctgtct cccagcctga 120

tgcccctggc caccagagga aagtggtgtc atggatagat gtgtatactc gcgctacctg 180

ccagccccgg gaggtggtgg tgcccttgac tgtggagctc atgggcaccg tggccaaaca 240

gctggtgccc agctgcgtga ctgtgcagcg ctgtggtggc tgctgccctg acgatggcct 300

ggagtgtgtg cccactgggc agcaccaagt ccggatgcag atcctcatga tccggtaccc 360

gagcagtcag ctgggggaga tgtccctgga agaacacagc cagtgtgaat gcagacctaa 420

aaaaaaggac agtgctgtga agccagacag ggctgccact ccccaccacc gtccccagcc 480
ccgttctgtt ccgggctggg actctgcccc cggagcaccc tccccagctg acatcaccca 540
tcccactcca gccccaggcc cctctgccca cgctgcaccc agcaccacca gcgccctgac 600
ccccggacct gccgctgccg ctgccgacgc cgcagcttcc tccgttgcca agggcggggc 660
ttagagctca acccagacac ctgcaggtgc cggaagctgc gaaggtgaca catggctttt 720
cagactcagc agggtgactt gcctcagagg ctatatccca gtgggggaac aaagaggagc 780
ctggtaaaaa acagccaagc cccaagacc tcagcccagg cagaagctgc tctaggacct 840
gggcctctca gagggctctt ctgccatccc ttgtctccct gaggccatca tcaaacagga 900
cagagttgga agaggagact gggaggcagc aagaggggtc acataccagc tcaggggaga 960
atggagtact gtctcagttt ctaaccactc tgtgcaagta agcatcttac aactggctct 1020
tcctcccctc actaagaaga cccaaacctc tgcataatgg gatttgggct ttggtacaag 1080
aactgtgacc cccaaccctg ataaaagaga tggaaggaaa aaaaaaaaaa aaaaaaaaaa 1140
aaaaaaaaaa aaaaaaaaaa aaaaaaaaaa aa 1172

<210> 2

<211> 207

<212> PRT

<213> Homo sapiens

<220>

<221> VEGF-B polypeptide sequence

<222> (1)..(207)

<400> 2

Met Ser Pro Leu Leu Arg Arg Leu Leu Leu Ala Ala Leu Leu Gln Leu
1 5 10 15

Ala Pro Ala Gln Ala Pro Val Ser Gln Pro Asp Ala Pro Gly His Gln
20 25 30

Arg Lys Val Val Ser Trp Ile Asp Val Tyr Thr Arg Ala Thr Cys Gln
35 40 45

Pro Arg Glu Val Val Val Pro Leu Thr Val Glu Leu Met Gly Thr Val
50 55 60

Ala Lys Gln Leu Val Pro Ser Cys Val Thr Val Gln Arg Cys Gly Gly
65 70 75 80

Cys Cys Pro Asp Asp Gly Leu Glu Cys Val Pro Thr Gly Gln His Gln
85 90 95

Val Arg Met Gln Ile Leu Met Ile Arg Tyr Pro Ser Ser Gln Leu Gly
100 105 110

Glu Met Ser Leu Glu Glu His Ser Gln Cys Glu Cys Arg Pro Lys Lys
115 120 125

Lys Asp Ser Ala Val Lys Pro Asp Arg Ala Ala Thr Pro His His Arg
130 135 140

Pro Gln Pro Arg Ser Val Pro Gly Trp Asp Ser Ala Pro Gly Ala Pro
145 150 155 160

Ser Pro Ala Asp Ile Thr His Pro Thr Pro Ala Pro Gly Pro Ser Ala
165 170 175

His Ala Ala Pro Ser Thr Thr Ser Ala Leu Thr Pro Gly Pro Ala Ala

180 185 190

Ala Ala Ala Asp Ala Ala Ala Ser Ser Val Ala Lys Gly Gly Ala

195 200 205

<210> 3

<211> 809

<212> DNA

<213> Homo sapiens

<220>

<221> apolipoprotein D mRNA

<222> (1)..(809)

<400> 3

atgcctgtct tcatcttgaa agaaaagctc caggtccctt ctccagccac ccagccccaa 60

gatggtgatg ctgctgctgc tgctttccgc actggctggc ctcttcggtg cggcagaggg 120

acaagcattt catcttggga agtgccccaa tcctccggtg caggagaatt ttgacgtgaa 180

taagtatctc ggaagatggt acgaaattga gaagatccca acaacctttg agaatggacg 240

ctgcatccag gccaactact cactaatgga aaacggaaag atcaaagtgt taaaccagga 300

gttgagagct gatggaactg tgaatcaaat cgaaggtgaa gccaccccag ttaacctcac 360

agagcctgcc aagctggaag ttaagttttc ctggtttatg ccatcggcac cgtactggat 420

cctggccacc gactatgaga actatgccct cgtgtattcc tgtacctgca tcatccaact 480

tttcacgtg gattttgctt ggatcttggc aagaaaccct aatctccctc cagaaacagt 540

ggactctcta aaaaatatcc tgacttctaa taacattgat gtcaagaaaa tgacggtcac 600

agaccaggtg aactgcccca agctctcgta accaggttct acagggaggc tgcacccact 660

ccatgttact tctgcttcgc tttcccctac cccacccccc cccataaaga caaaccaatc 720

aaccacgaca aaggaagttg acctaaacat gtaaccatgc cctaccctgt taccttgcta 780

gctgcaaaat aaacttgttg ctgacctgc 809

<210> 4

<211> 189

<212> PRT

<213> Homo sapiens

<220>

<221> apolipoprotein D polypeptide sequence

<222> (1)..(189)

<400> 4

Met Val Met Leu Leu Leu Leu Leu Ser Ala Leu Ala Gly Leu Phe Gly

1 5 10 15

Ala Ala Glu Gly Gln Ala Phe His Leu Gly Lys Cys Pro Asn Pro Pro

20 25 30

Val Gln Glu Asn Phe Asp Val Asn Lys Tyr Leu Gly Arg Trp Tyr Glu

35 40 45

Ile Glu Lys Ile Pro Thr Thr Phe Glu Asn Gly Arg Cys Ile Gln Ala

50 55 60

Asn Tyr Ser Leu Met Glu Asn Gly Lys Ile Lys Val Leu Asn Gln Glu

65 70 75 80

Leu Arg Ala Asp Gly Thr Val Asn Gln Ile Glu Gly Glu Ala Thr Pro

85 90 95

Val Asn Leu Thr Glu Pro Ala Lys Leu Glu Val Lys Phe Ser Trp Phe

100 105 110

Met Pro Ser Ala Pro Tyr Trp Ile Leu Ala Thr Asp Tyr Glu Asn Tyr

115 120 125

Ala Leu Val Tyr Ser Cys Thr Cys Ile Ile Gln Leu Phe His Val Asp

130 135 140

Phe Ala Trp Ile Leu Ala Arg Asn Pro Asn Leu Pro Pro Glu Thr Val

145 150 155 160

Asp Ser Leu Lys Asn Ile Leu Thr Ser Asn Asn Ile Asp Val Lys Lys

165 170 175

Met Thr Val Thr Asp Gln Val Asn Cys Pro Lys Leu Ser

180 185

<210> 5

<211> 4040

<212> DNA

<213> Homo sapiens

<220>

<221> copper monamine oxidase mRNA

<222> (1)..(4040)

<400> 5

gtccttccca cccttagtcc caggcatctg actaccggga acctcagcca gagtccggga 60

gccccccacc ccgtccagga gccaacagag cccccgtctt gctggcgtga gaatacattg 120

ctctcctttg gttgaatcag ctgtccctct tcgtgggaaa atgaaccaga agacaatcct 180

cgtgctcctc attctggccg tcatcaccat ctttgccttg gtttgtgtcc tgctggtggg 240

caggggtgga gatgggggtg aacccagcca gcttccccat tgcccctctg tatctcccag 300

tgcccagcct tggacacacc ctggccagag ccagctgttt gcagacctga gccgagagga 360

gctgacggct gtgatgcgct ttctgaccca gcggctgggg ccagggctgg tggatgcagc 420

ccaggcccgg ccctcggaca actgtgtctt ctcagtggag ttgcagctgc ctcccaaggc 480

tgcagccctg gctcacttgg acagggggag ccccccacct gcccgggagg cactggccat 540

cgtcttcttt ggcaggcaac cccagcccaa cgtgagtgag ctggtggtgg ggccactgcc 600

tcacccctcc tacatgcggg acgtgactgt ggagcgtcat ggaggccccc tgccctatca 660

ccgacgcccc gtgctgttcc aagagtacct ggacatagac cagatgatct tcaacagaga 720

gctgccccag gcttctgggc ttctccacca ctgttgcttc tacaagcacc ggggacggaa 780

cctggtgaca atgaccacgg ctccccgtgg tctgcaatca ggggaccggg ccacctggtt 840

tggcctctac tacaacatct cgggcgctgg gttcttcctg caccacgtgg gcttggagct 900

gctagtgaac cacaaggccc ttgaccctgc ccgctggact atccagaagg tgttctatca 960

aggccgctac tacgacagcc tggcccagct ggaggcccag tttgaggccg gcctggtgaa 1020

tgtggtgctg atcccagaca atggcacagg tgggtcctgg tccctgaagt cccctgtgcc 1080

cccgggtcca gctcccccctc tacagttcta tccccaaggc ccccgcttca gtgtccaggg 1140

aagtcgagtg gcctcctcac tgtggacttt ctcctttggc ctcggagcat tcagtggccc 1200

aaggatcttt gacgttcgct tccaaggaga aagactagtt tatgagataa gcctccaaga 1260

ggccttggcc atctatggtg gaaattcccc agcagcaatg acgacccgct atgtggatgg 1320

aggctttggc atgggcaagt acaccacgcc cctgacccgt ggggtggact gcccctactt 1380

ggccacctac gtggactggc acttcctttt ggagtcccag gcccccaaga caatacgtga 1440

tgccttttgt gtgtttgaac agaaccaggg cctccccctg cggcgacacc actcagatct 1500

ctactcgcac tactttgggg gtcttgcgga aacggtgctg gtcgtcagat ctatgtccac 1560

cttgctcaac tatgactatg tgtgggatac ggtcttccac cccagtgggg ccatagaaat 1620

acgattctat gccacgggct acatcagctc ggcattcctc tttggtgcta ctgggaagta 1680

cgggaaccaa gtgtcagagc acaccctggg cacggtccac acccacagcg cccacttcaa 1740

ggtggatctg gatgtagcag gactggagaa ctgggtctgg gccgaggata tggtctttgt 1800

ccccatggct gtgccctgga gccctgagca ccagctgcag aggctgcagg tgacccggaa 1860

gctgctggag atggaggagc aggccgcctt cctcgtggga agcgccaccc ctcgctacct 1920

gtacctggcc agcaaccaca gcaacaagtg gggtcacccc cggggctacc gcatccagat 1980

gctcagcttt gctggagagc cgctgcccca aaacagctcc atggcgagag gcttcagctg 2040

ggagaggtac cagctggctg tgacccagcg gaaggaggag gagcccagta gcagcagcgt 2100

tttcaatcag aatgaccctt gggcccccac tgtggatttc agtgacttca tcaacaatga 2160

gaccattgct ggaaaggatt tggtggcctg ggtgacagct ggttttctgc atatcccaca 2220

tgcagaggac attcctaaca cagtgactgt ggggaacggc gtgggcttct tcctccgacc 2280

ctataacttc tttgacgaag acccctcctt ctactctgcc gactccatct acttccgagg 2340

ggaccaggat gctggggcct gcgaggtcaa ccccctagct tgcctgcccc aggctgctgc 2400

ctgtgccccc gacctccctg ccttctccca cgggggcttc tctcacaact aggcggtcct 2460

gggatggggc atgtggccaa gggctccagg gccagggtgt gagggatggg gagcagctgg 2520

gcactgggcc ggcagcctgg ttccctcttt cctgtgccag gactctcttt cttccactac 2580

cctccctcgc atccgcctct gagccaggag cctcctgacc ctgtgatgcc tgacacaggg 2640

gacactgaac cttgttgatg ccagctgtac tgagttctca tccacagagg ccaggcatgg 2700

cccagcctgg agccgtggcc gagggcttcc ctagatggtt ccctttgttg ctgtctggct 2760

ttcccgaatc tttttaggcc acctccaagg actctaaaag gggctattc cctggagacc 2820

```
ccagagtagg gttgccagtc ctgcaagtcc atagctgagc tggaaaggat gcttctgctc  2880
acattccctc tcatccaggt cctttccttc tcgtcttcct ctctctcacc tacttcctcc  2940
tcctcctcct gttcctgcct tctcttctat cctgcaattt ctcccgaatc ctgaggggat  3000
atccctatgt cccagcccct ggtactcccc cagccctcag ttttcagtca agttccgtct  3060
cctctccagc cctatggaag tctcaaggtc acgggacccc taatcagagt ggccaatccc  3120
tgtgtgtcgt tcccttgtgt ctgttgctta ttgggagtag gagttgctcc taccccctgtc  3180
ctggggctgg gtgtgtttca ggacagctgc ttctgtgcat ttgtgtctgc ctgcctcatg  3240
ctctctatag aggaggatgg tcatcgtgac agcagcagct caagttagca tttcaagtga  3300
tttgggggtg caatgataat gaagaatggc cattttgtac cagggctctg tattctgcaa  3360
cagcctgttt gggaggctgg agtggaaaca aagggtgggc atcaaagatg agaagccaaa  3420
gcccctacaa ctccagccac ccagccagga ggggctgtcc aatcacattc aggcatgcga  3480
atgagctggg ccctgggtga ggtgggggtc tggcctagtg gggaggggcc tggcctgggt  3540
ggggcagggc ctggcctggt ccaggcttgg gctccattcc catcactgct gtccctcctg  3600
aggtctggat tggggatggg gacaaagaaa tagcaagaga tgagaaacaa cagaaacttt  3660
tttctctaaa ggactggtta aatcaattct gatacagcct tacaatacaa tagtatgcag  3720
ctaaaaaata attgtatgtc tttatatact aatatgtaat aatcttcagg tgaaaaaggc  3780
aagccacaga aatgtgtwta gcgcacttcc catttgtgtt tcagaaagga gtagaatata  3840
aacacataat tgcttatgta tgcctattca gaataaatgg gtaacactga ttacttttgg  3900
gaggggaacc agtaggttga ggacaggaga gggaagggtc ttaacactta caccctttttg  3960
tacattttga attttgaacc atgtgactgt attacctatt caaaataaac aataaatggg  4020
cccaaaaaaa aaaaaaaaaa                                                4040
```

<210> 6

<211> 763

<212> PRT

<213> Homo sapiens

<220>

<221> copper monamine oxidase polypeptide sequence

<222> (1)..(763)

<400> 6

Met Asn Gln Lys Thr Ile Leu Val Leu Leu Ile Leu Ala Val Ile Thr
1 5 10 15

Ile Phe Ala Leu Val Cys Val Leu Leu Val Gly Arg Gly Gly Asp Gly
20 25 30

Gly Glu Pro Ser Gln Leu Pro His Cys Pro Ser Val Ser Pro Ser Ala
35 40 45

Gln Pro Trp Thr His Pro Gly Gln Ser Gln Leu Phe Ala Asp Leu Ser
50 55 60

Arg Glu Glu Leu Thr Ala Val Met Arg Phe Leu Thr Gln Arg Leu Gly
65 70 75 80

Pro Gly Leu Val Asp Ala Ala Gln Ala Arg Pro Ser Asp Asn Cys Val
85 90 95

Phe Ser Val Glu Leu Gln Leu Pro Pro Lys Ala Ala Ala Leu Ala His
100 105 110

Leu Asp Arg Gly Ser Pro Pro Pro Ala Arg Glu Ala Leu Ala Ile Val
115 120 125

Phe Phe Gly Arg Gln Pro Gln Pro Asn Val Ser Glu Leu Val Val Gly

130 135 140

Pro Leu Pro His Pro Ser Tyr Met Arg Asp Val Thr Val Glu Arg His

145 150 155 160

Gly Gly Pro Leu Pro Tyr His Arg Arg Pro Val Leu Phe Gln Glu Tyr

165 170 175

Leu Asp Ile Asp Gln Met Ile Phe Asn Arg Glu Leu Pro Gln Ala Ser

180 185 190

Gly Leu Leu His His Cys Cys Phe Tyr Lys His Arg Gly Arg Asn Leu

195 200 205

Val Thr Met Thr Thr Ala Pro Arg Gly Leu Gln Ser Gly Asp Arg Ala

210 215 220

Thr Trp Phe Gly Leu Tyr Tyr Asn Ile Ser Gly Ala Gly Phe Phe Leu

225 230 235 240

His His Val Gly Leu Glu Leu Leu Val Asn His Lys Ala Leu Asp Pro

245 250 255

Ala Arg Trp Thr Ile Gln Lys Val Phe Tyr Gln Gly Arg Tyr Tyr Asp

260 265 270

Ser Leu Ala Gln Leu Glu Ala Gln Phe Glu Ala Gly Leu Val Asn Val

275 280 285

Val Leu Ile Pro Asp Asn Gly Thr Gly Gly Ser Trp Ser Leu Lys Ser

290 295 300

Pro Val Pro Pro Gly Pro Ala Pro Pro Leu Gln Phe Tyr Pro Gln Gly

305 310 315 320

Pro Arg Phe Ser Val Gln Gly Ser Arg Val Ala Ser Ser Leu Trp Thr

325 330 335

Phe Ser Phe Gly Leu Gly Ala Phe Ser Gly Pro Arg Ile Phe Asp Val

340 345 350

Arg Phe Gln Gly Glu Arg Leu Val Tyr Glu Ile Ser Leu Gln Glu Ala

355 360 365

Leu Ala Ile Tyr Gly Gly Asn Ser Pro Ala Ala Met Thr Thr Arg Tyr

370 375 380

Val Asp Gly Gly Phe Gly Met Gly Lys Tyr Thr Thr Pro Leu Thr Arg

385 390 395 400

Gly Val Asp Cys Pro Tyr Leu Ala Thr Tyr Val Asp Trp His Phe Leu

405 410 415

Leu Glu Ser Gln Ala Pro Lys Thr Ile Arg Asp Ala Phe Cys Val Phe

420 425 430

Glu Gln Asn Gln Gly Leu Pro Leu Arg Arg His His Ser Asp Leu Tyr

435 440 445

Ser His Tyr Phe Gly Gly Leu Ala Glu Thr Val Leu Val Val Arg Ser

450 455 460

Met Ser Thr Leu Leu Asn Tyr Asp Tyr Val Trp Asp Thr Val Phe His

465 470 475 480

Pro Ser Gly Ala Ile Glu Ile Arg Phe Tyr Ala Thr Gly Tyr Ile Ser

485 490 495

Ser Ala Phe Leu Phe Gly Ala Thr Gly Lys Tyr Gly Asn Gln Val Ser

500 505 510

Glu His Thr Leu Gly Thr Val His Thr His Ser Ala His Phe Lys Val

515 520 525

Asp Leu Asp Val Ala Gly Leu Glu Asn Trp Val Trp Ala Glu Asp Met

530 535 540

Val Phe Val Pro Met Ala Val Pro Trp Ser Pro Glu His Gln Leu Gln

545 550 555 560

Arg Leu Gln Val Thr Arg Lys Leu Leu Glu Met Glu Glu Gln Ala Ala

565 570 575

Phe Leu Val Gly Ser Ala Thr Pro Arg Tyr Leu Tyr Leu Ala Ser Asn

580 585 590

His Ser Asn Lys Trp Gly His Pro Arg Gly Tyr Arg Ile Gln Met Leu

595 600 605

Ser Phe Ala Gly Glu Pro Leu Pro Gln Asn Ser Ser Met Ala Arg Gly

610 615 620

Phe Ser Trp Glu Arg Tyr Gln Leu Ala Val Thr Gln Arg Lys Glu Glu

625 630 635 640

Glu Pro Ser Ser Ser Ser Val Phe Asn Gln Asn Asp Pro Trp Ala Pro

645 650 655

Thr Val Asp Phe Ser Asp Phe Ile Asn Asn Glu Thr Ile Ala Gly Lys

660 665 670

Asp Leu Val Ala Trp Val Thr Ala Gly Phe Leu His Ile Pro His Ala

675 680 685

Glu Asp Ile Pro Asn Thr Val Thr Val Gly Asn Gly Val Gly Phe Phe

690 695 700

Leu Arg Pro Tyr Asn Phe Phe Asp Glu Asp Pro Ser Phe Tyr Ser Ala

705 710 715 720

Asp Ser Ile Tyr Phe Arg Gly Asp Gln Asp Ala Gly Ala Cys Glu Val

725 730 735

Asn Pro Leu Ala Cys Leu Pro Gln Ala Ala Ala Cys Ala Pro Asp Leu

740 745 750

Pro Ala Phe Ser His Gly Gly Phe Ser His Asn

755 760

<210> 7

<211> 3948

<212> DNA

<213> Homo sapiens

<220>

<221> dipeptidyl peptidase IV mRNA

<222> (1)..(3948)

<400> 7

```
ctttcactgg caagagacgg agtcctgggt ttcagttcca gttgcctgcg gtgggctgtg     60
tgagtttgcc aaagtcccct gccctctctg ggtctcggtt ccctcgcctg tccacgtgag    120
gttggaggag ctgaacgccg acgtcatttt tagctaagag ggagcagggt ccccgagtcg    180
ccggcccagg gtctgcgcat ccgaggccgc gcgccctttc ccctccccca cggctcctcc    240
gggccccgca ctctgcgccc cggctgccgc ccagcgccct acaccgccct caggggggccc   300
tcgcgggctc cccccggccg ggatgccagt gccccgcgcc acgcgcgcct gctcccgcgc    360
cgcctgccct gcagcctgcc cgcggcgcct ttatacccag cgggctcggc gctcactaat    420
gtttaactcg ggccgaaac ttgccagcgg cgagtgactc caccgcccgg agcagcggtg     480
```

caggacgcgc gtctccgccg cccgcggtga cttctgcctg cgctccttct ctgaacgctc 540

acttccgagg agacgccgac gatgaagaca ccgtggaagg ttcttctggg actgctgggt 600

gctgctgcgc ttgtcaccat catcaccgtg cccgtggttc tgctgaacaa aggcacagat 660

gatgctacag ctgacagtcg caaaacttac actctaactg attacttaaa aaatacttat 720

agactgaagt tatactcctt aagatggatt tcagatcatg aatatctcta caaacaagaa 780

aataatatct tggtattcaa tgctgaatat ggaaacagct cagttttctt ggagaacagt 840

acatttgatg agtttggaca ttctatcaat gattattcaa tatctcctga tgggcagttt 900

attctcttag aatacaacta cgtgaagcaa tggaggcatt cctacacagc ttcatatgac 960

atttatgatt taaataaaag gcagctgatt acagaagaga ggattccaaa caacacacag 1020

tgggtcacat ggtcaccagt gggtcataaa ttggcatatg tttggaacaa tgacatttat 1080

gttaaaattg aaccaaattt accaagttac agaatcacat ggacggggaa agaagatata 1140

atatataatg gaataactga ctgggtttat gaagaggaag tcttcagtgc ctactctgct 1200

ctgtggtggt ctccaaacgg cactttttta gcatatgccc aatttaacga cacagaagtc 1260

ccacttattg aatactcctt ctactctgat gagtcactgc agtacccaaa gactgtacgg 1320

gttccatatc caaaggcagg agctgtgaat ccaactgtaa agttctttgt tgtaaataca 1380

gactctctca gctcagtcac caatgcaact tccatacaaa tcactgctcc tgcttctatg 1440

ttgatagggg atcactactt gtgtgatgtg acatgggcaa cacaagaaag aatttctttg 1500

cagtggctca ggaggattca gaactattcg gtcatggata tttgtgacta tgatgaatcc 1560

agtggaagat ggaactgctt agtggcacgg caacacattg aaatgagtac tactggctgg 1620

gttggaagat ttaggccttc agaacctcat tttacccttg atggtaatag cttctacaag 1680

atcatcagca atgaagaagg ttacagacac atttgctatt tccaaataga taaaaaagac 1740

tgcacattta ttacaaaagg cacctgggaa gtcatcggga tagaagctct aaccagtgat 1800

tatctatact acattagtaa tgaatataaa ggaatgccag gaggaaggaa tctttataaa 1860

atccaactta gtgactatac aaaagtgaca tgcctcagtt gtgagctgaa tccggaaagg 1920

tgtcagtact attctgtgtc attcagtaaa gaggcgaagt attatcagct gagatgttcc 1980

ggtcctggtc tgcccctcta tactctacac agcagcgtga atgataaagg gctgagagtc 2040

ctggaagaca attcagcttt ggataaaatg ctgcagaatg tccagatgcc ctccaaaaaa 2100

ctggacttca ttattttgaa tgaaacaaaa ttttggtatc agatgatctt gcctcctcat 2160

tttgataaat ccaagaaata tcctctacta ttagatgtgt atgcaggccc atgtagtcaa 2220

aaagcagaca ctgtcttcag actgaactgg gccacttacc ttgcaagcac agaaaacatt 2280

atagtagcta gctttgatgg cagaggaagt ggttaccaag gagataagat catgcatgca 2340

atcaacagaa gactgggaac atttgaagtt gaagatcaaa ttgaagcagc cagacaattt 2400

tcaaaaatgg gatttgtgga caacaaacga attgcaattt ggggctggtc atatggaggg 2460

tacgtaacct caatggtcct gggatcggga agtggcgtgt tcaagtgtgg aatagccgtg 2520

gcgcctgtat cccggtggga gtactatgac tcagtgtaca cagaacgtta catgggtctc 2580

ccaactccag aagacaacct tgaccattac agaaattcaa cagtcatgag cagagctgaa 2640

aattttaaac aagttgagta cctccttatt catggaacag cagatgataa cgttcacttt 2700

cagcagtcag ctcagatctc caaagccctg gtcgatgttg gagtggattt ccaggcaatg 2760

tggtatactg atgaagacca tggaatagct agcagcacag cacaccaaca tatatatacc 2820

cacatgagcc acttcataaa acaatgtttc tctttacctt agcacctcaa aataccatgc 2880

catttaaagc ttattaaaac tcatttttgt tttcattatc tcaaaactgc actgtcaaga 2940

tgatgatgat ctttaaaata cacactcaaa tcaagaaact taaggttacc tttgttccca 3000

aatttcatac ctatcatctt aagtagggac ttctgtcttc acaacagatt attaccttac 3060

agaagtttga attatccggt cgggttttat tgtttaaaat catttctgca tcagctgctg 3120

aaacaacaaa taggaattgt ttttatggag gctttgcata gattccctga gcaggatttt 3180

aatctttttc taactggact ggttcaaatg ttgttctctt ctttaaaggg atggcaagat 3240

gtgggcagtg atgtcactag ggcagggaca ggataagagg gattagggag agaagatagc 3300

agggcatggc tgggaaccca agtccaagca taccaacacg agcaggctac tgtcagctcc 3360

cctcggagaa gagctgttca cagccagact ggcacagttt tctgagaaag actattcaaa 3420

cagtctcagg aaatcaaata tgcaaagcac tgacttctaa gtaaaaccac agcagttgaa 3480

aagactccaa agaaatgtaa gggaaactgc cagcaacgca ggcccccagg tgccagttat 3540

ggctataggt gctacaaaaa cacagcaagg gtgatgggaa agcattgtaa atgtgctttt 3600

aaaaaaaaat actgatgttc ctagtgaaag aggcagcttg aaactgagat gtgaacacat 3660

cagcttgccc tgttaaaaga tgaaaatatt tgtatcacaa atcttaactt gaaggagtcc 3720

ttgcatcaat ttttcttatt tcatttcttt gagtgtctta attaaaagaa tattttaact 3780

tccttggact cattttaaaa aatggaacat aaaatacaat gttatgtatt attattccca 3840

ttctacatac tatggaattt ctcccagtca tttaataaat gtgccttcat tttttcagaa 3900

aaaaaaaaaa aaagtcagtg cttgcatact caaggagtct tatctaga 3948

<210> 8

<211> 766

<212> PRT

<213> Homo sapiens

<220>

<221> dipeptidyl peptidase IV polypeptide sequence

<222> (1)..(766)

<400> 8

Met Lys Thr Pro Trp Lys Val Leu Leu Gly Leu Leu Gly Ala Ala Ala

1 5 10 15

Leu Val Thr Ile Ile Thr Val Pro Val Val Leu Leu Asn Lys Gly Thr

20 25 30

Asp Asp Ala Thr Ala Asp Ser Arg Lys Thr Tyr Thr Leu Thr Asp Tyr

35 40 45

Leu Lys Asn Thr Tyr Arg Leu Lys Leu Tyr Ser Leu Arg Trp Ile Ser

50 55 60

Asp His Glu Tyr Leu Tyr Lys Gln Glu Asn Asn Ile Leu Val Phe Asn

65 70 75 80

Ala Glu Tyr Gly Asn Ser Ser Val Phe Leu Glu Asn Ser Thr Phe Asp

85 90 95

Glu Phe Gly His Ser Ile Asn Asp Tyr Ser Ile Ser Pro Asp Gly Gln

100 105 110

Phe Ile Leu Leu Glu Tyr Asn Tyr Val Lys Gln Trp Arg His Ser Tyr

115 120 125

Thr Ala Ser Tyr Asp Ile Tyr Asp Leu Asn Lys Arg Gln Leu Ile Thr

130 135 140

Glu Glu Arg Ile Pro Asn Asn Thr Gln Trp Val Thr Trp Ser Pro Val

145 150 155 160

Gly His Lys Leu Ala Tyr Val Trp Asn Asn Asp Ile Tyr Val Lys Ile

165 170 175

Glu Pro Asn Leu Pro Ser Tyr Arg Ile Thr Trp Thr Gly Lys Glu Asp

180 185 190

Ile Ile Tyr Asn Gly Ile Thr Asp Trp Val Tyr Glu Glu Glu Val Phe

195 200 205

Ser Ala Tyr Ser Ala Leu Trp Trp Ser Pro Asn Gly Thr Phe Leu Ala

210 215 220

Tyr Ala Gln Phe Asn Asp Thr Glu Val Pro Leu Ile Glu Tyr Ser Phe

225 230 235 240

Tyr Ser Asp Glu Ser Leu Gln Tyr Pro Lys Thr Val Arg Val Pro Tyr

245 250 255

Pro Lys Ala Gly Ala Val Asn Pro Thr Val Lys Phe Phe Val Val Asn

260 265 270

Thr Asp Ser Leu Ser Ser Val Thr Asn Ala Thr Ser Ile Gln Ile Thr

275 280 285

Ala Pro Ala Ser Met Leu Ile Gly Asp His Tyr Leu Cys Asp Val Thr

290 295 300

Trp Ala Thr Gln Glu Arg Ile Ser Leu Gln Trp Leu Arg Arg Ile Gln

305 310 315 320

Asn Tyr Ser Val Met Asp Ile Cys Asp Tyr Asp Glu Ser Ser Gly Arg

325 330 335

Trp Asn Cys Leu Val Ala Arg Gln His Ile Glu Met Ser Thr Thr Gly

340 345 350

Trp Val Gly Arg Phe Arg Pro Ser Glu Pro His Phe Thr Leu Asp Gly

355 360 365

Asn Ser Phe Tyr Lys Ile Ile Ser Asn Glu Glu Gly Tyr Arg His Ile

370 375 380

Cys Tyr Phe Gln Ile Asp Lys Lys Asp Cys Thr Phe Ile Thr Lys Gly

385 390 395 400

Thr Trp Glu Val Ile Gly Ile Glu Ala Leu Thr Ser Asp Tyr Leu Tyr
405 410 415

Tyr Ile Ser Asn Glu Tyr Lys Gly Met Pro Gly Gly Arg Asn Leu Tyr
420 425 430

Lys Ile Gln Leu Ser Asp Tyr Thr Lys Val Thr Cys Leu Ser Cys Glu
435 440 445

Leu Asn Pro Glu Arg Cys Gln Tyr Tyr Ser Val Ser Phe Ser Lys Glu
450 455 460

Ala Lys Tyr Tyr Gln Leu Arg Cys Ser Gly Pro Gly Leu Pro Leu Tyr
465 470 475 480

Thr Leu His Ser Ser Val Asn Asp Lys Gly Leu Arg Val Leu Glu Asp
485 490 495

Asn Ser Ala Leu Asp Lys Met Leu Gln Asn Val Gln Met Pro Ser Lys
500 505 510

Lys Leu Asp Phe Ile Ile Leu Asn Glu Thr Lys Phe Trp Tyr Gln Met
515 520 525

Ile Leu Pro Pro His Phe Asp Lys Ser Lys Lys Tyr Pro Leu Leu Leu
530 535 540

Asp Val Tyr Ala Gly Pro Cys Ser Gln Lys Ala Asp Thr Val Phe Arg
545 550 555 560

Leu Asn Trp Ala Thr Tyr Leu Ala Ser Thr Glu Asn Ile Ile Val Ala
565 570 575

Ser Phe Asp Gly Arg Gly Ser Gly Tyr Gln Gly Asp Lys Ile Met His
580 585 590

Ala Ile Asn Arg Arg Leu Gly Thr Phe Glu Val Glu Asp Gln Ile Glu

595 600 605

Ala Ala Arg Gln Phe Ser Lys Met Gly Phe Val Asp Asn Lys Arg Ile

610 615 620

Ala Ile Trp Gly Trp Ser Tyr Gly Gly Tyr Val Thr Ser Met Val Leu

625 630 635 640

Gly Ser Gly Ser Gly Val Phe Lys Cys Gly Ile Ala Val Ala Pro Val

645 650 655

Ser Arg Trp Glu Tyr Tyr Asp Ser Val Tyr Thr Glu Arg Tyr Met Gly

660 665 670

Leu Pro Thr Pro Glu Asp Asn Leu Asp His Tyr Arg Asn Ser Thr Val

675 680 685

Met Ser Arg Ala Glu Asn Phe Lys Gln Val Glu Tyr Leu Leu Ile His

690 695 700

Gly Thr Ala Asp Asp Asn Val His Phe Gln Gln Ser Ala Gln Ile Ser

705 710 715 720

Lys Ala Leu Val Asp Val Gly Val Asp Phe Gln Ala Met Trp Tyr Thr

725 730 735

Asp Glu Asp His Gly Ile Ala Ser Ser Thr Ala His Gln His Ile Tyr

740 745 750

Thr His Met Ser His Phe Ile Lys Gln Cys Phe Ser Leu Pro

755 760 765

<210> 9

<211> 6803

<212> DNA

<213> Homo sapiens

<220>

<221> Fibroblast Growth Factor 2 mRNA

<222> (1)..(6803)

<400> 9

cggccccaga aaacccgagc gagtaggggg cggcgcgcag gagggaggag aactgggggc 60

gcgggaggct ggtgggtgtc gggggtggag atgtagaaga tgtgacgccg cggcccggcg 120

ggtgccagat tagcggacgc gctgcccgcg gttgcaacgg gatcccgggc gctgcagctt 180

gggaggcggc tctccccagg cggcgtccgc ggagacaccc atccgtgaac cccaggtccc 240

gggccgccgg ctcgccgcgc accaggggcc ggcggacaga agagcggccg agcggctcga 300

ggctggggga ccgcgggcgc ggccgcgcgc tgccgggcgg gaggctgggg ggccggggcc 360

ggggccgtgc cccggagcgg gtcggaggcc ggggccgggg ccgggggacg gcggctcccc 420

gcgcggctcc agcggctcgg ggatcccggc cgggccccgc agggaccatg gcagccggga 480

gcatcaccac gctgcccgcc ttgcccgagg atggcggcag cggcgccttc ccgcccggcc 540

acttcaagga ccccaagcgg ctgtactgca aaaacggggg cttcttcctg cgcatccacc 600

ccgacggccg agttgacggg gtccgggaga agagcgaccc tcacatcaag ctacaacttc 660

aagcagaaga gagaggagtt gtgtctatca aaggagtgtg tgctaaccgt tacctggcta 720

tgaaggaaga tggaagatta ctggcttcta aatgtgttac ggatgagtgt ttcttttttg 780

aacgattgga atctaataac tacaatactt accggtcaag gaaatacacc agttggtatg 840

tggcactgaa acgaactggg cagtataaac ttggatccaa aacaggacct gggcagaaag 900

ctatactttt tcttccaatg tctgctaaga gctgatttta atggccacat ctaatctcat 960

ttcacatgaa agaagaagta tattttagaa atttgttaat gagagtaaaa gaaaataaat 1020

gtgtatagct cagtttggat aattggtcaa acaatttttt atccagtagt aaaatatgta 1080

accattgtcc cagtaaagaa aaataacaaa agttgtaaaa tgtatattct ccctttttata 1140

ttgcatctgc tgttacccag tgaagcttac ctagagcaat gatcttttc acgcatttgc 1200

tttattcgaa aagaggcttt taaaatgtgc atgtttagaa acaaaatttc ttcatggaaa 1260

tcatatacat tagaaaatca cagtcagatg tttaatcaat ccaaaatgtc cactatttct 1320

tatgtcattc gttagtctac atgtttctaa acatataaat gtgaatttaa tcaattcctt 1380

tcatagtttt ataattctct ggcagttcct tatgatagag tttataaaac agtcctgtgt 1440

aaactgctgg aagttcttcc acagtcaggt caattttgtc aaacccttct ctgtacccat 1500

acagcagcag cctagcaact ctgctggtga tgggagttgt attttcagtc ttcgccaggt 1560

cattgagatc catccactca catcttaagc attcttcctg gcaaaaattt atggtgaatg 1620

aatatggctt taggcggcag atgatataca tatctgactt cccaaaagct ccaggatttg 1680

tgtgctgttg ccgaatactc aggacggacc tgaattctga ttttatacca gtctcttcaa 1740

aaacttctcg aaccgctgtg tctcctacgt aaaaaaagag atgtacaaat caataataat 1800

tacactttta gaaactgtat catcaaagat tttcagttaa agtagcatta tgtaaaggct 1860

caaaacatta ccctaacaaa gtaaagtttt caatacaaat tctttgcctt gtggatatca 1920

agaaatccca aaatattttc ttaccactgt aaattcaaga agcttttgaa atgctgaata 1980

tttctttggc tgctacttgg aggcttatct acctgtacat ttttggggtc agctcttttt 2040

aacttcttgc tgctcttttt cccaaaaggt aaaaatatag attgaaaagt taaaacattt 2100

tgcatggctg cagttccttt gtttcttgag ataagattcc aaagaactta gattcatttc 2160

ttcaacaccg aaatgctgga ggtgtttgat cagttttcaa gaaacttgga atataaataa 2220

ttttataatt caacaaaggt tttcacattt tataaggttg atttttcaat taaatgcaaa 2280

tttgtgtggc aggattttta ttgccattaa catatttttg tggctgcttt ttctacacat 2340

ccagatggtc cctctaactg ggctttctct aattttgtga tgttctgtca ttgtctccca 2400

aagtatttag gagaagccct ttaaaaagct gccttcctct accactttgc tggaaagctt 2460
cacaattgtc acagacaaag atttttgttc caatactcgt tttgcctcta tttttcttgt 2520
ttgtcaaata gtaaatgata tttgcccttg cagtaattct actggtgaaa aacatgcaaa 2580
gaagaggaag tcacagaaac atgtctcaat tcccatgtgc tgtgactgta gactgtctta 2640
ccatagactg tcttacccat cccctggata tgctcttgtt ttttccctct aatagctatg 2700
gaaagatgca tagaaagagt ataatgtttt aaaacataag gcattcatct gccatttttc 2760
aattacatgc tgacttccct tacaattgag atttgcccat aggttaaaca tggttagaaa 2820
caactgaaag cataaaagaa aaatctaggc cgggtgcagt ggctcatgcc tatattccct 2880
gcactttggg aggccaaagc aggaggatcg cttgagccca ggagttcaag accaacctgg 2940
tgaaaccccg tctctacaaa aaaacacaaa aaatagccag gcatggtggc gtgtacatgt 3000
ggtctcagat acttgggagg ctgaggtggg agggttgatc acttgaggct gagaggtcaa 3060
ggttgcagtg agccataatc gtgccactgc agtccagcct aggcaacaga gtgagacttt 3120
gtctcaaaaa aagagaaatt ttccttaata agaaaagtaa tttttactct gatgtgcaat 3180
acatttgtta ttaaatttat tatttaagat ggtagcacta gtcttaaatt gtataaaata 3240
tcccctaaca tgtttaaatg tccattttta ttcattatgc tttgaaaaat aattatgggg 3300
aaatacatgt ttgttattaa atttattatt aaagatagta gcactagtct taaatttgat 3360
ataacatctc ctaacttgtt taaatgtcca tttttattct ttatgcttga aaataaatta 3420
tggggatcct atttagctct tagtaccact aatcaaaagt tcggcatgta gctcatgatc 3480
tatgctgttt ctatgtcgtg gaagcaccgg atgggggtag tgagcaaatc tgccctgctc 3540
agcagtcacc atagcagctg actgaaaatc agcactgcct gagtagtttt gatcagttta 3600
acttgaatca ctaactgact gaaaattgaa tgggcaaata agtgcttttg tctccagagt 3660
atgcgggaga cccttccacc tcaagatgga tatttcttcc ccaaggattt caagatgaat 3720
tgaaattttt aatcaagata gtgtgcttta ttctgttgta ttttttatta ttttaatata 3780
ctgtaagcca aactgaaata acatttgctg ttttataggt ttgaagaaca taggaaaaac 3840

taagaggttt tgtttttatt tttgctgatg aagagatatg tttaaatatg ttgtattgtt 3900

ttgtttagtt acaggacaat aatgaaatgg agtttatatt tgttatttct attttgttat 3960

atttaataat agaattagat tgaaataaaa tataatggga aataatctgc agaatgtggg 4020

tttcctggtg tttcctctga ctctagtgca ctgatgatct ctgataaggc tcagctgctt 4080

tatagttctc tggctaatgc agcagatact cttcctgcca gtggtaatac gattttttaa 4140

gaaggcagtt tgtcaatttt aatcttgtgg atacctttat actcttaggg tattatttta 4200

tacaaaagcc ttgaggattg cattctattt tctatatgac cctcttgata tttaaaaaac 4260

actatggata acaattcttc atttacctag tattatgaaa gaatgaagga gttcaaacaa 4320

atgtgtttcc cagttaacta gggtttactg tttgagccaa tataaatgtt taactgtttg 4380

tgatggcagt attcctaaag tacattgcat gttttcctaa atacagagtt taaataattt 4440

cagtaattct tagatgattc agcttcatca ttaagaatat cttttgtttt atgttgagtt 4500

agaaatgcct tcatatagac atagtctttc agacctctac tgtcagtttt catttctagc 4560

tgctttcagg gttttatgaa ttttcaggca aagctttaat ttatactaag cttaggaagt 4620

atggctaatg ccaacggcag ttttttttctt cttaattcca catgactgag gcatatatga 4680

tctctgggta ggtgagttgt tgtgacaacc acaagcactt tttttttttt taaagaaaaa 4740

aaggtagtga atttttaatc atctggactt taagaaggat tctggagtat acttaggcct 4800

gaaattatat atatttggct tggaaatgtg tttttcttca attacatcta caagtaagta 4860

cagctgaaat tcagaggacc cataagagtt cacatgaaaa aaatcaattc atttgaaaag 4920

gcaagatgca ggagagagga agccttgcaa acctgcagac tgctttttgc ccaatataga 4980

ttgggtaagg ctgcaaaaca taagcttaat tagctcacat gctctgctct cacgtggcac 5040

cagtggatag tgtgagagaa ttaggctgta gaacaaatgg ccttctcttt cagcattcac 5100

accactacaa aatcatcttt tatatcaaca gaagaataag cataaactaa gcaaaaggtc 5160

aataagtacc tgaaaccaag attggctaga gatatatctt aatgcaatcc attttctgat 5220

ggattgttac gagttggcta tataatgtat gtatggtatt ttgatttgtg taaaagtttt 5280

aaaaatcaag ctttaagtac atggacattt ttaaataaaa tatttaaaga caatttagaa 5340

aattgcctta atatcattgt tggctaaata gaataggga catgcatatt aaggaaaagg 5400

tcatggagaa ataatattgg tatcaaacaa atacattgat ttgtcatgat acacattgaa 5460

tttgatccaa tagtttaagg aataggtagg aaaatttggt ttctattttt cgatttcctg 5520

taaatcagtg acataaataa ttcttagctt attttatatt tccttgtctt aaatactgag 5580

ctcagtaagt tgtgttaggg gattatttct cagttgagac tttcttatat gacattttac 5640

tatgttttga cttcctgact attaaaaata aatagtagaa acaattttca taaagtgaag 5700

aattatataa tcactgcttt ataactgact ttattatatt tatttcaaag ttcatttaaa 5760

ggctactatt catcctctgt gatggaatgg tcaggaattt gttttctcat agtttaattc 5820

caacaacaat attagtcgta tccaaaataa cctttaatgc taaactttac tgatgtatat 5880

ccaaagcttc tccttttcag acagattaat ccagaagcag tcataaacag aagaataggt 5940

ggtatgttcc taatgatatt atttctacta atggaataaa ctgtaatatt agaaattatg 6000

ctgctaatta tatcagctct gaggtaattt ctgaaatgtt cagactcagt cggaacaaat 6060

tggaaaattt aaatttttat tcttagctat aaagcaagaa agtaaacaca ttaatttcct 6120

caacattttt aagccaatta aaaatataaa agatacacac caatatcttc ttcaggctct 6180

gacaggcctc ctggaaactt ccacatattt ttcaactgca gtataaagtc agaaaataaa 6240

gttaacataa ctttcactaa cacacacata tgtagatttc acaaaatcca cctataattg 6300

gtcaaagtgg ttgagaatat attttttagt aattgcatgc aaaatttttc tagcttccat 6360

cctttctccc tcgtttcttc ttttttgggg ggagctggta actgatgaaa tcttttccca 6420

ccttttctct tcaggaaata taagtggttt tgtttggtta acgtgataca ttctgtatga 6480

atgaaacatt ggagggaaac atctactgaa tttctgtaat ttaaaatatt ttgctgctag 6540

ttaactatga acagatagaa gaatcttaca gatgctgcta taaataagta gaaaatataa 6600

atttcatcac taaaatatgc tattttaaaa tctatttcct atattgtatt tctaatcaga 6660

tgtattactc ttattatttc tattgtatgt gttaatgatt ttatgtaaaa atgtaattgc 6720

ttttcatgag tagtatgaat aaaattgatt agtttgtgtt ttcttgtctc ccgaaaaaaa 6780

aaaaaaaaaa aaaaaaaaaa aaa 6803

<210> 10

<211> 288

<212> PRT

<213> Homo sapiens

<220>

<221> fibroblast growth factor 2 polypeptide sequence

<222> (1)..(288)

<400> 10

Met Val Gly Val Gly Gly Gly Asp Val Glu Asp Val Thr Pro Arg Pro
1 5 10 15

Gly Gly Cys Gln Ile Ser Gly Arg Ala Ala Arg Gly Cys Asn Gly Ile
20 25 30

Pro Gly Ala Ala Ala Trp Glu Ala Ala Leu Pro Arg Arg Arg Pro Arg
35 40 45

Arg His Pro Ser Val Asn Pro Arg Ser Arg Ala Ala Gly Ser Pro Arg
50 55 60

Thr Arg Gly Arg Arg Thr Glu Glu Arg Pro Ser Gly Ser Arg Leu Gly
65 70 75 80

Asp Arg Gly Arg Gly Arg Ala Leu Pro Gly Gly Arg Leu Gly Gly Arg
85 90 95

Gly Arg Gly Arg Ala Pro Glu Arg Val Gly Gly Arg Gly Arg Gly Arg

100 105 110

Gly Thr Ala Ala Pro Arg Ala Ala Pro Ala Ala Arg Gly Ser Arg Pro

115 120 125

Gly Pro Ala Gly Thr Met Ala Ala Gly Ser Ile Thr Thr Leu Pro Ala

130 135 140

Leu Pro Glu Asp Gly Gly Ser Gly Ala Phe Pro Pro Gly His Phe Lys

145 150 155 160

Asp Pro Lys Arg Leu Tyr Cys Lys Asn Gly Gly Phe Phe Leu Arg Ile

165 170 175

His Pro Asp Gly Arg Val Asp Gly Val Arg Glu Lys Ser Asp Pro His

180 185 190

Ile Lys Leu Gln Leu Gln Ala Glu Glu Arg Gly Val Val Ser Ile Lys

195 200 205

Gly Val Cys Ala Asn Arg Tyr Leu Ala Met Lys Glu Asp Gly Arg Leu

210 215 220

Leu Ala Ser Lys Cys Val Thr Asp Glu Cys Phe Phe Phe Glu Arg Leu

225 230 235 240

Glu Ser Asn Asn Tyr Asn Thr Tyr Arg Ser Arg Lys Tyr Thr Ser Trp

245 250 255

Tyr Val Ala Leu Lys Arg Thr Gly Gln Tyr Lys Leu Gly Ser Lys Thr

260 265 270

Gly Pro Gly Gln Lys Ala Ile Leu Phe Leu Pro Met Ser Ala Lys Ser

275 280 285

<210> 11

<211> 5826

<212> DNA

<213> Homo sapiens

<220>

<221> thrombospondin 2 mRNA

<222> (1)..(5826)

<400> 11

gaggaggaga cggcatccag tacagagggg ctggacttgg acccctgcag cagccctgca 60

caggagaagc ggcatataaa gccgcgctgc ccgggagccg ctcggccacg tccaccggag 120

catcctgcac tgcagggccg gtctctcgct ccagcagagc ctgcgccttt ctgactcggt 180

ccggaacact gaaaccagtc atcactgcat ctttttggca aaccaggagc tcagctgcag 240

gaggcaggat ggtctggagg ctggtcctgc tggctctgtg ggtgtggccc agcacgcaag 300

ctggtcacca ggacaaagac acgaccttcg accttttcag tatcagcaac atcaaccgca 360

agaccattgg cgccaagcag ttccgcgggc ccgaccccgg cgtgccggct taccgcttcg 420

tgcgctttga ctacatccca ccggtgaacg cagatgacct cagcaagatc accaagatca 480

tgcggcagaa ggagggcttc ttcctcacgg cccagctcaa gcaggacggc aagtccaggg 540

gcacgctgtt ggctctggag ggccccggtc tctcccagag gcagttcgag atcgtctcca 600

acggccccgc ggacacgctg gatctcacct actggattga cggcacccgg catgtggtct 660

ccctggagga cgtcggcctg gctgactcgc agtggaagaa cgtcaccgtg caggtggctg 720

gcgagaccta cagcttgcac gtgggctgcg acctcataga cagcttcgct ctggacgagc 780

ccttctacga gcacctgcag gcggaaaaga gccggatgta cgtggccaaa ggctctgcca 840

gagagagtca cttcaggggt ttgcttcaga acgtccacct agtgtttgaa aactctgtgg 900

aagatattct aagcaagaag ggttgccagc aaggccaggg agctgagatc aacgccatca 960

gtgagaacac agagacgctg cgcctgggtc cgcatgtcac caccgagtac gtgggcccca 1020

gctcggagag gaggcccgag gtgtgcgaac gctcgtgcga ggagctggga aacatggtcc 1080

aggagctctc ggggctccac gtcctcgtga accagctcag cgagaacctc aagagagtgt 1140

cgaatgataa ccagtttctc tgggagctca ttggtggccc tcctaagaca aggaacatgt 1200

cagcttgctg gcaggatggc cggttctttg cggaaaatga aacgtgggtg gtggacagct 1260

gcaccacgtg tacctgcaag aaatttaaaa ccatttgcca ccaaatcacc tgcccgcctg 1320

caacctgcgc cagtccatcc tttgtggaag gcgaatgctg cccttcctgc ctccactcgg 1380

tggacggtga ggagggctgg tctccgtggg cagagtggac ccagtgctcc gtgacgtgtg 1440

gctctgggac ccagcagaga ggccggtcct gtgacgtcac cagcaacacc tgcttggggc 1500

cctccatcca gacacgggct tgcagtctga gcaagtgtga cacccgcatc cggcaggacg 1560

gcggctggag ccactggtca ccttggtctt catgctctgt gacctgtgga gttggcaata 1620

tcacacgcat ccgtctctgc aactccccag tgccccagat gggggggcaag aattgcaaag 1680

ggagtggccg ggagaccaaa gcctgccagg gcgccccatg cccaatcgat ggccgctgga 1740

gcccctggtc ccgtggtcg gcctgcactg tcacctgtgc cggtgggatc cgggagcgca 1800

cccgggtctg caacagccct gagcctcagt acggagggaa ggcctgcgtg ggggatgtgc 1860

aggagcgtca gatgtgcaac aagaggagct gccccgtgga tggctgttta tccaacccct 1920

gcttcccggg agcccagtgc agcagcttcc ccgatgggtc ctggtcatgc ggctcctgcc 1980

ctgtgggctt cttgggcaat ggcacccact gtgaggacct ggacgagtgt gccctggtcc 2040

ccgacatctg cttctccacc agcaaggtgc ctcgctgtgt caacactcag cctggcttcc 2100

actgcctgcc ctgcccgccc cgatacagag ggaaccagcc cgtcggggtc ggcctggaag 2160

cagccaagac ggaaaagcaa gtgtgtgagc ccgaaaaccc atgcaaggac aagacacaca 2220

actgccacaa gcacgcggag tgcatctacc tgggccactt cagcgacccc atgtacaagt 2280

gcgagtgcca gacaggctac gcgggcgacg ggctcatctg cggggaggac tcggacctgg 2340

```
acggctggcc caacctcaat ctggtctgcg ccaccaacgc cacctaccac tgcatcaagg  2400
ataactgccc ccatctgcca aattctgggc aggaagactt tgacaaggac gggattggcg  2460
atgcctgtga tgatgacgat gacaatgacg gtgtgaccga tgagaaggac aactgccagc  2520
tcctcttcaa tccccgccag gctgactatg acaaggatga ggttggggac cgctgtgaca  2580
actgccctta cgtgcacaac cctgcccaga tcgacacaga caacaatgga gagggtgacg  2640
cctgctccgt ggacattgat ggggacgatg tcttcaatga acgagacaat tgtccctacg  2700
tctacaacac tgaccagagg gacacggatg gtgacggtgt gggggatcac tgtgacaact  2760
gccccctggt gcacaaccct gaccagaccg acgtggacaa tgaccttgtt ggggaccagt  2820
gtgacaacaa cgaggacata gatgacgacg gccaccagaa caaccaggac aactgcccct  2880
acatctccaa cgccaaccag gctgaccatg acagagacgg ccagggcgac gcctgtgacc  2940
ctgatgatga caacgatggc gtccccgatg acagggacaa ctgccggctt gtgttcaacc  3000
cagaccagga ggacttggac ggtgatggac ggggtgatat ttgtaaagat gattttgaca  3060
atgacaacat cccagatatt gatgatgtgt gtcctgaaaa caatgccatc agtgagacag  3120
acttcaggaa cttccagatg gtccccttgg atcccaaagg gaccacccaa attgatccca  3180
actgggtcat tcgccatcaa ggcaaggagc tggttcagac agccaactcg gaccccggca  3240
tcgctgtagg ttttgacgag tttgggtctg tggacttcag tggcacattc tacgtaaaca  3300
ctgaccggga cgacgactat gccggcttcg tctttggtta ccagtcaagc agccgcttct  3360
atgtggtgat gtggaagcag gtgacgcaga cctactggga ggaccagccc acgcgggcct  3420
atggctactc cggcgtgtcc ctcaaggtgg tgaactccac cacggggacg ggcgagcacc  3480
tgaggaacgc gctgtggcac acggggaaca cgccggggca ggtgcgaacc ttatggcacg  3540
accccaggaa cattggctgg aaggactaca cggcctatag gtggcacctg actcacaggc  3600
ccaagactgg ctacatcaga gtcttagtgc atgaaggaaa acaggtcatg gcagactcag  3660
gacctatcta tgaccaaacc tacgctggcg ggcggctggg tctatttgtc ttctctcaag  3720
aaatggtcta tttctcagac ctcaagtacg aatgcagaga tatttaaaca agatttgctg  3780
```

catttccggc aatgccctgt gcatgccatg gtccctagac acctcagttc attgtggtcc 3840
ttgtggcttc tctctctagc agcacctcct gtcccttgac cttaactctg atggttcttc 3900
acctcctgcc agcaacccca aacccaagtg ccttcagagg ataaatatca atggaactca 3960
gagatgaaca tctaacccac tagaggaaac cagtttggtg atatatgaga ctttatgtgg 4020
agtgaaaatt gggcatgcca ttacattgct ttttcttgtt tgtttaaaaa gaatgacgtt 4080
tacatataaa atgtaattac ttattgtatt tatgtgtata tggagttgaa gggaatactg 4140
tgcataagcc attatgataa attaagcatg aaaaatattg ctgaactact tttggtgctt 4200
aaagttgtca ctattcttga attagagttg ctctacaatg acacacaaat cccattaaat 4260
aaattataaa caagggtcaa ttcaaatttg aagtaatgtt ttagtaagga gagattagaa 4320
gacaacaggc atagcaaatg acataagcta ccgattaact aatcggaaca tgtaaaacag 4380
ttacaaaaat aaacgaactc tcctcttgtc ctacaatgaa agccctcatg tgcagtagag 4440
atgcagtttc atcaaagaac aaacatcctt gcaaatgggt gtgacgcggt tccagatgtg 4500
gatttggcaa aacctcattt aagtaaaagg ttagcagagc aaagtgcggt gctttagctg 4560
ctgcttgtgc cgctgtggcg tcggggaggc tcctgcctga gcttccttcc ccagctttgc 4620
tgcctgagag gaaccagagc agacgcacag gccggaaaag gcgcatctaa cgcgtatcta 4680
ggctttggta actgcggaca agttgctttt acctgatttg atgatacatt tcattaaggt 4740
tccagttata aatattttgt taatatttat taagtgacta tagaatgcaa ctccatttac 4800
cagtaactta ttttaaatat gcctagtaac acatatgtag tataatttct agaaacaaac 4860
atctaataag tatataatcc tgtgaaaata tgaggcttga taatattagg ttgtcacgat 4920
gaagcatgct agaagctgta acagaataca tagagaataa tgaggagttt atgatggaac 4980
cttaaatata taatgttgcc agcgatttta gttcaatatt tgttactgtt atctatctgc 5040
tgtatatgga attcttttaa ttcaaacgct gaaaagaatc agcatttagt cttgccaggc 5100
acacccaata atcagtcatg tgtaatatgc acaagtttgt ttttgttttt gttttttttg 5160
ttggttggtt tgtttttttg ctttaagttg catgatcttt ctgcaggaaa tagtcactca 5220

tcccactcca cataaggggt ttagtaagag aagtctgtct gtctgatgat ggataggggg 5280

caaatctttt tcccctttct gttaatagtc atcacatttc tatgccaaac aggaacaatc 5340

cataacttta gtcttaatgt acacattgca ttttgataaa attaattttg ttgtttcctt 5400

tgaggttgat cgttgtgttg ttgttttgct gcacttttta ctttttttgcg tgtggagctg 5460

tattcccgag accaacgaag cgttgggata cttcattaaa tgtagcgact gtcaacagcg 5520

tgcaggtttt ctgtttctgt gttgtggggt caaccgtaca atggtgtggg agtgacgatg 5580

atgtgaatat ttagaatgta ccatattttt tgtaaattat ttatgttttt ctaaacaaat 5640

ttatcgtata ggttgatgaa acgtcatgtg ttttgccaaa gactgtaaat atttatttat 5700

gtgttcacat ggtcaaaatt tcaccactga aaccctgcac ttagctagaa cctcattttt 5760

aaagattaac aacaggaaat aaattgtaaa aaaggttttc tatacatgaa aaaaaaaaaa 5820

aaaaaa 5826

<210> 12

<211> 1172

<212> PRT

<213> Homo sapiens

<220>

<221> thrombospondin 2 polypeptide sequence

<222> (1)..(1172)

<400> 12

Met Val Trp Arg Leu Val Leu Leu Ala Leu Trp Val Trp Pro Ser Thr

1 5 10 15

Gln Ala Gly His Gln Asp Lys Asp Thr Thr Phe Asp Leu Phe Ser Ile

20 25 30

Ser Asn Ile Asn Arg Lys Thr Ile Gly Ala Lys Gln Phe Arg Gly Pro

35 40 45

Asp Pro Gly Val Pro Ala Tyr Arg Phe Val Arg Phe Asp Tyr Ile Pro

50 55 60

Pro Val Asn Ala Asp Asp Leu Ser Lys Ile Thr Lys Ile Met Arg Gln

65 70 75 80

Lys Glu Gly Phe Phe Leu Thr Ala Gln Leu Lys Gln Asp Gly Lys Ser

85 90 95

Arg Gly Thr Leu Leu Ala Leu Glu Gly Pro Gly Leu Ser Gln Arg Gln

100 105 110

Phe Glu Ile Val Ser Asn Gly Pro Ala Asp Thr Leu Asp Leu Thr Tyr

115 120 125

Trp Ile Asp Gly Thr Arg His Val Val Ser Leu Glu Asp Val Gly Leu

130 135 140

Ala Asp Ser Gln Trp Lys Asn Val Thr Val Gln Val Ala Gly Glu Thr

145 150 155 160

Tyr Ser Leu His Val Gly Cys Asp Leu Ile Asp Ser Phe Ala Leu Asp

165 170 175

Glu Pro Phe Tyr Glu His Leu Gln Ala Glu Lys Ser Arg Met Tyr Val

180 185 190

Ala Lys Gly Ser Ala Arg Glu Ser His Phe Arg Gly Leu Leu Gln Asn

195 200 205

Val His Leu Val Phe Glu Asn Ser Val Glu Asp Ile Leu Ser Lys Lys

210 215 220

Gly Cys Gln Gln Gly Gln Gly Ala Glu Ile Asn Ala Ile Ser Glu Asn

225 230 235 240

Thr Glu Thr Leu Arg Leu Gly Pro His Val Thr Thr Glu Tyr Val Gly

245 250 255

Pro Ser Ser Glu Arg Arg Pro Glu Val Cys Glu Arg Ser Cys Glu Glu

260 265 270

Leu Gly Asn Met Val Gln Glu Leu Ser Gly Leu His Val Leu Val Asn

275 280 285

Gln Leu Ser Glu Asn Leu Lys Arg Val Ser Asn Asp Asn Gln Phe Leu

290 295 300

Trp Glu Leu Ile Gly Gly Pro Pro Lys Thr Arg Asn Met Ser Ala Cys

305 310 315 320

Trp Gln Asp Gly Arg Phe Phe Ala Glu Asn Glu Thr Trp Val Val Asp

325 330 335

Ser Cys Thr Thr Cys Thr Cys Lys Lys Phe Lys Thr Ile Cys His Gln

340 345 350

Ile Thr Cys Pro Pro Ala Thr Cys Ala Ser Pro Ser Phe Val Glu Gly

355 360 365

Glu Cys Cys Pro Ser Cys Leu His Ser Val Asp Gly Glu Glu Gly Trp

370 375 380

Ser Pro Trp Ala Glu Trp Thr Gln Cys Ser Val Thr Cys Gly Ser Gly

385 390 395 400

Thr Gln Gln Arg Gly Arg Ser Cys Asp Val Thr Ser Asn Thr Cys Leu
405 410 415

Gly Pro Ser Ile Gln Thr Arg Ala Cys Ser Leu Ser Lys Cys Asp Thr
420 425 430

Arg Ile Arg Gln Asp Gly Gly Trp Ser His Trp Ser Pro Trp Ser Ser
435 440 445

Cys Ser Val Thr Cys Gly Val Gly Asn Ile Thr Arg Ile Arg Leu Cys
450 455 460

Asn Ser Pro Val Pro Gln Met Gly Gly Lys Asn Cys Lys Gly Ser Gly
465 470 475 480

Arg Glu Thr Lys Ala Cys Gln Gly Ala Pro Cys Pro Ile Asp Gly Arg
485 490 495

Trp Ser Pro Trp Ser Pro Trp Ser Ala Cys Thr Val Thr Cys Ala Gly
500 505 510

Gly Ile Arg Glu Arg Thr Arg Val Cys Asn Ser Pro Glu Pro Gln Tyr
515 520 525

Gly Gly Lys Ala Cys Val Gly Asp Val Gln Glu Arg Gln Met Cys Asn
530 535 540

Lys Arg Ser Cys Pro Val Asp Gly Cys Leu Ser Asn Pro Cys Phe Pro
545 550 555 560

Gly Ala Gln Cys Ser Ser Phe Pro Asp Gly Ser Trp Ser Cys Gly Ser
565 570 575

Cys Pro Val Gly Phe Leu Gly Asn Gly Thr His Cys Glu Asp Leu Asp
580 585 590

Glu Cys Ala Leu Val Pro Asp Ile Cys Phe Ser Thr Ser Lys Val Pro

595 600 605

Arg Cys Val Asn Thr Gln Pro Gly Phe His Cys Leu Pro Cys Pro Pro

610 615 620

Arg Tyr Arg Gly Asn Gln Pro Val Gly Val Gly Leu Glu Ala Ala Lys

625 630 635 640

Thr Glu Lys Gln Val Cys Glu Pro Glu Asn Pro Cys Lys Asp Lys Thr

645 650 655

His Asn Cys His Lys His Ala Glu Cys Ile Tyr Leu Gly His Phe Ser

660 665 670

Asp Pro Met Tyr Lys Cys Glu Cys Gln Thr Gly Tyr Ala Gly Asp Gly

675 680 685

Leu Ile Cys Gly Glu Asp Ser Asp Leu Asp Gly Trp Pro Asn Leu Asn

690 695 700

Leu Val Cys Ala Thr Asn Ala Thr Tyr His Cys Ile Lys Asp Asn Cys

705 710 715 720

Pro His Leu Pro Asn Ser Gly Gln Glu Asp Phe Asp Lys Asp Gly Ile

725 730 735

Gly Asp Ala Cys Asp Asp Asp Asp Asp Asn Asp Gly Val Thr Asp Glu

740 745 750

Lys Asp Asn Cys Gln Leu Leu Phe Asn Pro Arg Gln Ala Asp Tyr Asp

755 760 765

Lys Asp Glu Val Gly Asp Arg Cys Asp Asn Cys Pro Tyr Val His Asn

770 775 780

Pro Ala Gln Ile Asp Thr Asp Asn Asn Gly Glu Gly Asp Ala Cys Ser
785 790 795 800

Val Asp Ile Asp Gly Asp Asp Val Phe Asn Glu Arg Asp Asn Cys Pro
805 810 815

Tyr Val Tyr Asn Thr Asp Gln Arg Asp Thr Asp Gly Asp Gly Val Gly
820 825 830

Asp His Cys Asp Asn Cys Pro Leu Val His Asn Pro Asp Gln Thr Asp
835 840 845

Val Asp Asn Asp Leu Val Gly Asp Gln Cys Asp Asn Asn Glu Asp Ile
850 855 860

Asp Asp Asp Gly His Gln Asn Asn Gln Asp Asn Cys Pro Tyr Ile Ser
865 870 875 880

Asn Ala Asn Gln Ala Asp His Asp Arg Asp Gly Gln Gly Asp Ala Cys
885 890 895

Asp Pro Asp Asp Asp Asn Asp Gly Val Pro Asp Asp Arg Asp Asn Cys
900 905 910

Arg Leu Val Phe Asn Pro Asp Gln Glu Asp Leu Asp Gly Asp Gly Arg
915 920 925

Gly Asp Ile Cys Lys Asp Asp Phe Asp Asn Asp Asn Ile Pro Asp Ile
930 935 940

Asp Asp Val Cys Pro Glu Asn Asn Ala Ile Ser Glu Thr Asp Phe Arg
945 950 955 960

Asn Phe Gln Met Val Pro Leu Asp Pro Lys Gly Thr Thr Gln Ile Asp
965 970 975

Pro Asn Trp Val Ile Arg His Gln Gly Lys Glu Leu Val Gln Thr Ala

980 985 990

Asn Ser Asp Pro Gly Ile Ala Val Gly Phe Asp Glu Phe Gly Ser Val

995 1000 1005

Asp Phe Ser Gly Thr Phe Tyr Val Asn Thr Asp Arg Asp Asp Asp

1010 1015 1020

Tyr Ala Gly Phe Val Phe Gly Tyr Gln Ser Ser Ser Arg Phe Tyr

1025 1030 1035

Val Val Met Trp Lys Gln Val Thr Gln Thr Tyr Trp Glu Asp Gln

1040 1045 1050

Pro Thr Arg Ala Tyr Gly Tyr Ser Gly Val Ser Leu Lys Val Val

1055 1060 1065

Asn Ser Thr Thr Gly Thr Gly Glu His Leu Arg Asn Ala Leu Trp

1070 1075 1080

His Thr Gly Asn Thr Pro Gly Gln Val Arg Thr Leu Trp His Asp

1085 1090 1095

Pro Arg Asn Ile Gly Trp Lys Asp Tyr Thr Ala Tyr Arg Trp His

1100 1105 1110

Leu Thr His Arg Pro Lys Thr Gly Tyr Ile Arg Val Leu Val His

1115 1120 1125

Glu Gly Lys Gln Val Met Ala Asp Ser Gly Pro Ile Tyr Asp Gln

1130 1135 1140

Thr Tyr Ala Gly Gly Arg Leu Gly Leu Phe Val Phe Ser Gln Glu

1145 1150 1155

Met Val Tyr Phe Ser Asp Leu Lys Tyr Glu Cys Arg Asp Ile

1160 1165 1170

<210> 13

<211> 2313

<212> DNA

<213> Homo sapiens

<220>

<221> fibulin-1 C mRNA

<222> (1)..(2313)

<400> 13

ctcctcccgg gcgggataat tgaacggcgc ggccctggcc cagcgttggc tgccgaggct 60

cggccggagc gtggagcccg cgccgctgcc ccaggaccgc gcccgcgcct ttgtccgccg 120

ccgcccaccg cccgtcgccc gccgcccatg gagcgcgccg cgccgtcgcg ccgggtcccg 180

cttccgctgc tgctgctcgg cggccttgcg ctgctggcgg ccggagtgga cgcggatgtc 240

ctcctggagg cctgctgtgc ggacggacac cggatggcca ctcatcagaa ggactgctcg 300

ctgccatatg ctacggaatc caaagaatgc aggatggtgc aggagcagtg ctgccacagc 360

cagctggagg agctgcactg tgccacgggc atcagcctgg ccaacgagca ggaccgctgt 420

gccacgcccc acggtgacaa cgccagcctg gaggccacat ttgtgaagag gtgctgccat 480

tgctgtctgc tggggagggc ggcccaggcc cagggccaga gctgcgagta cagcctcatg 540

gttggctacc agtgtggaca ggtcttccgg gcatgctgtg tcaagagcca ggagaccgga 600

gatttggatg tcgggggcct ccaagaaacg gataagatca ttgaggttga ggaggaacaa 660

gaggacccat atctgaatga ccgctgccga ggaggcgggc cctgcaagca gcagtgccga 720

gacacgggtg acgaggtggt ctgctcctgc ttcgtgggct accagctgct gtctgatggt 780

gtctcctgtg aagatgtcaa tgaatgcatc acgggcagcc acagctgccg gcttggagaa 840
tcctgcatca acacagtggg ctctttccgc tgccagcggg acagcagctg cgggactggc 900
tatgagctca cagaggacaa tagctgcaaa gatattgacg agtgtgagag tggtattcat 960
aactgcctcc ccgattttat ctgtcagaat actctgggat ccttccgctg ccgacccaag 1020
ctacagtgca agagtggctt tatacaagat gctctaggca actgtattga tatcaatgag 1080
tgtttgagta tcagtgcccc gtgccctatc gggcatacat gcatcaacac agagggctcc 1140
tacacgtgcc agaagaacgt gcccaactgt ggccgtggct accatctcaa cgaggaggga 1200
acgcgctgtg ttgatgtgga cgagtgcgcg ccacctgctg agccctgtgg gaagggacat 1260
cgctgcgtga actctcccgg cagtttccgc tgcgaatgca agacgggtta ctattttgac 1320
ggcatcagca ggatgtgtgt cgatgtcaac gagtgccagc gctaccccgg gcgcctgtgt 1380
ggccacaagt gcgagaacac gctgggctcc tacctctgca gctgttccgt gggcttccgg 1440
ctctctgtgg atggcaggtc atgtgaagac atcaatgagt gcagcagcag cccctgtagc 1500
caggagtgtg ccaacgtcta cggctcctac cagtgttact gccggcgagg ctaccagctc 1560
agcgatgtgg atggagtcac ctgtgaagac atcgacgagt gcgccctgcc caccgggggc 1620
cacatctgct cctaccgctg catcaacatc cctggaagct tccagtgcag ctgcccctcg 1680
tctggctaca ggctggcccc caatggccgc aactgccaag acattgatga gtgtgtgact 1740
ggcatccaca actgctccat caacgagacc tgcttcaaca tccagggcgg cttccgctgc 1800
ctggccttcg agtgccctga gaactaccgc cgctccgcag ccacccgctg tgagcgcttg 1860
ccttgccatg agaatcggga gtgctccaag ctgcctctga gaataaccta ctaccacctc 1920
tctttcccca ccaacatcca agcgcccgcg gtggttttcc gcatgggccc ctccagtgct 1980
gtccccgggg acagcatgca gctggccatc accggcggca atgaggaggg ctttttcacc 2040
acccggaagg tgagccccca cagtggggtg gtggccctca ccaagcctgt ccccgagccc 2100
agggacttgc tcctgaccgt caagatggat ctctctcgcc acggcaccgt cagctccttt 2160
gtggccaagc ttttcatctt tgtgtctgca gagctctgag cactcgcttc gcgtcgcggg 2220

gtctccctcc tgttgctttc ctaaccctgc cctccggggc gttaataaag tcttagcaag 2280

cgtcccacac agtgaaaaaa aaaaaaaaaa aaa 2313

<210> 14

<211> 683

<212> PRT

<213> Homo sapiens

<220>

<221> fibulin 1 C polypeptide sequence

<222> (1)..(683)

<400> 14

Met Glu Arg Ala Ala Pro Ser Arg Arg Val Pro Leu Pro Leu Leu Leu
1 5 10 15

Leu Gly Gly Leu Ala Leu Leu Ala Ala Gly Val Asp Ala Asp Val Leu
20 25 30

Leu Glu Ala Cys Cys Ala Asp Gly His Arg Met Ala Thr His Gln Lys
35 40 45

Asp Cys Ser Leu Pro Tyr Ala Thr Glu Ser Lys Glu Cys Arg Met Val
50 55 60

Gln Glu Gln Cys Cys His Ser Gln Leu Glu Glu Leu His Cys Ala Thr
65 70 75 80

Gly Ile Ser Leu Ala Asn Glu Gln Asp Arg Cys Ala Thr Pro His Gly
85 90 95

Asp Asn Ala Ser Leu Glu Ala Thr Phe Val Lys Arg Cys Cys His Cys
100 105 110

Cys Leu Leu Gly Arg Ala Ala Gln Ala Gln Gly Gln Ser Cys Glu Tyr
115 120 125

Ser Leu Met Val Gly Tyr Gln Cys Gly Gln Val Phe Arg Ala Cys Cys
130 135 140

Val Lys Ser Gln Glu Thr Gly Asp Leu Asp Val Gly Gly Leu Gln Glu
145 150 155 160

Thr Asp Lys Ile Ile Glu Val Glu Glu Glu Gln Glu Asp Pro Tyr Leu
165 170 175

Asn Asp Arg Cys Arg Gly Gly Gly Pro Cys Lys Gln Gln Cys Arg Asp
180 185 190

Thr Gly Asp Glu Val Val Cys Ser Cys Phe Val Gly Tyr Gln Leu Leu
195 200 205

Ser Asp Gly Val Ser Cys Glu Asp Val Asn Glu Cys Ile Thr Gly Ser
210 215 220

His Ser Cys Arg Leu Gly Glu Ser Cys Ile Asn Thr Val Gly Ser Phe
225 230 235 240

Arg Cys Gln Arg Asp Ser Ser Cys Gly Thr Gly Tyr Glu Leu Thr Glu
245 250 255

Asp Asn Ser Cys Lys Asp Ile Asp Glu Cys Glu Ser Gly Ile His Asn
260 265 270

Cys Leu Pro Asp Phe Ile Cys Gln Asn Thr Leu Gly Ser Phe Arg Cys
275 280 285

Arg Pro Lys Leu Gln Cys Lys Ser Gly Phe Ile Gln Asp Ala Leu Gly

290 295 300

Asn Cys Ile Asp Ile Asn Glu Cys Leu Ser Ile Ser Ala Pro Cys Pro

305 310 315 320

Ile Gly His Thr Cys Ile Asn Thr Glu Gly Ser Tyr Thr Cys Gln Lys

325 330 335

Asn Val Pro Asn Cys Gly Arg Gly Tyr His Leu Asn Glu Glu Gly Thr

340 345 350

Arg Cys Val Asp Val Asp Glu Cys Ala Pro Pro Ala Glu Pro Cys Gly

355 360 365

Lys Gly His Arg Cys Val Asn Ser Pro Gly Ser Phe Arg Cys Glu Cys

370 375 380

Lys Thr Gly Tyr Tyr Phe Asp Gly Ile Ser Arg Met Cys Val Asp Val

385 390 395 400

Asn Glu Cys Gln Arg Tyr Pro Gly Arg Leu Cys Gly His Lys Cys Glu

405 410 415

Asn Thr Leu Gly Ser Tyr Leu Cys Ser Cys Ser Val Gly Phe Arg Leu

420 425 430

Ser Val Asp Gly Arg Ser Cys Glu Asp Ile Asn Glu Cys Ser Ser Ser

435 440 445

Pro Cys Ser Gln Glu Cys Ala Asn Val Tyr Gly Ser Tyr Gln Cys Tyr

450 455 460

Cys Arg Arg Gly Tyr Gln Leu Ser Asp Val Asp Gly Val Thr Cys Glu

465 470 475 480

Asp Ile Asp Glu Cys Ala Leu Pro Thr Gly Gly His Ile Cys Ser Tyr

485 490 495

Arg Cys Ile Asn Ile Pro Gly Ser Phe Gln Cys Ser Cys Pro Ser Ser

500 505 510

Gly Tyr Arg Leu Ala Pro Asn Gly Arg Asn Cys Gln Asp Ile Asp Glu

515 520 525

Cys Val Thr Gly Ile His Asn Cys Ser Ile Asn Glu Thr Cys Phe Asn

530 535 540

Ile Gln Gly Gly Phe Arg Cys Leu Ala Phe Glu Cys Pro Glu Asn Tyr

545 550 555 560

Arg Arg Ser Ala Ala Thr Arg Cys Glu Arg Leu Pro Cys His Glu Asn

565 570 575

Arg Glu Cys Ser Lys Leu Pro Leu Arg Ile Thr Tyr Tyr His Leu Ser

580 585 590

Phe Pro Thr Asn Ile Gln Ala Pro Ala Val Val Phe Arg Met Gly Pro

595 600 605

Ser Ser Ala Val Pro Gly Asp Ser Met Gln Leu Ala Ile Thr Gly Gly

610 615 620

Asn Glu Glu Gly Phe Phe Thr Thr Arg Lys Val Ser Pro His Ser Gly

625 630 635 640

Val Val Ala Leu Thr Lys Pro Val Pro Glu Pro Arg Asp Leu Leu Leu

645 650 655

Thr Val Lys Met Asp Leu Ser Arg His Gly Thr Val Ser Ser Phe Val

660 665 670

Ala Lys Leu Phe Ile Phe Val Ser Ala Glu Leu

675 680

<210> 15

<211> 2485

<212> DNA

<213> Homo sapiens

<220>

<221> annexin A11 mRNA

<222> (1)..(2485)

<400> 15

gcactgcctc tggcacctgg ggcagccgcg cccgcggagt tttccgcccg gcgctgacgg 60

ctgctgcgcc cgcggctccc cagtgccccg agtgccccgc gggccccgcg agcgggagtg 120

ggacccagcc ctaggcagaa cccaggcgcc gcgcccggga cgcccgcgga gagagccact 180

cccgcccacg tcccatttcg cccctcgcgt ccggagtccc cgtggccaga tctaaccatg 240

agctaccctg gctatccccc gcccccaggt ggctacccac cagctgcacc aggtggtggt 300

ccctggggag gtgctgccta ccctcctccg cccagcatgc cccccatcgg gctggataac 360

gtggccacct atgcggggca gttcaaccag gactatctct cgggaatggc ggccaacatg 420

tctgggacat ttggaggagc caacatgccc aacctgtacc ctggggcccc tggggctggc 480

tacccaccag tgccccctgg cggctttggg cagccccct ctgcccagca gcctgttcct 540

ccctatggga tgtatccacc cccaggagga aacccaccct ccaggatgcc ctcatatccg 600

ccatacccag ggcccctgt gccgggccag cccatgccac cccccggaca gcagccccca 660

ggggcctacc ctgggcagcc accagtgacc taccctggtc agcctccagt gccactccct 720

gggcagcagc agccagtgcc gagctaccca ggatacccgg ggtctgggac tgtcaccccc 780

gctgtgcccc caacccagtt tggaagccga ggcaccatca ctgatgctcc cggctttgac 840

cccctgcgag atgccgaggt cctgcggaag gccatgaaag gcttcgggac ggatgagcag 900

gccatcattg actgcctggg gagtcgctcc aacaagcagc ggcagcagat cctactttcc 960

ttcaagacgg cttacggcaa ggatttgatc aaagatctga aatctgaact gtcaggaaac 1020

tttgagaaga caatcttggc tctgatgaag accccagtcc tctttgacat ttatgagata 1080

aaggaagcca tcaagggggt tggcactgat gaagcctgcc tgattgagat cctcgcttcc 1140

cgcagcaatg agcacatccg agaattaaac agagcctaca aagcagaatt caaaaagacc 1200

ctggaagagg ccattcgaag cgacacatca gggcacttcc agcggctcct catctctctc 1260

tctcagggaa accgtgatga aagcacaaac gtggacatgt cactcgccca gagagatgcc 1320

caggagctgt atgcggccgg ggagaaccgc ctgggaacag acgagtccaa gttcaatgcg 1380

gttctgtgct cccggagccg ggcccacctg gtagcagttt tcaatgagta ccagagaatg 1440

acaggccggg acattgagaa gagcatctgc cgggagatgt ccggggacct ggaggagggc 1500

atgctggccg tggtgaaatg tctcaagaat accccagcct tctttgcgga gaggctcaac 1560

aaggccatga gggggcagg aacaaaggac cggaccctga ttcgcatcat ggtgtctcgc 1620

agcgagaccg acctcctgga catcagatca gagtataagc ggatgtacgg caagtcgctg 1680

taccacgaca tctcgggaga tacttcaggg gattaccgga agattctgct gaagatctgt 1740

ggtggcaatg actgaacagt gactggtggc tcacttctgc ccacctgccg gcaacaccag 1800

tgccaggaaa aggccaaaag aatgtctgtt tctaacaaat ccacaaatag ccccgagatt 1860

caccgtccta gagcttaggc ctgtcttcca cccctcctga cccgtatagt gtgccacagg 1920

acctgggtcg gtctagaact ctctcaggat gccttttcta ccccatccct cacagcctct 1980

tgctgctaaa atagatgttt cattttctg actcatgcaa tcattcccct ttgcctgtgg 2040

ctaagacttg gcttcatttc gtcatgtaat tgtatatttt tatttggagg catattttct 2100

tttcttacag tcattgccag acagaggcat acaagtctgt ttgctgcata cacatttctg 2160

gtgagggcga ctgggtgggt gaagcaccgt gtcctcgctg aggagagaaa gggaggcgtg 2220

cctgagaggg tagcctgtgc atctggtgag tgtgtcacga gctttgttac tgccaaactc 2280

actccttttt agaaaaaaca aaaaaaaagg gccagaaagt cattccttcc atcttccttg 2340

cagaaaccac gagaacaaag ccagttccct gtcagtgaca gggcttcttg taatttgtgg 2400

tatgtgcctt aaacctgaat gtctgtagcc aaaacttgtt tccacattaa gagtcagcca 2460

gctctggaat ggtctggaaa tgtca 2485

<210> 16

<211> 505

<212> PRT

<213> Homo sapiens

<220>

<221> annexin A11 polypeptide sequence

<222> (1)..(505)

<400> 16

Met Ser Tyr Pro Gly Tyr Pro Pro Pro Pro Gly Gly Tyr Pro Pro Ala
1 5 10 15

Ala Pro Gly Gly Gly Pro Trp Gly Gly Ala Ala Tyr Pro Pro Pro Pro
20 25 30

Ser Met Pro Pro Ile Gly Leu Asp Asn Val Ala Thr Tyr Ala Gly Gln
35 40 45

Phe Asn Gln Asp Tyr Leu Ser Gly Met Ala Ala Asn Met Ser Gly Thr
50 55 60

Phe Gly Gly Ala Asn Met Pro Asn Leu Tyr Pro Gly Ala Pro Gly Ala

65 70 75 80

Gly Tyr Pro Pro Val Pro Pro Gly Gly Phe Gly Gln Pro Pro Ser Ala

85 90 95

Gln Gln Pro Val Pro Pro Tyr Gly Met Tyr Pro Pro Pro Gly Gly Asn

100 105 110

Pro Pro Ser Arg Met Pro Ser Tyr Pro Pro Tyr Pro Gly Ala Pro Val

115 120 125

Pro Gly Gln Pro Met Pro Pro Pro Gly Gln Gln Pro Pro Gly Ala Tyr

130 135 140

Pro Gly Gln Pro Pro Val Thr Tyr Pro Gly Gln Pro Pro Val Pro Leu

145 150 155 160

Pro Gly Gln Gln Gln Pro Val Pro Ser Tyr Pro Gly Tyr Pro Gly Ser

165 170 175

Gly Thr Val Thr Pro Ala Val Pro Pro Thr Gln Phe Gly Ser Arg Gly

180 185 190

Thr Ile Thr Asp Ala Pro Gly Phe Asp Pro Leu Arg Asp Ala Glu Val

195 200 205

Leu Arg Lys Ala Met Lys Gly Phe Gly Thr Asp Glu Gln Ala Ile Ile

210 215 220

Asp Cys Leu Gly Ser Arg Ser Asn Lys Gln Arg Gln Gln Ile Leu Leu

225 230 235 240

Ser Phe Lys Thr Ala Tyr Gly Lys Asp Leu Ile Lys Asp Leu Lys Ser

245 250 255

Glu Leu Ser Gly Asn Phe Glu Lys Thr Ile Leu Ala Leu Met Lys Thr

260 265 270

Pro Val Leu Phe Asp Ile Tyr Glu Ile Lys Glu Ala Ile Lys Gly Val

275 280 285

Gly Thr Asp Glu Ala Cys Leu Ile Glu Ile Leu Ala Ser Arg Ser Asn

290 295 300

Glu His Ile Arg Glu Leu Asn Arg Ala Tyr Lys Ala Glu Phe Lys Lys

305 310 315 320

Thr Leu Glu Glu Ala Ile Arg Ser Asp Thr Ser Gly His Phe Gln Arg

325 330 335

Leu Leu Ile Ser Leu Ser Gln Gly Asn Arg Asp Glu Ser Thr Asn Val

340 345 350

Asp Met Ser Leu Ala Gln Arg Asp Ala Gln Glu Leu Tyr Ala Ala Gly

355 360 365

Glu Asn Arg Leu Gly Thr Asp Glu Ser Lys Phe Asn Ala Val Leu Cys

370 375 380

Ser Arg Ser Arg Ala His Leu Val Ala Val Phe Asn Glu Tyr Gln Arg

385 390 395 400

Met Thr Gly Arg Asp Ile Glu Lys Ser Ile Cys Arg Glu Met Ser Gly

405 410 415

Asp Leu Glu Glu Gly Met Leu Ala Val Val Lys Cys Leu Lys Asn Thr

420 425 430

Pro Ala Phe Phe Ala Glu Arg Leu Asn Lys Ala Met Arg Gly Ala Gly

435 440 445

Thr Lys Asp Arg Thr Leu Ile Arg Ile Met Val Ser Arg Ser Glu Thr

450 455 460

Asp Leu Leu Asp Ile Arg Ser Glu Tyr Lys Arg Met Tyr Gly Lys Ser

465 470 475 480

Leu Tyr His Asp Ile Ser Gly Asp Thr Ser Gly Asp Tyr Arg Lys Ile

485 490 495

Leu Leu Lys Ile Cys Gly Gly Asn Asp

500 505

<210> 17

<211> 3309

<212> DNA

<213> Homo sapiens

<220>

<221> Protein S mRNA

<222> (1)..(3309)

<400> 17

ctgcaggggg gggggggggg gggggggggg ggggggggcg cagcacggct cagaccgagg 60

cgcacaggct cgcagctccg ggcgcctagc gcccggtccc cgccgcgacg cgccaccgtc 120

cctgccggcg cctccgcgcc ttcgaaatga gggtcctggg tgggcgctgc ggggcgccgc 180

tggcgtgtct cctcctagtg cttcccgtct cagaggcaaa ccttctgtca aagcaacagg 240

cttcacaagt cctggttagg aagcgtcgtg caaattcttt acttgaagaa accaaacagg 300

gtaatcttga aagagaatgc atcgaagaac tgtgcaataa agaagaagcc agggaggtct 360
ttgaaaatga cccggaaacg gattattttt atccaaaata cttagtttgt cttcgctctt 420
ttcaaactgg gttattcact gctgcacgtc agtcaactaa tgcttatcct gacctaagaa 480
gctgtgtcaa tgccattcca gaccagtgta gtcctctgcc atgcaatgaa gatggatata 540
tgagctgcaa agatggaaaa gcttctttta cttgcacttg taaaccaggt tggcaaggag 600
aaaagtgtga atttgacata aatgaatgca aagatccctc aaatataaat ggaggttgca 660
gtcaaatttg tgataataca cctggaagtt accactgttc ctgtaaaaat ggttttgtta 720
tgctttcaaa taagaaagat tgtaaagatg tggatgaatg ctctttgaag ccaagcattt 780
gtggcacagc tgtgtgcaag aacatcccag gagattttga atgtgaatgc cccgaaggct 840
acagatataa tctcaaatca aagtcttgtg aagatataga tgaatgctct gagaacatgt 900
gtgctcagct ttgtgtcaat taccctggag gttacacttg ctattgtgat gggaagaaag 960
gattcaaact tgcccaagat cagaagagtt gtgaggttgt ttcagtgtgc cttcccttga 1020
accttgacac aaagtatgaa ttactttact tggcggagca gtttgcaggg gttgttttat 1080
atttaaaatt tcgtttgcca gaaatcagca gattttcagc agaatttgat ttccggacat 1140
atgattcaga aggcgtgata ctgtacgcag aatctatcga tcactcagcg tggctcctga 1200
ttgcacttcg tggtggaaag attgaagttc agcttaagaa tgaacataca tccaaaatca 1260
caactggagg tgatgttatt aataatggtc tatggaatat ggtgtctgtg gaagaattag 1320
aacatagtat tagcattaaa atagctaaag aagctgtgat ggatataaat aaacctggac 1380
cccttttttaa gccggaaaat ggattgctgg aaaccaaagt atactttgca ggattccctc 1440
ggaaagtgga aagtgaactc attaaaccga ttaaccctcg tctagatgga tgtatacgaa 1500
gctggaattt gatgaagcaa ggagcttctg gaataaagga aattattcaa gaaaaacaaa 1560
ataagcattg cctggttact gtggagaagg gctcctacta tcctggttct ggaattgctc 1620
aatttcacat agattataat aatgtatcca gtgctgaggg ttggcatgta aatgtgacct 1680
tgaatattcg tccatccacg ggcactggtg ttatgcttgc cttggtttct ggtaacaaca 1740

cagtgccctt tgctgtgtcc ttggtggact ccacctctga aaaatcacag gatattctgt 1800

tatctgttga aaatactgta atatatcgga tacaggccct aagtctatgt tccgatcaac 1860

aatctcatct ggaatttaga gtcaacagaa acaatctgga gttgtcgaca ccacttaaaa 1920

tagaaaccat ctcccatgaa gaccttcaaa gacaacttgc cgtcttggac aaagcaatga 1980

aagcaaaagt ggccacatac ctgggtggcc ttccagatgt tccattcagt gccacaccag 2040

tgaatgcctt ttataatggc tgcatggaag tgaatattaa tggtgtacag ttggatctgg 2100

atgaagccat ttctaaacat aatgatatta gagctcactc atgtccatca gtttggaaaa 2160

agacaaagaa ttcttaaggc atcttttctc tgcttataat accttttcct tgtgtgtaat 2220

tatacttatg tttcaataac agctgaaggg ttttatttac aatgtgcagt ctttgattat 2280

tttgtggtcc tttcctggga tttttaaaag gtcctttgtc aaggaaaaaa attctgttgt 2340

gatataaatc acagtaaaga aattcttact tctcttgcta tctaagaata gtgaaaaata 2400

acaattttaa atttgaattt ttttcctaca aatgacagtt tcaatttttg tttgtaaaac 2460

taaattttaa ttttatcatc atgaactagt gtctaaatac ctatgttttt ttcagaaagc 2520

aaggaagtaa actcaaacaa aagtgcgtgt aattaaatac tattaatcat aggcagatac 2580

tattttgttt atgtttttgt tttttcctg atgaaggcag aagagatggt ggtctattaa 2640

atatgaattg aatggagggt cctaatgcct tatttcaaaa caattcctca gggggaccag 2700

ctttggcttc atctttctct tgtgtggctt cacatttaaa ccagtatctt tattgaatta 2760

gaaaacaagt gggacatatt ttcctgagag cagcacagga atcttcttct tggcagctgc 2820

agtctgtcag gatgagatat cagattaggt tggataggtg gggaaatctg aagtgggtac 2880

attttttaaa ttttgctgtg tgggtcacac aaggtctaca ttacaaaaga cagaattcag 2940

ggatggaaag gagaatgaac aaatgtggga gttcatagtt ttccttgaat ccaactttta 3000

attaccagag taagttgcca aaatgtgatt gttgaagtac aaaaggaact atgaaaacca 3060

gaacaaattt taacaaaagg acaaccacag agggatatag tgaatatcgt atcattgtaa 3120

tcaaagaagt aaggaggtaa gattgccacg tgcctgctgg tactgtgatg catttcaagt 3180

ggcagtttta tcacgtttga atctaccatt catagccaga tgtgtatcag atgtttcact 3240

gacagttttt aacaataaat tcttttcact gtattttata tcacttataa taaatcggtg 3300

tataatttt 3309

<210> 18

<211> 676

<212> PRT

<213> Homo sapiens

<220>

<221> Protein S polypeptide sequence

<222> (1)..(676)

<400> 18

Met Arg Val Leu Gly Gly Arg Cys Gly Ala Pro Leu Ala Cys Leu Leu
1 5 10 15

Leu Val Leu Pro Val Ser Glu Ala Asn Leu Leu Ser Lys Gln Gln Ala
20 25 30

Ser Gln Val Leu Val Arg Lys Arg Arg Ala Asn Ser Leu Leu Glu Glu
35 40 45

Thr Lys Gln Gly Asn Leu Glu Arg Glu Cys Ile Glu Glu Leu Cys Asn
50 55 60

Lys Glu Glu Ala Arg Glu Val Phe Glu Asn Asp Pro Glu Thr Asp Tyr
65 70 75 80

Phe Tyr Pro Lys Tyr Leu Val Cys Leu Arg Ser Phe Gln Thr Gly Leu

85 90 95

Phe Thr Ala Ala Arg Gln Ser Thr Asn Ala Tyr Pro Asp Leu Arg Ser

100 105 110

Cys Val Asn Ala Ile Pro Asp Gln Cys Ser Pro Leu Pro Cys Asn Glu

115 120 125

Asp Gly Tyr Met Ser Cys Lys Asp Gly Lys Ala Ser Phe Thr Cys Thr

130 135 140

Cys Lys Pro Gly Trp Gln Gly Glu Lys Cys Glu Phe Asp Ile Asn Glu

145 150 155 160

Cys Lys Asp Pro Ser Asn Ile Asn Gly Gly Cys Ser Gln Ile Cys Asp

165 170 175

Asn Thr Pro Gly Ser Tyr His Cys Ser Cys Lys Asn Gly Phe Val Met

180 185 190

Leu Ser Asn Lys Lys Asp Cys Lys Asp Val Asp Glu Cys Ser Leu Lys

195 200 205

Pro Ser Ile Cys Gly Thr Ala Val Cys Lys Asn Ile Pro Gly Asp Phe

210 215 220

Glu Cys Glu Cys Pro Glu Gly Tyr Arg Tyr Asn Leu Lys Ser Lys Ser

225 230 235 240

Cys Glu Asp Ile Asp Glu Cys Ser Glu Asn Met Cys Ala Gln Leu Cys

245 250 255

Val Asn Tyr Pro Gly Gly Tyr Thr Cys Tyr Cys Asp Gly Lys Lys Gly

260 265 270

Phe Lys Leu Ala Gln Asp Gln Lys Ser Cys Glu Val Val Ser Val Cys

275 280 285

Leu Pro Leu Asn Leu Asp Thr Lys Tyr Glu Leu Leu Tyr Leu Ala Glu

290 295 300

Gln Phe Ala Gly Val Val Leu Tyr Leu Lys Phe Arg Leu Pro Glu Ile

305 310 315 320

Ser Arg Phe Ser Ala Glu Phe Asp Phe Arg Thr Tyr Asp Ser Glu Gly

325 330 335

Val Ile Leu Tyr Ala Glu Ser Ile Asp His Ser Ala Trp Leu Leu Ile

340 345 350

Ala Leu Arg Gly Gly Lys Ile Glu Val Gln Leu Lys Asn Glu His Thr

355 360 365

Ser Lys Ile Thr Thr Gly Gly Asp Val Ile Asn Asn Gly Leu Trp Asn

370 375 380

Met Val Ser Val Glu Glu Leu Glu His Ser Ile Ser Ile Lys Ile Ala

385 390 395 400

Lys Glu Ala Val Met Asp Ile Asn Lys Pro Gly Pro Leu Phe Lys Pro

405 410 415

Glu Asn Gly Leu Leu Glu Thr Lys Val Tyr Phe Ala Gly Phe Pro Arg

420 425 430

Lys Val Glu Ser Glu Leu Ile Lys Pro Ile Asn Pro Arg Leu Asp Gly

435 440 445

Cys Ile Arg Ser Trp Asn Leu Met Lys Gln Gly Ala Ser Gly Ile Lys

450 455 460

Glu Ile Ile Gln Glu Lys Gln Asn Lys His Cys Leu Val Thr Val Glu

465 470 475 480

Lys Gly Ser Tyr Tyr Pro Gly Ser Gly Ile Ala Gln Phe His Ile Asp

485 490 495

Tyr Asn Asn Val Ser Ser Ala Glu Gly Trp His Val Asn Val Thr Leu

500 505 510

Asn Ile Arg Pro Ser Thr Gly Thr Gly Val Met Leu Ala Leu Val Ser

515 520 525

Gly Asn Asn Thr Val Pro Phe Ala Val Ser Leu Val Asp Ser Thr Ser

530 535 540

Glu Lys Ser Gln Asp Ile Leu Leu Ser Val Glu Asn Thr Val Ile Tyr

545 550 555 560

Arg Ile Gln Ala Leu Ser Leu Cys Ser Asp Gln Gln Ser His Leu Glu

565 570 575

Phe Arg Val Asn Arg Asn Asn Leu Glu Leu Ser Thr Pro Leu Lys Ile

580 585 590

Glu Thr Ile Ser His Glu Asp Leu Gln Arg Gln Leu Ala Val Leu Asp

595 600 605

Lys Ala Met Lys Ala Lys Val Ala Thr Tyr Leu Gly Gly Leu Pro Asp

610 615 620

Val Pro Phe Ser Ala Thr Pro Val Asn Ala Phe Tyr Asn Gly Cys Met

625 630 635 640

Glu Val Asn Ile Asn Gly Val Gln Leu Asp Leu Asp Glu Ala Ile Ser

645 650 655

Lys His Asn Asp Ile Arg Ala His Ser Cys Pro Ser Val Trp Lys Lys

660 665 670

Thr Lys Asn Ser

675

<210> 19

<211> 3926

<212> DNA

<213> Homo sapiens

<220>

<221> H factor 1 (complement) mRNA

<222> (1)..(3926)

<400> 19

aattcttgga agaggagaac tggacgttgt gaacagagtt agctggtaaa tgtcctctta 60

aaagatccaa aaaatgagac ttctagcaaa gattatttgc cttatgttat gggctatttg 120

tgtagcagaa gattgcaatg aacttcctcc aagaagaaat acagaaattc tgacaggttc 180

ctggtctgac caaacatatc cagaaggcac ccaggctatc tataaatgcc gccctggata 240

tagatctctt ggaaatgtaa taatggtatg caggaaggga gaatgggttg ctcttaatcc 300

attaaggaaa tgtcagaaaa ggccctgtgg acatcctgga gatactcctt ttggtacttt 360

tacccttaca ggaggaaatg tgtttgaata tggtgtaaaa gctgtgtata catgtaatga 420

ggggtatcaa ttgctaggtg agattaatta ccgtgaatgt gacacagatg gatggaccaa 480

tgatattcct atatgtgaag ttgtgaagtg tttaccagtg acagcaccag agaatggaaa 540

aattgtcagt agtgcaatgg aaccagatcg ggaataccat tttggacaag cagtacggtt 600

tgtatgtaac tcaggctaca agattgaagg agatgaagaa atgcattgtt cagacgatgg 660

```
tttttggagt aaagagaaac caaagtgtgt ggaaatttca tgcaaatccc cagatgttat   720
aaatggatct cctatatctc agaagattat ttataaggag aatgaacgat ttcaatataa   780
atgtaacatg ggttatgaat acagtgaaag aggagatgct gtatgcactg aatctggatg   840
gcgtccgttg ccttcatgtg aagaaaaatc atgtgataat ccttatattc caaatggtga   900
ctactcacct ttaaggatta aacacagaac tggagatgaa atcacgtacc agtgtagaaa   960
tggtttttat cctgcaaccc ggggaaatac agccaaatgc acaagtactg gctggatacc  1020
tgctccgaga tgtaccttga aaccttgtga ttatccagac attaaacatg gaggtctata  1080
tcatgagaat atgcgtagac catactttcc agtagctgta ggaaaatatt actcctatta  1140
ctgtgatgaa cattttgaga ctccgtcagg aagttactgg gatcacattc attgcacaca  1200
agatggatgg tcgccagcag taccatgcct cagaaaatgt tattttcctt atttggaaaa  1260
tggatataat caaaatcatg gaagaaagtt tgtacagggt aaatctatag acgttgcctg  1320
ccatcctggc tacgctcttc caaaagcgca gaccacagtt acatgtatgg agaatggctg  1380
gtctcctact cccagatgca tccgtgtcaa aacatgttcc aaatcaagta tagatattga  1440
gaatgggttt atttctgaat ctcagtatac atatgcctta aaagaaaaag cgaaatatca  1500
atgcaaacta ggatatgtaa cagcagatgg tgaaacatca ggatcaatta gatgtgggaa  1560
agatggatgg tcagctcaac ccacgtgcat taaatcttgt gatatcccag tatttatgaa  1620
tgccagaact aaaaatgact tcacatggtt taagctgaat gacacattgg actatgaatg  1680
ccatgatggt tatgaaagca atactggaag caccactggt tccatagtgt gtggttacaa  1740
tggttggtct gatttaccca tatgttatga aagagaatgc gaacttccta aaatagatgt  1800
acacttagtt cctgatcgca agaaagacca gtataaagtt ggagaggtgt tgaaattctc  1860
ctgcaaacca ggatttacaa tagttggacc taattccgtt cagtgctacc actttggatt  1920
gtctcctgac ctcccaatat gtaaagagca agtacaatca tgtggtccac ctcctgaact  1980
cctcaatggg aatgttaagg aaaaaacgaa agaagaatat ggacacagtg aagtggtgga  2040
atattattgc aatcctagat ttctaatgaa gggacctaat aaaattcaat gtgttgatgg  2100
```

agagtggaca actttaccag tgtgtattgt ggaggagagt acctgtggag atatacctga 2160

acttgaacat ggctgggccc agctttcttc ccctccttat tactatggag attcagtgga 2220

attcaattgc tcagaatcat ttacaatgat tggacacaga tcaattacgt gtattcatgg 2280

agtatggacc caacttcccc agtgtgtggc aatagataaa cttaagaagt gcaaatcatc 2340

aaatttaatt atacttgagg aacatttaaa aaacaagaag gaattcgatc ataattctaa 2400

cataaggtac agatgtagag gaaaagaagg atggatacac acagtctgca taaatggaag 2460

atgggatcca gaagtgaact gctcaatggc acaaatacaa ttatgcccac ctccacctca 2520

gattcccaat tctcacaata tgacaaccac actgaattat cgggatggag aaaaagtatc 2580

tgttctttgc caagaaaatt atctaattca ggaaggagaa gaaattacat gcaaagatgg 2640

aagatggcag tcaataccac tctgtgttga aaaaattcca tgttcacaac cacctcagat 2700

agaacacgga accattaatt catccaggtc ttcacaagaa agttatgcac atgggactaa 2760

attgagttat acttgtgagg gtggtttcag gatatctgaa gaaaatgaaa caacatgcta 2820

catgggaaaa tggagttctc cacctcagtg tgaaggcctt ccttgtaaat ctccacctga 2880

gatttctcat ggtgttgtag ctcacatgtc agacagttat cagtatggag aagaagttac 2940

gtacaaatgt tttgaaggtt ttggaattga tgggcctgca attgcaaaat gcttaggaga 3000

aaaatggtct caccctccat catgcataaa aacagattgt ctcagtttac ctagctttga 3060

aaatgccata cccatgggag agaagaagga tgtgtataag gcgggtgagc aagtgactta 3120

cacttgtgca acatattaca aaatggatgg agccagtaat gtaacatgca ttaatagcag 3180

atggacagga aggccaacat gcagagacac ctcctgtgtg aatccgccca cagtacaaaa 3240

tgcttatata gtgtcgagac agatgagtaa atatccatct ggtgagagag tacgttatca 3300

atgtaggagc ccttatgaaa tgtttgggga tgaagaagtg atgtgtttaa atggaaactg 3360

gacggaacca cctcaatgca aagattctac aggaaaatgt gggcccccctc cacctattga 3420

caatggggac attacttcat tcccgttgtc agtatatgct ccagcttcat cagttgagta 3480

ccaatgccag aacttgtatc aacttgaggg taacaagcga ataacatgta gaaatggaca 3540

atggtcagaa ccaccaaaat gcttacatcc gtgtgtaata tcccgagaaa ttatggaaaa 3600

ttataacata gcattaaggt ggacagccaa acagaagctt tattcgagaa caggtgaatc 3660

agttgaattt gtgtgtaaac ggggatatcg tctttcatca cgttctcaca cattgcgaac 3720

aacatgttgg gatgggaaac tggagtatcc aacttgtgca aaaagataga atcaatcata 3780

aagtgcacac ctttattcag aactttagta ttaaatcagt tctcaatttc atttttttatg 3840

tattgtttta ctcctttttta ttcatacgta aaattttgga ttaatttgtg aaaatgtaat 3900

tataagctga gaccggtggc tctctt 3926

<210> 20

<211> 1231

<212> PRT

<213> Homo sapiens

<220>

<221> H factor 1 polypeptide sequence

<222> (1)..(1231)

<400> 20

Met Arg Leu Leu Ala Lys Ile Ile Cys Leu Met Leu Trp Ala Ile Cys

1 5 10 15

Val Ala Glu Asp Cys Asn Glu Leu Pro Pro Arg Arg Asn Thr Glu Ile

20 25 30

Leu Thr Gly Ser Trp Ser Asp Gln Thr Tyr Pro Glu Gly Thr Gln Ala

35 40 45

Ile Tyr Lys Cys Arg Pro Gly Tyr Arg Ser Leu Gly Asn Val Ile Met

50 55 60

Val Cys Arg Lys Gly Glu Trp Val Ala Leu Asn Pro Leu Arg Lys Cys

65 70 75 80

Gln Lys Arg Pro Cys Gly His Pro Gly Asp Thr Pro Phe Gly Thr Phe

85 90 95

Thr Leu Thr Gly Gly Asn Val Phe Glu Tyr Gly Val Lys Ala Val Tyr

100 105 110

Thr Cys Asn Glu Gly Tyr Gln Leu Leu Gly Glu Ile Asn Tyr Arg Glu

115 120 125

Cys Asp Thr Asp Gly Trp Thr Asn Asp Ile Pro Ile Cys Glu Val Val

130 135 140

Lys Cys Leu Pro Val Thr Ala Pro Glu Asn Gly Lys Ile Val Ser Ser

145 150 155 160

Ala Met Glu Pro Asp Arg Glu Tyr His Phe Gly Gln Ala Val Arg Phe

165 170 175

Val Cys Asn Ser Gly Tyr Lys Ile Glu Gly Asp Glu Glu Met His Cys

180 185 190

Ser Asp Asp Gly Phe Trp Ser Lys Glu Lys Pro Lys Cys Val Glu Ile

195 200 205

Ser Cys Lys Ser Pro Asp Val Ile Asn Gly Ser Pro Ile Ser Gln Lys

210 215 220

Ile Ile Tyr Lys Glu Asn Glu Arg Phe Gln Tyr Lys Cys Asn Met Gly

225 230 235 240

Tyr Glu Tyr Ser Glu Arg Gly Asp Ala Val Cys Thr Glu Ser Gly Trp

245 250 255

Arg Pro Leu Pro Ser Cys Glu Glu Lys Ser Cys Asp Asn Pro Tyr Ile

260 265 270

Pro Asn Gly Asp Tyr Ser Pro Leu Arg Ile Lys His Arg Thr Gly Asp

275 280 285

Glu Ile Thr Tyr Gln Cys Arg Asn Gly Phe Tyr Pro Ala Thr Arg Gly

290 295 300

Asn Thr Ala Lys Cys Thr Ser Thr Gly Trp Ile Pro Ala Pro Arg Cys

305 310 315 320

Thr Leu Lys Pro Cys Asp Tyr Pro Asp Ile Lys His Gly Gly Leu Tyr

325 330 335

His Glu Asn Met Arg Arg Pro Tyr Phe Pro Val Ala Val Gly Lys Tyr

340 345 350

Tyr Ser Tyr Tyr Cys Asp Glu His Phe Glu Thr Pro Ser Gly Ser Tyr

355 360 365

Trp Asp His Ile His Cys Thr Gln Asp Gly Trp Ser Pro Ala Val Pro

370 375 380

Cys Leu Arg Lys Cys Tyr Phe Pro Tyr Leu Glu Asn Gly Tyr Asn Gln

385 390 395 400

Asn His Gly Arg Lys Phe Val Gln Gly Lys Ser Ile Asp Val Ala Cys

405 410 415

His Pro Gly Tyr Ala Leu Pro Lys Ala Gln Thr Thr Val Thr Cys Met

420 425 430

Glu Asn Gly Trp Ser Pro Thr Pro Arg Cys Ile Arg Val Lys Thr Cys
435 440 445

Ser Lys Ser Ser Ile Asp Ile Glu Asn Gly Phe Ile Ser Glu Ser Gln
450 455 460

Tyr Thr Tyr Ala Leu Lys Glu Lys Ala Lys Tyr Gln Cys Lys Leu Gly
465 470 475 480

Tyr Val Thr Ala Asp Gly Glu Thr Ser Gly Ser Ile Arg Cys Gly Lys
485 490 495

Asp Gly Trp Ser Ala Gln Pro Thr Cys Ile Lys Ser Cys Asp Ile Pro
500 505 510

Val Phe Met Asn Ala Arg Thr Lys Asn Asp Phe Thr Trp Phe Lys Leu
515 520 525

Asn Asp Thr Leu Asp Tyr Glu Cys His Asp Gly Tyr Glu Ser Asn Thr
530 535 540

Gly Ser Thr Thr Gly Ser Ile Val Cys Gly Tyr Asn Gly Trp Ser Asp
545 550 555 560

Leu Pro Ile Cys Tyr Glu Arg Glu Cys Glu Leu Pro Lys Ile Asp Val
565 570 575

His Leu Val Pro Asp Arg Lys Lys Asp Gln Tyr Lys Val Gly Glu Val
580 585 590

Leu Lys Phe Ser Cys Lys Pro Gly Phe Thr Ile Val Gly Pro Asn Ser
595 600 605

Val Gln Cys Tyr His Phe Gly Leu Ser Pro Asp Leu Pro Ile Cys Lys
610 615 620

Glu Gln Val Gln Ser Cys Gly Pro Pro Pro Glu Leu Leu Asn Gly Asn

625 630 635 640

Val Lys Glu Lys Thr Lys Glu Glu Tyr Gly His Ser Glu Val Val Glu

645 650 655

Tyr Tyr Cys Asn Pro Arg Phe Leu Met Lys Gly Pro Asn Lys Ile Gln

660 665 670

Cys Val Asp Gly Glu Trp Thr Thr Leu Pro Val Cys Ile Val Glu Glu

675 680 685

Ser Thr Cys Gly Asp Ile Pro Glu Leu Glu His Gly Trp Ala Gln Leu

690 695 700

Ser Ser Pro Pro Tyr Tyr Tyr Gly Asp Ser Val Glu Phe Asn Cys Ser

705 710 715 720

Glu Ser Phe Thr Met Ile Gly His Arg Ser Ile Thr Cys Ile His Gly

725 730 735

Val Trp Thr Gln Leu Pro Gln Cys Val Ala Ile Asp Lys Leu Lys Lys

740 745 750

Cys Lys Ser Ser Asn Leu Ile Ile Leu Glu Glu His Leu Lys Asn Lys

755 760 765

Lys Glu Phe Asp His Asn Ser Asn Ile Arg Tyr Arg Cys Arg Gly Lys

770 775 780

Glu Gly Trp Ile His Thr Val Cys Ile Asn Gly Arg Trp Asp Pro Glu

785 790 795 800

Val Asn Cys Ser Met Ala Gln Ile Gln Leu Cys Pro Pro Pro Pro Gln

805 810 815

Ile Pro Asn Ser His Asn Met Thr Thr Thr Leu Asn Tyr Arg Asp Gly
820 825 830

Glu Lys Val Ser Val Leu Cys Gln Glu Asn Tyr Leu Ile Gln Glu Gly
835 840 845

Glu Glu Ile Thr Cys Lys Asp Gly Arg Trp Gln Ser Ile Pro Leu Cys
850 855 860

Val Glu Lys Ile Pro Cys Ser Gln Pro Pro Gln Ile Glu His Gly Thr
865 870 875 880

Ile Asn Ser Ser Arg Ser Ser Gln Glu Ser Tyr Ala His Gly Thr Lys
885 890 895

Leu Ser Tyr Thr Cys Glu Gly Gly Phe Arg Ile Ser Glu Glu Asn Glu
900 905 910

Thr Thr Cys Tyr Met Gly Lys Trp Ser Ser Pro Pro Gln Cys Glu Gly
915 920 925

Leu Pro Cys Lys Ser Pro Pro Glu Ile Ser His Gly Val Val Ala His
930 935 940

Met Ser Asp Ser Tyr Gln Tyr Gly Glu Glu Val Thr Tyr Lys Cys Phe
945 950 955 960

Glu Gly Phe Gly Ile Asp Gly Pro Ala Ile Ala Lys Cys Leu Gly Glu
965 970 975

Lys Trp Ser His Pro Pro Ser Cys Ile Lys Thr Asp Cys Leu Ser Leu
980 985 990

Pro Ser Phe Glu Asn Ala Ile Pro Met Gly Glu Lys Lys Asp Val Tyr
995 1000 1005

Lys Ala Gly Glu Gln Val Thr Tyr Thr Cys Ala Thr Tyr Tyr Lys

1010 1015 1020

Met Asp Gly Ala Ser Asn Val Thr Cys Ile Asn Ser Arg Trp Thr

1025 1030 1035

Gly Arg Pro Thr Cys Arg Asp Thr Ser Cys Val Asn Pro Pro Thr

1040 1045 1050

Val Gln Asn Ala Tyr Ile Val Ser Arg Gln Met Ser Lys Tyr Pro

1055 1060 1065

Ser Gly Glu Arg Val Arg Tyr Gln Cys Arg Ser Pro Tyr Glu Met

1070 1075 1080

Phe Gly Asp Glu Glu Val Met Cys Leu Asn Gly Asn Trp Thr Glu

1085 1090 1095

Pro Pro Gln Cys Lys Asp Ser Thr Gly Lys Cys Gly Pro Pro Pro

1100 1105 1110

Pro Ile Asp Asn Gly Asp Ile Thr Ser Phe Pro Leu Ser Val Tyr

1115 1120 1125

Ala Pro Ala Ser Ser Val Glu Tyr Gln Cys Gln Asn Leu Tyr Gln

1130 1135 1140

Leu Glu Gly Asn Lys Arg Ile Thr Cys Arg Asn Gly Gln Trp Ser

1145 1150 1155

Glu Pro Pro Lys Cys Leu His Pro Cys Val Ile Ser Arg Glu Ile

1160 1165 1170

Met Glu Asn Tyr Asn Ile Ala Leu Arg Trp Thr Ala Lys Gln Lys

1175 1180 1185

Leu Tyr Ser Arg Thr Gly Glu Ser Val Glu Phe Val Cys Lys Arg
1190 1195 1200

Gly Tyr Arg Leu Ser Ser Arg Ser His Thr Leu Arg Thr Thr Cys
1205 1210 1215

Trp Asp Gly Lys Leu Glu Tyr Pro Thr Cys Ala Lys Arg
1220 1225 1230

<210> 21

<211> 1984

<212> DNA

<213> Homo sapiens

<220>

<221> Superoxide dismutase 3 mRNA

<222> (1)..(1984)

<400> 21

ggatccagag atttagattt tttataagct ttcctgccac cgaaacgggt gtttgggacc 60

tcacgaggcc ctgttcattc ttcgtcgctg cgctccccac tctgtactgg atgcatttac 120

tgacgttgtt gtctccgtcc ccagagtatg aacccccaag gtgactcatg cagctgtggg 180

tgcccggcat acagcatggt gactggaatg gatgagcacc caataaacat ttgttgcagg 240

aatgcaggag gacgggcagg ccagcaagca ggctgcctgg tttttcccac atgggctttt 300

ctgggaaaga agagcttcta tttttggaaa gggctgctat gattgagaaa agttcatggc 360

agcaaaaaaa ggacagacgt cgggagggaa acactcctag ttctcccaga caacacattt 420

tttaaaaaga ctccttcatc tctttaataa taacggtaac gacaatgaca atgatgatta 480

```
cttatgagtg cggctagtgc cagccactgt gttgtcactg ggcgagtaat gatctcattg   540
gatcttcacg gtgggcgtgc ggggctccag ggacagcctg cgttcctggg ctggctgggt   600
gcagctctct tttcaggaga gaaagctctc ttggaggagc tggaaaggtg cccgactcca   660
gccatgctgg cgctactgtg ttcctgcctg ctcctggcag ccggtgcctc ggacgcctgg   720
acgggcgagg actcggcgga gcccaactct gactcggcgg agtggatccg agacatgtac   780
gccaaggtca cggagatctg gcaggaggtc atgcagcggc gggacgacga cggcacgctc   840
cacgccgcct gccaggtgca gccgtcggcc acgctggacg ccgcgcagcc ccgggtgacc   900
ggcgtcgtcc tcttccggca gcttgcgccc cgcgccaagc tcgacgcctt cttcgccctg   960
gagggcttcc cgaccgagcc gaacagctcc agccgcgcca tccacgtgca ccagttcggg  1020
gacctgagcc agggctgcga gtccaccggg ccccactaca acccgctggc cgtgccgcac  1080
ccgcagcacc cgggcgactt cggcaacttc gcggtccgcg acggcagcct ctggaggtac  1140
cgcgccggcc tggccgcctc gctcgcgggc ccgcactcca tcgtgggccg ggccgtggtc  1200
gtccacgctg gcgaggacga cctgggccgc ggcggcaacc aggccagcgt ggagaacggg  1260
aacgcgggcc ggcggctggc ctgctgcgtg gtgggcgtgt gcgggcccgg gctctgggag  1320
cgccaggcgc gggagcactc agagcgcaag aagcggcggc gcgagagcga gtgcaaggcc  1380
gcctgagcgc ggcccccacc cggcggcggc cagggacccc cgaggccccc ctctgccttt  1440
gagcttctcc tctgctccaa cagacacctt ccactctgag gtctcacctt cgcctctgct  1500
gaagtctccc cgcagccctc tccacccaga ggtctcccta taccgagacc caccatcctt  1560
ccatcctgag gaccgcccca accctcggag ccccccactc agtaggtctg aaggcctcca  1620
tttgtaccga aacaccccgc tcacgctgac agcctcctag gctccctgag gtacctttcc  1680
acccagaccc tccttcccca ccccataagc cctgagactc ccgcctttga cctgacgatc  1740
ttccccttc ccgccttcag gttcctccta ggcgctcaga ggccgctctg gggggttgcc  1800
tcgagtcccc ccacccctcc ccacccacca ccgctcccgc ggcaagccag cccgtgcaac  1860
ggaagccagg ccaactgccc cgcgtcttca gctgtttcgc atccaccgcc accccactga  1920
```

gagctgctcc tttgggggaa tgtttggcaa cctttgtgtt acagattaaa aattcagcaa 1980

ttca 1984

<210> 22

<211> 240

<212> PRT

<213> Homo sapiens

<220>

<221> Superoxide dismutase 3 polypeptide sequence

<222> (1)..(240)

<400> 22

Met Leu Ala Leu Leu Cys Ser Cys Leu Leu Leu Ala Ala Gly Ala Ser
1               5                   10                  15

Asp Ala Trp Thr Gly Glu Asp Ser Ala Glu Pro Asn Ser Asp Ser Ala
            20                  25                  30

Glu Trp Ile Arg Asp Met Tyr Ala Lys Val Thr Glu Ile Trp Gln Glu
        35                  40                  45

Val Met Gln Arg Arg Asp Asp Asp Gly Thr Leu His Ala Ala Cys Gln
    50                  55                  60

Val Gln Pro Ser Ala Thr Leu Asp Ala Ala Gln Pro Arg Val Thr Gly
65                  70                  75                  80

Val Val Leu Phe Arg Gln Leu Ala Pro Arg Ala Lys Leu Asp Ala Phe

85 90 95

Phe Ala Leu Glu Gly Phe Pro Thr Glu Pro Asn Ser Ser Ser Arg Ala

100 105 110

Ile His Val His Gln Phe Gly Asp Leu Ser Gln Gly Cys Glu Ser Thr

115 120 125

Gly Pro His Tyr Asn Pro Leu Ala Val Pro His Pro Gln His Pro Gly

130 135 140

Asp Phe Gly Asn Phe Ala Val Arg Asp Gly Ser Leu Trp Arg Tyr Arg

145 150 155 160

Ala Gly Leu Ala Ala Ser Leu Ala Gly Pro His Ser Ile Val Gly Arg

165 170 175

Ala Val Val Val His Ala Gly Glu Asp Asp Leu Gly Arg Gly Gly Asn

180 185 190

Gln Ala Ser Val Glu Asn Gly Asn Ala Gly Arg Arg Leu Ala Cys Cys

195 200 205

Val Val Gly Val Cys Gly Pro Gly Leu Trp Glu Arg Gln Ala Arg Glu

210 215 220

His Ser Glu Arg Lys Lys Arg Arg Arg Glu Ser Glu Cys Lys Ala Ala

225 230 235 240

<210> 23

<211> 1338

<212> DNA

<213> Homo sapiens

<220>

<221> neuronatin mRNA

<222> (1)..(1338)

<400> 23

```
taggtggcgg gcgggtactt aaggcgcggc caccgcggct gcggcagtgc gcccaacagc    60
ggactccgag accagcggat ctcggcaaac cctctttctc gaccacccac ctaccattct   120
tggaaccatg gcggcagtgg cggcggcctc ggctgaactg ctcatcatcg gctggtacat   180
cttccgcgtg ctgctgcagg tgttcctgga atgctgcatt tactgggtag gattcgcttt   240
tcgaaatcct ccagggacac agcccattgc gagaagtgag gtgttcaggt actccctgca   300
gaagctggca tacacggtgt cgcggaccgg gcggcaggtg ttgggggagc gcaggcagcg   360
agcccccaac tgaggcccca gctcccagcc ctgggcggcc gtatcatcag gtgctcctgt   420
gcatctcggc cagcacggga gccagtgccg cgcaggaatg tggggtcccc tgtgttccct   480
cgccagagga gcacttggca aggtcagtga ggggccagta gacccccgga gaagcagtac   540
cgacaatgac gaagatacca gatcccttcc caaccccttt gcaccggtcc cactaagggg   600
cagggtcgag agaggagggg ggataggggg agcagacccc tgagatctgg gcataggcac   660
cgcattctga tctggacaaa gtcgggacag caccatccca gccccgaagc cagggccatg   720
ccagcaggcc ccaccatgga aatcaaaaca ccgcaccagc cagcagaatg gacattctga   780
catcgccagc cgacgccctg aatcttggtg cagcaccaac cgcgtgcctg tgtggcggga   840
ctggagggca cagttgagga aggagggtgg ttaagaaata cagtggggcc ctctcgctgt   900
cccttgccca gggcacttgc attccagcct cgctgcattt gctctctcga ttcccctttc   960
ctcctcactg cctcccaagc ccaccctact ccaaaataat gtgtcacttg atttggaact  1020
attcaagcag taaaagtaaa tgaatcccac ctttactaaa acactttctc tgaacccccc  1080
ttgcccctca ctgatcttgc ttttccctgg tctcatgcag ttgtggtcaa tattgtggta  1140
```

atcgctaatt gtactgattg tttaagtgtg cattagttgt gtctccccag ctagattgta 1200

agctcctgga ggacagggac cacctctaca aaaaataaaa aaagtacctc ccctgtctcg 1260

cacagtgtcc caggaccctg cggtgcagta gaggcgcacc aaaaaaaaaa aaaaaaaaaa 1320

aaaaaaaaaa aaaaaaaa 1338

<210> 24

<211> 81

<212> PRT

<213> Homo sapiens

<220>

<221> neuronatin polypeptide sequence

<222> (1)..(81)

<400> 24

Met Ala Ala Val Ala Ala Ala Ser Ala Glu Leu Leu Ile Ile Gly Trp
1 5 10 15

Tyr Ile Phe Arg Val Leu Leu Gln Val Phe Leu Glu Cys Cys Ile Tyr
20 25 30

Trp Val Gly Phe Ala Phe Arg Asn Pro Pro Gly Thr Gln Pro Ile Ala
35 40 45

Arg Ser Glu Val Phe Arg Tyr Ser Leu Gln Lys Leu Ala Tyr Thr Val
50 55 60

Ser Arg Thr Gly Arg Gln Val Leu Gly Glu Arg Arg Gln Arg Ala Pro

65 70 75 80

Asn

<210> 25

<211> 2500

<212> DNA

<213> Homo sapiens

<220>

<221> follistatin-like 3 glycoprotein mRNA

<222> (1)..(2500)

<400> 25

gttcgccatg cgtcccgggg cgccagggcc actctggcct ctgccctggg gggccctggc 60

ttgggccgtg ggcttcgtga gctccatggg ctcggggaac cccgcgcccg gtggtgtttg 120

ctggctccag cagggccagg aggccacctg cagcctggtg ctccagactg atgtcacccg 180

ggccgagtgc tgtgcctccg gcaacattga caccgcctgg tccaacctca cccacccggg 240

gaacaagatc aacctcctcg gcttcttggg ccttgtccac tgccttccct gcaaagattc 300

gtgcgacggc gtggagtgcg gcccgggcaa ggcgtgccgc atgctggggg gccgcccgcg 360

ctgcgagtgc gcgcccgact gctcggggct cccggcgcgg ctgcaggtct gcggctcaga 420

cggcgccacc taccgcgacg agtgcgagct gcgcgccgcg cgctgccgcg gccacccgga 480

cctgagcgtc atgtaccggg gccgctgccg caagtcctgt gagcacgtgg tgtgcccgcg 540

gccacagtcg tgcgtcgtgg accagacggg cagcgcccac tgcgtggtgt gtcgagcggc 600

gccctgccct gtgccctcca gccccggcca ggagctttgc ggcaacaaca acgtcaccta 660

catctcctcg tgccacatgc gccaggccac ctgcttcctg ggccgctcca tcggcgtgcg 720

ccacgcgggc agctgcgcag gcacccctga ggagccgcca ggtggtgagt ctgcagaaga 780

ggaagagaac ttcgtgtgag cctgcaggac aggcctgggc ctggtgcccg aggcccccca 840

tcatcccctg ttatttattg ccacagcaga gtctaattta tatgccacgg acactcctta 900

gagcccggat tcggaccact tggggatccc agaacctccc tgacgatatc ctggaaggac 960

tgaggaaggg aggcctgggg gccggctggt gggtgggata gacctgcgtt ccggacactg 1020

agcgcctgat ttagggccct tctctaggat gccccagccc ctaccctaag acctattgcc 1080

ggggaggatt ccacacttcc gctcctttgg ggataaacct attaattatt gctactatca 1140

agagggctgg gcattctctg ctggtaattc ctgaagaggc atgactgctt ttctcagccc 1200

caagcctcta gtctgggtgt gtacggaggg tctagcctgg gtgtgtacgg agggtctagc 1260

ctgggtgagt acggagggtc tagcctgggt gagtacggag ggtctagcct gggtgagtac 1320

ggagagtcta gcctgggtgt gtatggagga tctagcctgg gtgagtatgg agggtctagc 1380

ctgggtgagt atggagggtc tagcctgggt gtgtatggag ggtctagcct gggtgagtat 1440

ggagggtcta gcctgggtgt gtatggaggg tctagcctgg gtgagtatgg agggtctagc 1500

ctgggtgtgt acggagggtc tagtctgagt gcgtgtgggg acctcagaac actgtgacct 1560

tagcccagca agccaggccc ttcatgaagg ccaagaaggc tgccaccatt ccctgccagc 1620

ccaagaactc cagcttcccc actgcctctg tgtgcccctt tgcgtcctgt gaaggccatt 1680

gagaaatgcc cagtgtgccc cctgggaaag ggcacggcct gtgctcctga cacgggctgt 1740

gcttggccac agaaccaccc agcgtctccc ctgctgctgt ccacgtcagt tcatgaggca 1800

acgtcgcgtg gtctcagacg tggagcagcc agcggcagct cagagcaggg cactgtgtcc 1860

ggcggagcca agtccactct gggggagctc tggcggggac cacgggccac tgctcaccca 1920

ctggccccga ggggggtgta gacgccaaga ctcacgcatg tgtgacatcc ggagtcctgg 1980

agccgggtgt cccagtggca ccactaggtg cctgctgcct ccacagtggg gttcacaccc 2040

agggctcctt ggtcccccac aacctgcccc ggccaggcct gcagacccag actccagcca 2100

gacctgcctc acccaccaat gcagccgggg ctggcgacac cagccaggtg ctggtcttgg 2160

gccagttctc ccacgacggc tcaccctccc ctccatctgc gttgatgctc agaatcgcct 2220

acctgtgcct gcgtgtaaac cacagcctca gaccagctat ggggagagga caacacggag 2280

gatatccagc ttccccggtc tggggtgagg agtgtgggga gcttgggcat cctcctccag 2340

cctcctccag cccccaggca gtgccttacc tgtggtgccc agaaaagtgc ccctaggttg 2400

gtgggtctac aggagcctca gccaggcagc ccaccccacc ctggggccct gcctcaccaa 2460

ggaaataaag actcaaagaa gcctttttt tttttttttt 2500

<210> 26

<211> 263

<212> PRT

<213> Homo sapiens

<220>

<221> follistatin-like 3 glycoprotein polypeptide sequence

<222> (1)..(263)

<400> 26

Met Arg Pro Gly Ala Pro Gly Pro Leu Trp Pro Leu Pro Trp Gly Ala
1 5 10 15

Leu Ala Trp Ala Val Gly Phe Val Ser Ser Met Gly Ser Gly Asn Pro
20 25 30

Ala Pro Gly Gly Val Cys Trp Leu Gln Gln Gly Gln Glu Ala Thr Cys
35 40 45

Ser Leu Val Leu Gln Thr Asp Val Thr Arg Ala Glu Cys Cys Ala Ser

50 55 60

Gly Asn Ile Asp Thr Ala Trp Ser Asn Leu Thr His Pro Gly Asn Lys

65 70 75 80

Ile Asn Leu Leu Gly Phe Leu Gly Leu Val His Cys Leu Pro Cys Lys

85 90 95

Asp Ser Cys Asp Gly Val Glu Cys Gly Pro Gly Lys Ala Cys Arg Met

100 105 110

Leu Gly Gly Arg Pro Arg Cys Glu Cys Ala Pro Asp Cys Ser Gly Leu

115 120 125

Pro Ala Arg Leu Gln Val Cys Gly Ser Asp Gly Ala Thr Tyr Arg Asp

130 135 140

Glu Cys Glu Leu Arg Ala Ala Arg Cys Arg Gly His Pro Asp Leu Ser

145 150 155 160

Val Met Tyr Arg Gly Arg Cys Arg Lys Ser Cys Glu His Val Val Cys

165 170 175

Pro Arg Pro Gln Ser Cys Val Val Asp Gln Thr Gly Ser Ala His Cys

180 185 190

Val Val Cys Arg Ala Ala Pro Cys Pro Val Pro Ser Ser Pro Gly Gln

195 200 205

Glu Leu Cys Gly Asn Asn Asn Val Thr Tyr Ile Ser Ser Cys His Met

210 215 220

Arg Gln Ala Thr Cys Phe Leu Gly Arg Ser Ile Gly Val Arg His Ala

225 230 235 240

Gly Ser Cys Ala Gly Thr Pro Glu Glu Pro Pro Gly Gly Glu Ser Ala

245 250 255

Glu Glu Glu Glu Asn Phe Val

260

<210> 27

<211> 3750

<212> DNA

<213> Homo sapiens

<220>

<221> serine 23 protease mRNA

<222> (1)..(3750)

<400> 27

```
gcgctgctcg ccagcttgct cgcactcggc tgtgcggcgg ggcaggcatg ggagccgcgc    60
gctctctccc ggcgcccaca cctgtctgag cggcgcagcg agccgcggcc cgggcgggct   120
gctcggcgcg gaacagtgct cggcatggca gggattccag ggctcctctt ccttctcttc   180
tttctgctct gtgctgttgg gcaagtgagc ccttacagtg ccccctggaa acccacttgg   240
cctgcatacc gcctccctgt cgtcttgccc cagtctaccc tcaatttagc caagccagac   300
tttggagccg aagccaaatt agaagtatct tcttcatgtg gaccccagtg tcataaggga   360
actccactgc ccacttacga agaggccaag caatatctgt cttatgaaac gctctatgcc   420
aatggcagcc gcacagagac gcaggtgggc atctacatcc tcagcagtag tggagatggg   480
gcccaacacc gagactcagg gtcttcagga aagtctcgaa ggaagcggca gatttatggc   540
tatgacagca ggttcagcat ttttgggaag gacttcctgc tcaactaccc tttctcaaca   600
tcagtgaagt tatccacggg ctgcaccggc accctggtgg cagagaagca tgtcctcaca   660
gctgcccact gcatacacga tggaaaaacc tatgtgaaag gaacccagaa gcttcgagtg   720
```

ggcttcctaa agcccaagtt taaagatggt ggtcgagggg ccaacgactc cacttcagcc 780

atgcccgagc agatgaaatt tcagtggatc cgggtgaaac gcacccatgt gcccaagggt 840

tggatcaagg gcaatgccaa tgacatcggc atggattatg attatgccct cctggaactc 900

aaaaagcccc acaagagaaa atttatgaag attggggtga gccctcctgc taagcagctg 960

ccaggggggca gaattcactt ctctggttat gacaatgacc gaccaggcaa tttggtgtat 1020

cgcttctgtg acgtcaaaga cgagacctat gacttgctct accagcaatg cgatgcccag 1080

ccaggggcca gcgggtctgg ggtctatgtg aggatgtgga agagacagca gcagaagtgg 1140

gagcgaaaaa ttattggcat tttttcaggg caccagtggg tggacatgaa tggttcccca 1200

caggatttca acgtggctgt cagaatcact cctctcaaat atgcccagat ttgctattgg 1260

attaaaggaa actacctgga ttgtagggag gggtgacaca gtgttccctc ctggcagcaa 1320

ttaagggtct tcatgttctt attttaggag aggccaaatt gttttttgtc attggcgtgc 1380

acacgtgtgt gtgtgtgtgt gtgtgtgtgt aaggtgtctt ataatctttt acctatttct 1440

tacaattgca agatgactgg ctttactatt tgaaaactgg tttgtgtatc atatcatata 1500

tcatttaagc agtttgaagg catacttttg catagaaata aaaaaaatac tgatttgggg 1560

caatgaggaa tatttgacaa ttaagttaat cttcacgttt ttgcaaactt tgatttttat 1620

ttcatctgaa cttgtttcaa agatttatat taaatatttg gcatacaaga gatatgaatt 1680

cttatatgtg tgcatgtgtg ttttcttctg agattcatct tggtggtggg tttttttgtt 1740

tttttaattc agtgcctgat ctttaatgct tccataaggc agtgttccca tttaggaact 1800

ttgacagcat ttgttaggca gaatattttg gatttggagg catttgcatg gtagtctttg 1860

aacagtaaaa tgatgtgttg actatactga tacacatatt aaactatacc ttatagtaaa 1920

ccagtatccc aagctgcttt tagttccaaa aatagtttct tttccaaagg ttgttgctct 1980

actttgtagg aagtctttgc atatggccct cccaacttta aagtcatacc agagtggcca 2040

agagtgttta tcccaaccct tccatttaac aggatttcac tcacatttct ggaactagct 2100

atttttcaga agacaataat cagggcttaa ttagaacagg ctgtatttcc tcccagcaaa 2160

cagttgtggc cacactaaaa acaatcatag cattttaccc ctggattata gcacatctca 2220

tgttttatca tttggatgga gtaatttaaa atgaattaaa ttccagagaa caatggaagc 2280

attgcctggc agatgtcaca acagaataac cacttgtttg gagcctggca cagtcctcca 2340

gcctgatcaa aaattattct gcatagtttt cagtgtgctt tctgggagct atgtacttct 2400

tcaatttgga aacttttctc tctcatttat agtgaaaata cttggaagtt actttaagaa 2460

aaccagtgtg gcctttttcc ctctagcttt aaaagggccg cttttgctgg aatgctctag 2520

gttatagata aacaattagg tataatagca aaaatgaaaa ttggaagaat gcaaaatgga 2580

tcagaatcat gccttccaat aaaggccttt acacatgttt tatcaatatg attatcaaat 2640

cacagcatat acagaaaaga cttggactta ttgtatgttt ttattttatg gctctcggcc 2700

taagcacttc tttctaaatg tatcggagaa aaaatcaaat ggactacaag cacgtgtttg 2760

ctgtgcttgc accccaggta aacctgcatt gtagcaattt gtaaggatat tcagatggag 2820

cactgtcact tagacattct ctggggggatt ttctgcttgt ctttcttgag ctttttggaa 2880

ggataattct gataaggcac tcaagaaacg tacaaccaca gtgctttctt caaatcatat 2940

gagaaatact atgcatagca aggagatgca gagccgccag gaaaattctg agttccagca 3000

caattttctt tggaatctaa caggaatcta gcctgaggaa gaagggaggt ctccatttct 3060

atgtctggta tttgggggtt ttgtttgttt ttgctttagc ttggtgaaaa aaagttcact 3120

gaacaccaag accagaatgg atttttttaa aaaaatagat gttccttttg tgaagcacct 3180

tgattccttg attttgattt tttgcaaagt tagacaatgg cacaaagtca aaatgaaatc 3240

aatgtttagt tcacaagtag atgtaattta ctaaagaatg atacacccat atgctatata 3300

cagcttaact cacagaactg taaaagaaaa ttataaaata attcaacatg tccatctttt 3360

tagtgataat aaaagaaagc atggtattaa actatcatag aagtagacag aaaaagaaaa 3420

aaggactcat ggcattatta atataattag tgctttacat gtgttagtta tacatattag 3480

aagcatattt gcctagtaag gctagtagaa ccacatttcc caaagtgtgc tccttaaaca 3540

ctcatgcctt atgattttct accaaaagta aaaagggttg tattaagtca gaggaagatg 3600

cctctccatt ttccctctct ttatcagagg ttcacatgcc tgtctgcaca ttaaaagctc 3660

tgggaagacc tgttgtaaag ggacaagttg aggttgtaaa atctgcattt aaataaacat 3720

ctttgatcac aaaaaaaaaa aaaaaaaaaa 3750

<210> 28

<211> 383

<212> PRT

<213> Homo sapiens

<220>

<221> serine 23 protease polypeptide sequence

<222> (1)..(383)

<400> 28

Met Ala Gly Ile Pro Gly Leu Leu Phe Leu Leu Phe Phe Leu Leu Cys

1 5 10 15

Ala Val Gly Gln Val Ser Pro Tyr Ser Ala Pro Trp Lys Pro Thr Trp

20 25 30

Pro Ala Tyr Arg Leu Pro Val Val Leu Pro Gln Ser Thr Leu Asn Leu

35 40 45

Ala Lys Pro Asp Phe Gly Ala Glu Ala Lys Leu Glu Val Ser Ser Ser

50 55 60

Cys Gly Pro Gln Cys His Lys Gly Thr Pro Leu Pro Thr Tyr Glu Glu

65 70 75 80

Ala Lys Gln Tyr Leu Ser Tyr Glu Thr Leu Tyr Ala Asn Gly Ser Arg

85 90 95

Thr Glu Thr Gln Val Gly Ile Tyr Ile Leu Ser Ser Ser Gly Asp Gly

100 105 110

Ala Gln His Arg Asp Ser Gly Ser Ser Gly Lys Ser Arg Arg Lys Arg

115 120 125

Gln Ile Tyr Gly Tyr Asp Ser Arg Phe Ser Ile Phe Gly Lys Asp Phe

130 135 140

Leu Leu Asn Tyr Pro Phe Ser Thr Ser Val Lys Leu Ser Thr Gly Cys

145 150 155 160

Thr Gly Thr Leu Val Ala Glu Lys His Val Leu Thr Ala Ala His Cys

165 170 175

Ile His Asp Gly Lys Thr Tyr Val Lys Gly Thr Gln Lys Leu Arg Val

180 185 190

Gly Phe Leu Lys Pro Lys Phe Lys Asp Gly Gly Arg Gly Ala Asn Asp

195 200 205

Ser Thr Ser Ala Met Pro Glu Gln Met Lys Phe Gln Trp Ile Arg Val

210 215 220

Lys Arg Thr His Val Pro Lys Gly Trp Ile Lys Gly Asn Ala Asn Asp

225 230 235 240

Ile Gly Met Asp Tyr Asp Tyr Ala Leu Leu Glu Leu Lys Lys Pro His

245 250 255

Lys Arg Lys Phe Met Lys Ile Gly Val Ser Pro Pro Ala Lys Gln Leu

260 265 270

Pro Gly Gly Arg Ile His Phe Ser Gly Tyr Asp Asn Asp Arg Pro Gly

275 280 285

Asn Leu Val Tyr Arg Phe Cys Asp Val Lys Asp Glu Thr Tyr Asp Leu

290 295 300

Leu Tyr Gln Gln Cys Asp Ala Gln Pro Gly Ala Ser Gly Ser Gly Val

305 310 315 320

Tyr Val Arg Met Trp Lys Arg Gln Gln Gln Lys Trp Glu Arg Lys Ile

325 330 335

Ile Gly Ile Phe Ser Gly His Gln Trp Val Asp Met Asn Gly Ser Pro

340 345 350

Gln Asp Phe Asn Val Ala Val Arg Ile Thr Pro Leu Lys Tyr Ala Gln

355 360 365

Ile Cys Tyr Trp Ile Lys Gly Asn Tyr Leu Asp Cys Arg Glu Gly

370 375 380

<210> 29

<211> 1362

<212> DNA

<213> Homo sapiens

<220>

<221> annexin 2 mRNA

<222> (1)..(1362)

<223> synonym: lipocortin II

<400> 29

catttgggga cgctctcagc tctcggcgca cggcccagct tccttcaaaa tgtctactgt 60

tcacgaaatc ctgtgcaagc tcagcttgga gggtgatcac tctacacccc caagtgcata 120

tgggtctgtc aaagcctata ctaactttga tgctgagcgg gatgctttga acattgaaac 180

agccatcaag accaaaggtg tggatgaggt caccattgtc aacattttga ccaaccgcag 240

caatgcacag agacaggata ttgccttcgc ctaccagaga aggaccaaaa aggaacttgc 300

atcagcactg aagtcagcct tatctggcca cctggagacg gtgattttgg gcctattgaa 360

gacacctgct cagtatgacg cttctgagct aaaagcttcc atgaaggggc tgggaaccga 420

cgaggactct ctcattgaga tcatctgctc cagaaccaac caggagctgc aggaaattaa 480

cagagtctac aaggaaatgt acaagactga tctggagaag gacattattt cggacacatc 540

tggtgacttc cgcaagctga tggttgccct ggcaaagggt agaagagcag aggatggctc 600

tgtcattgat tatgaactga ttgaccaaga tgctcgggat ctctatgacg ctggagtgaa 660

gaggaaagga actgatgttc ccaagtggat cagcatcatg accgagcgga gcgtgcccca 720

cctccagaaa gtatttgata ggtacaagag ttacagccct tatgacatgt tggaaagcat 780

caggaaagag gttaaaggag acctggaaaa tgctttcctg aacctggttc agtgcattca 840

gaacaagccc ctgtattttg ctgatcggct gtatgactcc atgaagggca aggggacgcg 900

agataaggtc ctgatcagaa tcatggtctc ccgcagtgaa gtggacatgt tgaaaattag 960

gtctgaattc aagagaaagt acggcaagtc cctgtactat tatatccagc aagacactaa 1020

gggcgactac cagaaagcgc tgctgtacct gtgtggtgga gatgactgaa gcccgacacg 1080

gcctgagcgt ccagaaatgg tgctcaccat gcttccagct aacaggtcta gaaaaccagc 1140

ttgcgaataa cagtccccgt ggccatccct gtgagggtga cgttagcatt acccccaacc 1200

tcattttagt tgcctaagca ttgcctggcc ttcctgtcta gtctctcctg taagccaaag 1260

aaatgaacat tccaaggagt tggaagtgaa gtctatgatg tgaaacactt tgcctcctgt 1320

gtactgtgtc ataaacagat gaataaactg aatttgtact tt 1362

<210> 30

<211> 339

<212> PRT

<213> Homo sapiens

<220>

<221> Annexin 2 polypeptide sequence

<222> (1)..(339)

<400> 30

Met Ser Thr Val His Glu Ile Leu Cys Lys Leu Ser Leu Glu Gly Asp
1 5 10 15

His Ser Thr Pro Pro Ser Ala Tyr Gly Ser Val Lys Ala Tyr Thr Asn
20 25 30

Phe Asp Ala Glu Arg Asp Ala Leu Asn Ile Glu Thr Ala Ile Lys Thr
35 40 45

Lys Gly Val Asp Glu Val Thr Ile Val Asn Ile Leu Thr Asn Arg Ser
50 55 60

Asn Ala Gln Arg Gln Asp Ile Ala Phe Ala Tyr Gln Arg Arg Thr Lys
65 70 75 80

Lys Glu Leu Ala Ser Ala Leu Lys Ser Ala Leu Ser Gly His Leu Glu
85 90 95

Thr Val Ile Leu Gly Leu Leu Lys Thr Pro Ala Gln Tyr Asp Ala Ser

100 105 110

Glu Leu Lys Ala Ser Met Lys Gly Leu Gly Thr Asp Glu Asp Ser Leu

115 120 125

Ile Glu Ile Ile Cys Ser Arg Thr Asn Gln Glu Leu Gln Glu Ile Asn

130 135 140

Arg Val Tyr Lys Glu Met Tyr Lys Thr Asp Leu Glu Lys Asp Ile Ile

145 150 155 160

Ser Asp Thr Ser Gly Asp Phe Arg Lys Leu Met Val Ala Leu Ala Lys

165 170 175

Gly Arg Arg Ala Glu Asp Gly Ser Val Ile Asp Tyr Glu Leu Ile Asp

180 185 190

Gln Asp Ala Arg Asp Leu Tyr Asp Ala Gly Val Lys Arg Lys Gly Thr

195 200 205

Asp Val Pro Lys Trp Ile Ser Ile Met Thr Glu Arg Ser Val Pro His

210 215 220

Leu Gln Lys Val Phe Asp Arg Tyr Lys Ser Tyr Ser Pro Tyr Asp Met

225 230 235 240

Leu Glu Ser Ile Arg Lys Glu Val Lys Gly Asp Leu Glu Asn Ala Phe

245 250 255

Leu Asn Leu Val Gln Cys Ile Gln Asn Lys Pro Leu Tyr Phe Ala Asp

260 265 270

Arg Leu Tyr Asp Ser Met Lys Gly Lys Gly Thr Arg Asp Lys Val Leu

275 280 285

Ile Arg Ile Met Val Ser Arg Ser Glu Val Asp Met Leu Lys Ile Arg

290 295 300

Ser Glu Phe Lys Arg Lys Tyr Gly Lys Ser Leu Tyr Tyr Tyr Ile Gln

305 310 315 320

Gln Asp Thr Lys Gly Asp Tyr Gln Lys Ala Leu Leu Tyr Leu Cys Gly

325 330 335

Gly Asp Asp

<210> 31

<211> 1946

<212> DNA

<213> Homo sapiens

<220>

<221> LOX mRNA

<222> (1)..(1946)

<400> 31

agacactgcc cgctctccgg gactccgcgc cgctccccgt tgccttccag gactgagaaa 60

ggggaaaggg aagggtgcca cgtccgagca gccgccttga ctggggaagg gtctgaatcc 120

caccettggc attgcttggt ggagactgag atacccgtgc tccgctcgcc tccttggttg 180

aagatttctc cttccctcac gtgatttgag ccccgttttt attttctgtg agccacgtcc 240

tcctcgagcg gggtcaatct ggcaaaagga gtgatgcgct tcgcctggac cgtgctcctg 300

ctcgggcctt tgcagctctg cgcgctagtg cactgcgccc ctcccgccgc cggccaacag 360

cagcccccgc gcgagccgcc ggcggctccg ggcgcctggc gccagcagat ccaatgggag 420

aacaacgggc aggtgttcag cttgctgagc ctgggctcac agtaccagcc tcagcgccgc 480

cgggacccgg gcgccgccgt ccctggtgca gccaacgcct ccgcccagca gccccgcact 540

```
ccgatcctgc tgatccgcga caaccgcacc gccgcggcgc gaacgcggac ggccggctca    600
tctggagtca ccgctggccg ccccaggccc accgcccgtc actggttcca agctggctac    660
tcgacatcta gagcccgcga agctggcgcc tcgcgcgcgg agaaccagac agcgccggga    720
gaagttcctg cgctcagtaa cctgcggccg cccagccgcg tggacggcat ggtgggcgac    780
gacccttaca acccctacaa gtactctgac gacaaccctt attacaacta ctacgatact    840
tatgaaaggc ccagacctgg gggcaggtac cggcccggat acggcactgg ctacttccag    900
tacggtctcc cagacctggt ggccgacccc tactacatcc aggcgtccac gtacgtgcag    960
aagatgtcca tgtacaacct gagatgcgcg gcggaggaaa actgtctggc cagtacagca   1020
tacagggcag atgtcagaga ttatgatcac agggtgctgc tcagatttcc ccaaagagtg   1080
aaaaaccaag ggacatcaga tttcttaccc agccgaccaa gatattcctg ggaatggcac   1140
agttgtcatc aacattacca cagtatggat gagtttagcc actatgacct gcttgatgcc   1200
aacacccaga ggagagtggc tgaaggccac aaagcaagtt tctgtcttga agacacatcc   1260
tgtgactatg gctaccacag gcgatttgca tgtactgcac acacacaggg attgagtcct   1320
ggctgttatg atacctatgg tgcagacata gactgccagt ggattgatat tacagatgta   1380
aaacctggaa actatatcct aaaggtcagt gtaaacccca gctacctggt tcctgaatct   1440
gactatacca acaatgttgt gcgctgtgac attcgctaca caggacatca tgcgtatgcc   1500
tcaggctgca caatttcacc gtattagaag gcaaagcaaa actcccaatg gataaatcag   1560
tgcctggtgt tctgaagtgg gaaaaaatag actaacttca gtaggattta tgtattttga   1620
aaaagagaac agaaaacaac aaaagaattt ttgtttggac tgttttcaat aacaaagcac   1680
ataactggat tttgaacgct taagtcatca ttacttggga aatttttaat gtttattatt   1740
tacatcactt tgtgaattaa cacagtgttt caattctgta attacatatt tgactctttc   1800
aaagaaatcc aaatttctca tgttcctttt gaaattgtag tgcaaaatgg tcagtattat   1860
ctaaatgaat gagccaaaat gactttgaac tgaaactttt ctaaagtgct ggaactttag   1920
tgaaacataa taataatggg tttata                                         1946
```

<210> 32

<211> 417

<212> PRT

<213> Homo sapiens

<220>

<221> LOX polypeptide sequence

<222> (1)..(417)

<400> 32

Met Arg Phe Ala Trp Thr Val Leu Leu Leu Gly Pro Leu Gln Leu Cys
1 5 10 15

Ala Leu Val His Cys Ala Pro Pro Ala Ala Gly Gln Gln Gln Pro Pro
20 25 30

Arg Glu Pro Pro Ala Ala Pro Gly Ala Trp Arg Gln Gln Ile Gln Trp
35 40 45

Glu Asn Asn Gly Gln Val Phe Ser Leu Leu Ser Leu Gly Ser Gln Tyr
50 55 60

Gln Pro Gln Arg Arg Arg Asp Pro Gly Ala Ala Val Pro Gly Ala Ala
65 70 75 80

Asn Ala Ser Ala Gln Gln Pro Arg Thr Pro Ile Leu Leu Ile Arg Asp
85 90 95

Asn Arg Thr Ala Ala Ala Arg Thr Arg Thr Ala Gly Ser Ser Gly Val

100 105 110

Thr Ala Gly Arg Pro Arg Pro Thr Ala Arg His Trp Phe Gln Ala Gly

115 120 125

Tyr Ser Thr Ser Arg Ala Arg Glu Ala Gly Ala Ser Arg Ala Glu Asn

130 135 140

Gln Thr Ala Pro Gly Glu Val Pro Ala Leu Ser Asn Leu Arg Pro Pro

145 150 155 160

Ser Arg Val Asp Gly Met Val Gly Asp Asp Pro Tyr Asn Pro Tyr Lys

165 170 175

Tyr Ser Asp Asp Asn Pro Tyr Tyr Asn Tyr Tyr Asp Thr Tyr Glu Arg

180 185 190

Pro Arg Pro Gly Gly Arg Tyr Arg Pro Gly Tyr Gly Thr Gly Tyr Phe

195 200 205

Gln Tyr Gly Leu Pro Asp Leu Val Ala Asp Pro Tyr Tyr Ile Gln Ala

210 215 220

Ser Thr Tyr Val Gln Lys Met Ser Met Tyr Asn Leu Arg Cys Ala Ala

225 230 235 240

Glu Glu Asn Cys Leu Ala Ser Thr Ala Tyr Arg Ala Asp Val Arg Asp

245 250 255

Tyr Asp His Arg Val Leu Leu Arg Phe Pro Gln Arg Val Lys Asn Gln

260 265 270

Gly Thr Ser Asp Phe Leu Pro Ser Arg Pro Arg Tyr Ser Trp Glu Trp

275 280 285

His Ser Cys His Gln His Tyr His Ser Met Asp Glu Phe Ser His Tyr

290 295 300

Asp Leu Leu Asp Ala Asn Thr Gln Arg Arg Val Ala Glu Gly His Lys

305 310 315 320

Ala Ser Phe Cys Leu Glu Asp Thr Ser Cys Asp Tyr Gly Tyr His Arg

325 330 335

Arg Phe Ala Cys Thr Ala His Thr Gln Gly Leu Ser Pro Gly Cys Tyr

340 345 350

Asp Thr Tyr Gly Ala Asp Ile Asp Cys Gln Trp Ile Asp Ile Thr Asp

355 360 365

Val Lys Pro Gly Asn Tyr Ile Leu Lys Val Ser Val Asn Pro Ser Tyr

370 375 380

Leu Val Pro Glu Ser Asp Tyr Thr Asn Asn Val Val Arg Cys Asp Ile

385 390 395 400

Arg Tyr Thr Gly His His Ala Tyr Ala Ser Gly Cys Thr Ile Ser Pro

405 410 415

Tyr

<210> 33

<211> 1600

<212> DNA

<213> Homo sapiens

<220>

<221> ECGF 1 mRNA

<222> (1)..(1600)

<400> 33

gccccgccgc cggcagtgga ccgctgtgcg cgaaccctga accctacggt cccgacccgc 60

gggcgaggcc gggtacctgg gctgggatcc ggagcaagcg ggcgagggca gcgccctaag 120

caggcccgga gcgatggcag ccttgatgac cccgggaacc ggggccccac ccgcgcctgg 180

tgacttctcc ggggaaggga gccagggact tcccgaccct tcgccagagc ccaagcagct 240

cccggagctg atccgcatga agcgagacgg aggccgcctg agcgaagcgg acatcagggg 300

cttcgtggcc gctgtggtga atgggagcgc gcagggcgca cagatcgggg ccatgctgat 360

ggccatccga cttcggggca tggatctgga ggagacctcg gtgctgaccc aggccctggc 420

tcagtcggga cagcagctgg agtggccaga ggcctggcgc cagcagcttg tggacaagca 480

ttccacaggg ggtgtgggtg acaaggtcag cctggtcctc gcacctgccc tggcggcatg 540

tggctgcaag gtgccaatga tcagcggacg tggtctgggg cacacaggag gcaccttgga 600

taagctggag tctattcctg gattcaatgt catccagagc ccagagcaga tgcaagtgct 660

gctggaccag gcgggctgct gtatcgtggg tcagagtgag cagctggttc ctgcggacgg 720

aatcctatat gcagccagag atgtgacagc caccgtggac agcctgccac tcatcacagc 780

ctccattctc agtaagaaac tcgtggaggg gctgtccgct ctggtggtgg acgttaagtt 840

cggagggggc gccgtcttcc ccaaccagga gcaggcccgg gagctggcaa agacgctggt 900

tggcgtggga gccagcctag ggcttcgggt cgcggcagcg ctgaccgcca tggacaagcc 960

cctgggtcgc tgcgtgggcc acgccctgga ggtggaggag gcgctgctct gcatggacgg 1020

cgcaggcccg ccagacttaa gggacctggt caccacgctc gggggcgccc tgctctggct 1080

cagcggacac gcggggactc aggctcaggg cgctgcccgg gtggccgcgg cgctggacga 1140

cggctcggcc cttggccgct tcgagcggat gctggcggcg cagggcgtgg atcccggtct 1200

ggcccgagcc ctgtgctcgg gaagtcccgc agaacgccgg cagctgctgc ctcgcgcccg 1260

ggagcaggag gagctgctgg cgcccgcaga tggcaccgtg gagctggtcc gggcgctgcc 1320

gctggcgctg gtgctgcacg agctcggggc cgggcgcagc cgcgctgggg agccgctccg 1380

cctgggggtg ggcgcagagc tgctggtcga cgtgggtcag aggctgcgcc gtgggacccc 1440

ctggctccgc gtgcaccggg acggccccgc gctcagcggc ccgcagagcc gcgccctgca 1500

ggaggcgctc gtactctccg accgcgcgcc attcgccgcc ccctcgccct tcgcagagct 1560

cgttctgccg ccgcagcaat aaagctcctt tgccgcgaaa 1600

<210> 34

<211> 482

<212> PRT

<213> Homo sapiens

<220>

<221> ECGF1 polypeptide sequence

<222> (1)..(482)

<400> 34

Met Ala Ala Leu Met Thr Pro Gly Thr Gly Ala Pro Pro Ala Pro Gly
1 5 10 15

Asp Phe Ser Gly Glu Gly Ser Gln Gly Leu Pro Asp Pro Ser Pro Glu
20 25 30

Pro Lys Gln Leu Pro Glu Leu Ile Arg Met Lys Arg Asp Gly Gly Arg
35 40 45

Leu Ser Glu Ala Asp Ile Arg Gly Phe Val Ala Ala Val Val Asn Gly
50 55 60

Ser Ala Gln Gly Ala Gln Ile Gly Ala Met Leu Met Ala Ile Arg Leu
65 70 75 80

Arg Gly Met Asp Leu Glu Glu Thr Ser Val Leu Thr Gln Ala Leu Ala

85 90 95

Gln Ser Gly Gln Gln Leu Glu Trp Pro Glu Ala Trp Arg Gln Gln Leu

100 105 110

Val Asp Lys His Ser Thr Gly Gly Val Gly Asp Lys Val Ser Leu Val

115 120 125

Leu Ala Pro Ala Leu Ala Ala Cys Gly Cys Lys Val Pro Met Ile Ser

130 135 140

Gly Arg Gly Leu Gly His Thr Gly Gly Thr Leu Asp Lys Leu Glu Ser

145 150 155 160

Ile Pro Gly Phe Asn Val Ile Gln Ser Pro Glu Gln Met Gln Val Leu

165 170 175

Leu Asp Gln Ala Gly Cys Cys Ile Val Gly Gln Ser Glu Gln Leu Val

180 185 190

Pro Ala Asp Gly Ile Leu Tyr Ala Ala Arg Asp Val Thr Ala Thr Val

195 200 205

Asp Ser Leu Pro Leu Ile Thr Ala Ser Ile Leu Ser Lys Lys Leu Val

210 215 220

Glu Gly Leu Ser Ala Leu Val Val Asp Val Lys Phe Gly Gly Ala Ala

225 230 235 240

Val Phe Pro Asn Gln Glu Gln Ala Arg Glu Leu Ala Lys Thr Leu Val

245 250 255

Gly Val Gly Ala Ser Leu Gly Leu Arg Val Ala Ala Ala Leu Thr Ala

260 265 270

Met Asp Lys Pro Leu Gly Arg Cys Val Gly His Ala Leu Glu Val Glu

275 280 285

Glu Ala Leu Leu Cys Met Asp Gly Ala Gly Pro Pro Asp Leu Arg Asp

290 295 300

Leu Val Thr Thr Leu Gly Gly Ala Leu Leu Trp Leu Ser Gly His Ala

305 310 315 320

Gly Thr Gln Ala Gln Gly Ala Ala Arg Val Ala Ala Ala Leu Asp Asp

325 330 335

Gly Ser Ala Leu Gly Arg Phe Glu Arg Met Leu Ala Ala Gln Gly Val

340 345 350

Asp Pro Gly Leu Ala Arg Ala Leu Cys Ser Gly Ser Pro Ala Glu Arg

355 360 365

Arg Gln Leu Leu Pro Arg Ala Arg Glu Gln Glu Glu Leu Leu Ala Pro

370 375 380

Ala Asp Gly Thr Val Glu Leu Val Arg Ala Leu Pro Leu Ala Leu Val

385 390 395 400

Leu His Glu Leu Gly Ala Gly Arg Ser Arg Ala Gly Glu Pro Leu Arg

405 410 415

Leu Gly Val Gly Ala Glu Leu Leu Val Asp Val Gly Gln Arg Leu Arg

420 425 430

Arg Gly Thr Pro Trp Leu Arg Val His Arg Asp Gly Pro Ala Leu Ser

435 440 445

Gly Pro Gln Ser Arg Ala Leu Gln Glu Ala Leu Val Leu Ser Asp Arg

450 455 460

```
Ala Pro Phe Ala Ala Pro Ser Pro Phe Ala Glu Leu Val Leu Pro Pro
465                 470                 475                 480
Gln Gln
```